(19) Europäisches Patentamt
European Patent Office
Office européen des brevets

(11) EP 2 376 479 B1

(12) EUROPÄISCHE PATENTSCHRIFT

(45) Veröffentlichungstag und Bekanntmachung des Hinweises auf die Patenterteilung:
**02.03.2016 Patentblatt 2016/09**

(21) Anmeldenummer: **09803727.8**

(22) Anmeldetag: **16.12.2009**

(51) Int Cl.:
***C07D 405/06*** (2006.01) ***C07D 413/06*** (2006.01)
***C07D 417/06*** (2006.01) ***A61K 31/42*** (2006.01)
***A61K 31/425*** (2006.01) ***A61K 31/41*** (2006.01)

(86) Internationale Anmeldenummer:
**PCT/EP2009/008978**

(87) Internationale Veröffentlichungsnummer:
**WO 2010/075957 (08.07.2010 Gazette 2010/27)**

# (54) SUBSTITUIERTE FURANCARBOXAMIDE UND IHRE VERWENDUNG

SUBSTITUTED FURANCARBOXAMIDES, AND USE THEREOF

FURANE CARBOXAMIDES À SUBSTITUTION ET LEUR UTILISATION

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **17.12.2008 DE 102008062878**

(43) Veröffentlichungstag der Anmeldung:
**19.10.2011 Patentblatt 2011/42**

(73) Patentinhaber: **AiCuris GmbH & Co. KG**
**42117 Wuppertal (DE)**

(72) Erfinder:
- **THEDE, Kai**
**10405 Berlin (DE)**
- **GRESCHAT, Susanne**
**49419 Wagenfeld (DE)**
- **WILDUM, Steffen**
**58332 Schwelm (DE)**
- **PAULSEN, Daniela**
**42105 Wuppertal (DE)**

(74) Vertreter: **Kilger, Ute et al**
**Boehmert & Boehmert**
**Anwaltspartnerschaft mbB**
**Patentanwälte Rechtsanwälte**
**Pettenkoferstrasse 20-22**
**80336 München (DE)**

(56) Entgegenhaltungen:
**WO-A1-2004/076453 WO-A2-2005/007625**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 376 479 B1

## Beschreibung

Die vorliegende Erfindung betrifft neue substituierte Furancarboxamide, Verfahren zu ihrer Herstellung, diese Verbindungen zur Verwendung in der Behandlung und/oder Prophylaxe von Krankheiten sowie ihre Verwendung zur Herstellung von Arzneimitteln zur Behandlung und/oder Prophylaxe von Krankheiten, insbesondere von retroviralen Erkrankungen, bei Menschen und/oder Tieren.

HIV (Virus der humanen Immundefizienz) verursacht eine chronisch-persistente, progrediente Infektion. Die Erkrankung verläuft über verschiedene Stadien von der asymptomatischen Infektion bis zum Krankheitsbild AIDS {Acquired Immunodeficiency Syndrom). AIDS ist das finale Stadium der durch Infektion hervorgerufenen Erkrankung. Charakteristisch für die HIV/AIDS-Erkrankung ist die lange klinische Latenzzeit mit persistierender Virämie, die im Endstadium zum Versagen der Immunabwehr führt.

Durch die Einführung der anti-HIV Kombinationstherapie gelang es in den 90-er Jahren, die Krankheitsprogression nachhaltig zu verlangsamen und damit die Lebenserwartung HIV-infizierter Patienten substantiell zu verlängern (Palella et al., N. Engl. J. Med. 1998, 238, 853-860).

Die derzeit auf dem Markt befindlichen anti-HIV-Substanzen hemmen die Replikation des HI-Virus durch Inhibition der essentiellen viralen Enzyme Reverse Transkriptase (RT), Protease oder Integrase oder des Eintritts von HIV in die Zielzelle (Übersicht in Flexner, Nature Reviews Drug Discovery 2007, 6, 959-966). Es existieren zwei Klassen von RT-Inhibitoren: Nukleosidische und nukleotidische RT-Inhibitoren (NRTI) wirken durch kompetitive Inhibition oder Kettenabbruch bei der DNA-Polymerisation. Nicht-nukleosidische RT-Inhibitoren (NNRTI) binden allosterisch an einer hydrophoben Tasche in der Nähe des aktiven Zentrums der RT und vermitteln eine Konformationsänderung des Enzyms. Die derzeit verfügbaren Proteaseinhibitoren (PI) blockieren das aktive Zentrum der viralen Protease und verhindern somit die Reifung neuentstehender Partikel zu infektiösen Virionen. Der einzige derzeit zugelassene Integrase-Inhibitor Raltegravir bindet in das aktive Zentrum der HIV-Integrase und verhindert die Integration der proviralen DNA in das Wirtszellgenom. "Entry-Inhibitoren" (Fusions-Inhibitoren und Korezeptor-Antagonisten) verhindern die HIV-Infektion von Zellen durch Interaktion mit dem HIV-Hüllprotein oder durch Blockierung der zellulären Korezeptoren CCR5 oder CXCR4.

Da die Monotherapie mit den momentan verfügbaren anti-HIV-Medikamenten innerhalb sehr kurzer Zeit zum Therapieversagen durch Selektion resistenter Viren führt, erfolgt üblicherweise eine Kombinationstherapie mit mehreren anti-HIV-Substanzen aus verschiedenen Klassen (*highly active antiretroviral therapy* = HAART; Carpenter et al., J. Am. Med. Assoc. 2000, 283, 381-390).

Trotz der Fortschritte in der antiretroviralen Chemotherapie zeigen neuere Untersuchungen, dass mit den zur Verfügung stehenden Medikamenten eine Eradikation von HIV und damit verbunden eine Heilung der HIV-Infektion nicht zu erwarten ist. Das latente Virus verbleibt in ruhenden Lymphozyten und stellt ein Reservoir für eine Reaktivierung und damit für eine erneute Virusausbreitung dar (Finzi et al., Nature Med. 1999, 5, 512-517; Ramratnam et al., Nature Med. 2000, 6, 82-85). HIVinfizierte Patienten sind daher zeitlebens auf eine effiziente antivirale Therapie angewiesen. Trotz Kombinationstherapie kommt es nach einiger Zeit zur Selektion resistenter Viren. Da für jede therapeutische Klasse charakteristische Resistenzmutationen akkumulieren, bedeutet das Versagen einer Therapie oft einen Wirkverlust der kompletten Substanzklasse. Diese Kreuzresistenzproblematik ist bei der Klasse der NNRTIs am ausgeprägtesten, da hier oft schon eine einzelne Punktmutation in der RT ausreichen kann, um einen Wirkverlust aller NNRTIs zu bewirken (Übersicht in Kavlick & Mitsuya, Antiretroviral Chemotherapy (Hrsg. De Clercq E.), 2001, ASM Press, 279-312).

Begünstigt wird die Entstehung von Resistenzen meist durch die schlechte Compliance der Patienten, die durch ein ungünstiges Nebenwirkungsprofil und kompliziertes Dosierungsschema der anti-HIV-Medikamente hervorgerufen wird.

Somit besteht ein dringender Bedarf nach neuen therapeutischen Optionen zur Bekämpfung der HIV-Infektion. Dazu ist es ein vordringliches Ziel der Therapieforschung zu HIV, neue chemische Leitstrukturen zu identifizieren, die entweder ein neues Target in der Vermehrung des HIV adressieren und/oder gegen die wachsende Zahl resistenter klinischer HIV-Isolate wirksam sind.

Aus der US 5,342,835 sind Furancarboxamide mit fungizider Wirkung bekannt. Die WO 2004/076453 A1 offenbart Furancarboxamide als cholinergische Liganden an den nikotinischen Acetylcolinrezeptoren. Aus der WO 2005/007625 A2 sind Furancarboxamide mit Anti-Tuberkulosewirkung bekannt. Aus der WO 2008/043775 A1 sind Furancarboxamide bekannt, die als nützlich zur Prophylaxe und/oder Behandlung von Störungen des zentralen Nervensystems sowie von psychischen Störungen, Parkinson, Demenz vom Alzheimertyp, neurodegenerativen Erkrankungen sowie sexueller Dysfunktion beschrieben sind. Die WO 2006/062982 A2 offenbart Furancarboxamide als Inhibitoren von Proteinkinasen wie bspw. der MAP-Kinase. Die WO 2008/080056 offenbart Furancarboxamide zur Verwendung in der Krebstherapie. Aus der WO 2008/090382 A1 sind Furancarboxamide bekannt, die zur Behandlung von Prionenerkrankungen, Krebs und Störungen des zentralen Nervensystems sowie für die Regulation von Stammzellen verwendet werden.

Eine Aufgabe der vorliegenden Erfindung ist es daher, neue Verbindungen mit gleicher oder verbesserter

antiviraler Wirkung zur Behandlung von viralen Infektionskrankheiten bei Menschen und Tieren zur Verfügung zu stellen, die die zuvor beschriebenen Nachteile nicht aufweisen.

Überraschenderweise wurde gefunden, dass die in der vorliegenden Erfindung beschriebenen substituierten Furancarboxamide antiviral wirksam sind.

Gegenstand der Erfindung sind Verbindungen der Formel

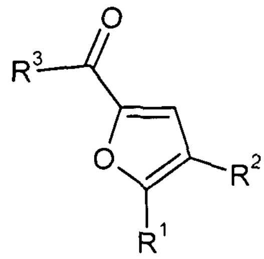

(I),

in welcher

$R^1$ für Phenyl steht,
wobei Phenyl substituiert ist mit 1 bis 3 Substituenten, wobei die Substituenten unabhängig voneinander ausgewählt werden aus der Gruppe bestehend aus Halogen, Hydroxy, Cyano, Nitro, Trifluormethyl, Trifluormethoxy, Trifluormethylthio, $(C_1-C_4)$-Alkyl und $(C_1-C_4)$-Alkoxy,
worin

$(C_1-C_4)$-Alkyl und $(C_1-C_4)$-Alkoxy ihrerseits ein- bis dreifach, gleich oder verschieden, mit Resten substituiert sein können, die ausgewählt werden aus der Reihe Halogen, Cyano, Hydroxy, $(C_1-C_4)$-Alkoxy, Amino, Mono-$(C_1-C_4)$-alkylamino, Di-$(C_1-C_4)$-alkylamino, $(C_3-C_7)$-Cycloalkyl und 4- bis 7-gliedriges Heterocyclyl,

wobei die letztgenannten Cycloalkyl- und Heterocyclyl-Reste ihrerseits jeweils bis zu dreifach, gleich oder verschieden, mit Halogen, Cyano, $(C_1-C_4)$-Alkyl, Trifluormethyl, Hydroxy, $(C_1-C_4)$-Alkoxy, Trifluormetho*xy, Oxo, Amino*, Mono-$(C_1-C_4)$-alkylamino und Di-$(C_1-C_4)$-alkylamino substituiert sein können,

$R^2$ für Phenyl steht,
wobei Phenyl substituiert ist mit 1 bis 3 Substituenten, wobei die Substituenten unabhängig voneinander ausgewählt werden aus der Gruppe bestehend aus Halogen, Hydroxy, Cyano, Nitro, Trifluormethyl, Trifluormethoxy, Trifluormethylthio, $(C_1-C_4)$-Alkyl und $(C_1-C_4)$-Alkoxy,
worin

$(C_1-C_4)$-Alkyl und $(C_1-C_4)$-Alkoxy ihrerseits ein- bis dreifach, gleich oder verschieden, mit Resten substituiert sein können, die ausgewählt werden aus der Reihe Halogen, Cyano, Hydroxy, $(C_1-C_4)$-Alkoxy, Amino, Mono-$(C_1-C_4)$-alkylamino, Di-$(C_1-C_4)$-alkylamino, $(C_3-C_7)$-Cycloalkyl und 4- bis 7-gliedriges Heterocyclyl,
wobei die letztgenannten Cycloalkyl- und Heterocyclyl-Reste ihrerseits jeweils bis zu dreifach, gleich oder verschieden, mit Halogen, Cyano, $(C_1-C_4)$-Alkyl, Trifluormethyl, Hydroxy, $(C_1-C_4)$-Alkoxy, Trifluormethoxy, Oxo, Amino, Mono-$(C_1-C_4)$-alkylamino und Di-$(C_1-C_4)$-alkylamino substituiert sein können, und

$R^3$ für einen über Stickstoff gebundenen Heterocyclyl-Rest steht,
wobei der Heterocyclyl-Rest substituiert sein kann mit 1 bis 3 Substituenten, wobei die Substituenten unabhängig voneinander ausgewählt werden aus der Gruppe bestehend aus Halogen, Hydroxy, Hydroxymethyl, Formyl, Amino, Oxo, Trifluormethyl, Trifluormethoxy, $(C_1-C_4)$-Alkyl, $(C_1-C_4)$-Alkoxy und $(C_1-C_4)$-Alkoxycarbonyl,

und ihre Salze, ihre Solvate und die Solvate ihrer Salze.

Erfindungsgemäße Verbindungen sind die Verbindungen der Formeln (I) und (Ia) und deren Salze, Solvate und Solvate der Salze, sowie die von den Formeln (I) und (Ia) umfassten, nachfolgend als Ausführungsbeispiel(e) genannten Verbindungen und deren Salze, Solvate und Solvate der Salze, soweit es sich bei den von den Formeln (I) und (Ia) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die

stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind, aber beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Im Rahmen der vorliegenden Erfindung haben die Substituenten, soweit nicht anders spezifiziert, die folgende Bedeutung:

Alkyl sowie die Alkylteile in Alkoxy und Alkoxycarbonyl stehen für geradkettiges oder verzweigtes Alkyl und umfassen, wenn nicht anders angegeben, ($C_1$-$C_6$)-Alkyl, insbesondere ($C_1$-$C_4$)-Alkyl, wie z.B. Methyl, Ethyl, Propyl, Isopropyl, Butyl, Isobutyl.

Alkoxy steht im Rahmen der Erfindung vorzugsweise für einen geradkettigen oder verzweigten Alkoxyrest insbesondere mit 1 bis 6, 1 bis 4 bzw. 1 bis 3 Kohlenstoffatomen. Bevorzugt ist ein geradkettiger oder verzweigter Alkoxyrest mit 1 bis 3 Kohlenstoffatomen. Beispielhaft und vorzugsweise seien genannt: Methoxy, Ethoxy, *n*-Propoxy, Isopropoxy, t-Butoxy, n-Pentoxy und n-Hexoxy.

Alkoxycarbonyl steht beispielhaft und vorzugsweise für Methoxycarbonyl, Ethoxycarbonyl, *n*-Propoxycarbonyl, Isopropoxycarbonyl, *t*-Butoxycarbonyl, n-Pentoxycarbonyl und *n*-Hexoxycarbonyl.

Heterocyclyl steht für einen monocyclischen, heterocyclischen Rest mit 4 bis 7, vorzugsweise 5 bis 6 Ringatomen und bis zu 3, vorzugsweise bis zu 2 Heteroatomen und/oder Heterogruppen aus der Reihe N, O, S, SO, $SO_2$, wobei ein Stickstoffatom auch ein N-Oxid bilden kann. Der Heterocyclus kann gesättigt oder teilweise ungesättigt sein. Bevorzugt sind 5- bis 7-gliedrige, monocyclische gesättigte Heterocyclen mit bis zu zwei Heteroatomen aus der Reihe O, N und S, beispielhaft und vorzugsweise 1,4-Oxazepanyl, Oxetan-3-yl, Pyrrolidin-1-yl, Pyrrolidin-2-yl, Pyrrolidin-3-yl, Tetrahydrofuranyl, Tetrahydrothienyl, Pyranyl, 1,3-Thiazolidinyl, Piperidin-1-yl, Piperidin-2-yl, Piperidin-3-yl, Piperidin-4-yl, Thiopyranyl, Morpholin-2-yl, Morpholin-3-yl, Morpholin-4-yl, Thiomorpholin-2-yl, Thiomorpholin-3-yl, Thiomorpholin-4-yl, Perhydroazepinyl, Piperazin-1-yl, Piperazin-2-yl.

Halogen steht für Fluor, Chlor, Brom oder Jod, wobei Fluor und Chlor bevorzugt sind, wenn nichts anderes angegeben ist.

Mono-($C_1$-$C_4$)-alklyamino steht im Rahmen der Erfindung für eine Amino-Gruppe mit einem geradkettigen oder verzweigten Alkylsubstituenten, der 1 bis 4 Kohlenstoffatome aufweist. Beispielhaft und vorzugsweise seien genannt: Methylamino, Ethylamino, *n*-Propylamino, Isopropylamino, *n*-Butylamino, *tert*.-Butylamino, *n*-Pentylamino und *n*-Hexylamino.

Di-($C_1$-$C_4$)-alkylamino steht im Rahmen der Erfindung für eine Amino-Gruppe mit zwei gleichen oder verschiedenen geradkettigen oder verzweigten Alkylsubstituenten, die jeweils 1 bis 4 Kohlenstoffatome aufweisen. Beispielhaft und vorzugsweise seien genannt: *N,N*-Dimethylamino, *N,N*-Diethylamino, *N*-Ethyl-*N*-methylamino, *N*-Methyl-*N*-*n*-propylamino, *N*-Isopropyl-*N*-*n*-propylamino, *N,N*-Diisopropylamino, *N*-*n*-Butyl-*N*-methylamino, *N*-t*ert*.-Butyl-*N*-methylamino, *N*-Methyl-*N*-*n*-pentylamino und *N*-*n*-Hexyl-*N*-methylamino.

$(C_3-C_7)$-Cycloalkyl steht im Rahmen der Erfindung für einen monocyclischen, gesättigten Carbocyclus mit 3 bis 7 bzw. 3 bis 6 Ring-Kohlenstoffatomen. Beispielhaft und vorzugsweise seien genannt: Cyclopropyl, Cyclobutyl, Cyclopentyl, Cyclohexyl und Cycloheptyl.

Die oben aufgeführten allgemeinen oder in Vorzugsbereichen angegebenen Restedefinitionen gelten sowohl für die Endprodukte der Formeln (I) und (Ia) als auch entsprechend für die jeweils zur Herstellung benötigten Ausgangsstoffe bzw. Zwischenprodukte.

Die in den jeweiligen Kombinationen bzw. bevorzugten Kombinationen von Resten im Einzelnen angegebenen Restedefinitionen werden unabhängig von den jeweilig angegebenen Kombinationen der Reste beliebig auch durch Restedefinitionen anderer Kombinationen ersetzt.

Gegenstand der Erfindung sind auch Verbindungen der Formel (I), in welcher

$R^1$ für Phenyl steht,
wobei Phenyl substituiert ist mit 1 bis 2 Substituenten, wobei die Substituenten unabhängig voneinander ausgewählt werden aus der Gruppe bestehend aus Halogen, Hydroxy, Cyano, Nitro, Trifluormethyl, Trifluormethoxy, Trifluormethylthio, $(C_1-C_4)$-Alkyl und $(C_1-C_4)$-Alkoxy,

$R^2$ für Phenyl steht,
wobei Phenyl substituiert ist mit 1 bis 2 Substituenten, wobei die Substituenten unabhängig voneinander ausgewählt werden aus der Gruppe bestehend aus Halogen, Hydroxy, Cyano, Nitro, Trifluormethoxy, Trifluormethylthio, $(C_1-C_4)$-Alkyl und $(C_1-C_4)$-Alkoxy, worin

$(C_1-C_4)$-Alkoxy seinerseits ein- bis dreifach, gleich oder verschieden, mit Resten substituiert sein kann, die ausgewählt werden aus der Reihe Halogen, Cyano, Hydroxy, $(C_1-C_4)$-Alkoxy, Amino, Mono-$(C_1-C_4)$-alkylamino, Di-$(C_1-C_4)$-alkylamino, $(C_3-C_7)$-Cycloalkyl und 4- bis 7-gliedriges Heterocyclyl,

wobei die letztgenannten Cycloalkyl- und Heterocyclyl-Reste ihrerseits jeweils bis zu dreifach, gleich oder verschieden, mit Halogen, Cyano, $(C_1-C_4)$-Alkyl, Trifluormethyl, Hydroxy, $(C_1-C_4)$-Alkoxy, Trifluormethoxy, Oxo, Amino, Mono-$(C_1-C_4)$-alkylamino und Di-$(C_1-C_4)$-alkylamino substituiert sein können, und

$R^3$ für einen über Stickstoff gebundenen Heterocyclyl-Rest steht,
wobei der Heterocyclyl-Rest substituiert sein kann mit 1 bis 3 Substituenten, wobei die Substituenten unabhängig voneinander ausgewählt werden aus der Gruppe bestehend aus Halogen, Hydroxy, Hydroxymethyl, Formyl, Amino, Oxo, Trifluormethyl, Trifluormethoxy, $(C_1-C_4)$-Alkyl, $(C_1-C_4)$-Alkoxy und $(C_1-C_4)$-Alkoxycarbonyl,

und ihre Salze, ihre Solvate und die Solvate ihrer Salze.

Gegenstand der Erfindung sind auch Verbindungen der Formel (I), in welcher

$R^1$ für Phenyl steht,
wobei Phenyl substituiert ist mit 1 bis 2 Substituenten, wobei die Substituenten unabhängig voneinander ausgewählt werden aus der Gruppe bestehend aus Halogen, Cyano, Trifluormethyl, Methyl und Methoxy,

$R^2$ für Phenyl steht,
wobei Phenyl substituiert ist mit 1 bis 2 Substituenten, wobei die Substituenten unabhängig voneinander ausgewählt werden aus der Gruppe bestehend aus Halogen, Cyano, Trifluormethoxy, Methyl und $(C_1-C_3)$-Alkoxy, und

$R^3$ für einen über Stickstoff gebundenen Heterocyclyl-Rest steht,
wobei der Heterocyclyl-Rest substituiert sein kann mit 1 bis 3 Substituenten, wobei die Substituenten unabhängig voneinander ausgewählt werden aus der Gruppe bestehend aus Halogen, Hydroxy, Amino, Oxo, Trifluormethyl, $(C_1-C_4)$-Alkyl und $(C_1-C_4)$-Alkoxy,

und ihre Salze, ihre Solvate und die Solvate ihrer Salze.

Gegenstand der Erfindung sind auch Verbindungen der Formel (I), in welcher

$R^1$ für Phenyl steht,
wobei Phenyl substituiert ist mit 1 bis 2 Substituenten, wobei die Substituenten unabhängig voneinander ausgewählt werden aus der Gruppe bestehend aus Halogen, Cyano und Methyl,

$R^2$ für Phenyl steht,
wobei Phenyl substituiert ist mit 1 bis 2 Substituenten, wobei die Substituenten unabhängig voneinander ausgewählt werden aus der Gruppe bestehend aus Halogen, Cyano, Methyl und Methoxy, und

$R^3$ für einen über Stickstoff gebundenen Heterocyclyl-Rest steht,
wobei der Heterocyclyl-Rest substituiert sein kann mit 1 bis 2 Substituenten aus der Gruppe Hydroxy und Oxo,

und ihre Salze, ihre Solvate und die Solvate ihrer Salze.

Gegenstand der Erfindung sind auch Verbindungen der Formel (I), in welcher

$R^1$ für Phenyl steht,
wobei Phenyl substituiert ist mit 1 bis 2 Substituenten, wobei die Substituenten unabhängig voneinander ausgewählt werden aus der Gruppe bestehend aus Halogen und Cyano,

$R^2$ für Phenyl steht,
wobei Phenyl substituiert ist mit 1 bis 2 Substituenten, wobei die Substituenten unabhängig voneinander ausgewählt werden aus der Gruppe bestehend aus Halogen und Cyano, und

$R^3$ für

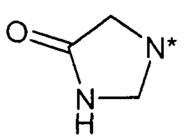 , 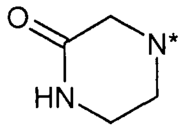 , 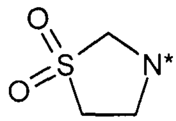 ,

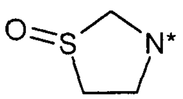 , 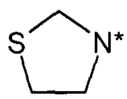 oder 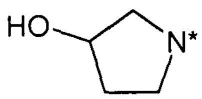

steht, wobei
* die Anknüpfungsstelle an die Carbonylgruppe bedeutet,

und ihre Salze, ihre Solvate und die Solvate ihrer Salze.

Gegenstand der Erfindung sind auch Verbindungen der Formel

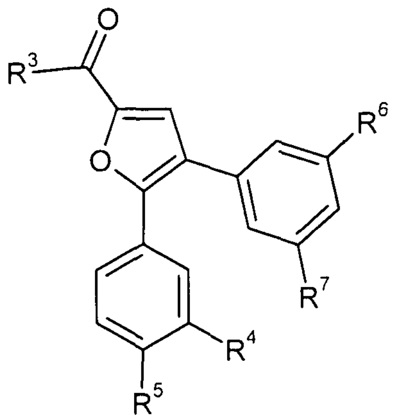

(Ia),

in welcher

$R^3$ für einen über Stickstoff gebundenen Heterocyclyl-Rest steht,
wobei der Heterocyclyl-Rest substituiert sein kann mit 1 bis 2 Substituenten aus der Gruppe Hydroxy und Oxo,
$R^4$ für Halogen, Cyano oder Methyl steht,
$R^5$ für Wasserstoff oder Halogen steht,

$R^6$ für Halogen, Cyano, Methyl oder Methoxy steht, und
$R^7$ für Wasserstoff oder Halogen steht,

und ihre Salze, ihre Solvate und die Solvate ihrer Salze.

Gegenstand der Erfindung sind auch Verbindungen der Formel (Ia), in welcher

$R^3$ für

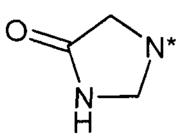 , 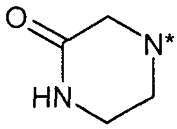 , 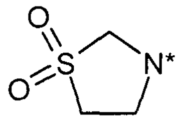 ,

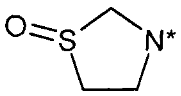 , 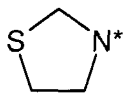 oder 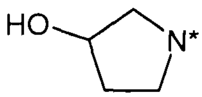

steht, wobei
* die Anknüpfungsstelle an die Carbonylgruppe bedeutet,
$R^4$ für Halogen, Cyano oder Methyl steht,
$R^5$ für Wasserstoff oder Halogen steht,
$R^6$ für Halogen, Cyano, Methyl oder Methoxy steht, und
$R^7$ für Wasserstoff oder Halogen steht,

und ihre Salze, ihre Solvate und die Solvate ihrer Salze.

Gegenstand der Erfindung sind auch Verbindungen der Formel (Ia), in welcher

$R^3$ für

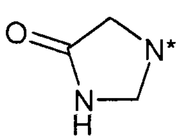 , 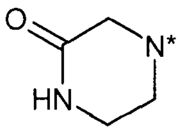 , 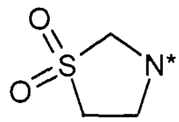 ,

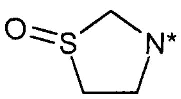 , 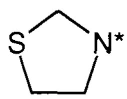 oder 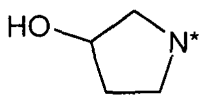

steht, wobei
* die Anknüpfungsstelle an die Carbonylgruppe bedeutet,
$R^4$ für Fluor, Chlor, Cyano oder Methyl steht,
$R^5$ für Wasserstoff oder Fluor steht,
$R^6$ für Fluor, Chlor, Cyano, Methyl oder Methoxy steht, und
$R^7$ für Wasserstoff oder Fluor steht,

und ihre Salze, ihre Solvate und die Solvate ihrer Salze.

Gegenstand der Erfindung sind auch Verbindungen der Formel (Ia), in welcher

$R^3$ für

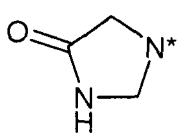

oder

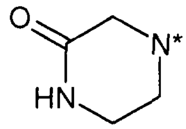

steht, wobei

* die Anknüpfungsstelle an die Carbonylgruppe bedeutet,

$R^4$ für Chlor oder Cyano steht,

$R^5$ für Wasserstoff oder Fluor steht,

$R^6$ für Chlor oder Cyano steht, und

$R^7$ für Wasserstoff oder Fluor steht,

und ihre Salze, ihre Solvate und die Solvate ihrer Salze.

Gegenstand der Erfindung sind auch Verbindungen der Formel (Ia), in welcher

$R^3$ für

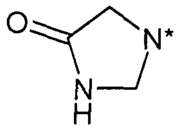

steht, wobei

* die Anknüpfungsstelle an die Carbonylgruppe bedeutet,

$R^4$ für Chlor oder Cyano steht,

$R^5$ für Wasserstoff oder Fluor steht,

$R^6$ für Chlor oder Cyano steht, und

$R^7$ für Wasserstoff oder Fluor steht,

und ihre Salze, ihre Solvate und die Solvate ihrer Salze.

Gegenstand der Erfindung ist weiterhin ein Verfahren zur Herstellung der Verbindungen der Formeln (I) und (Ia), wobei Verbindungen der Formel

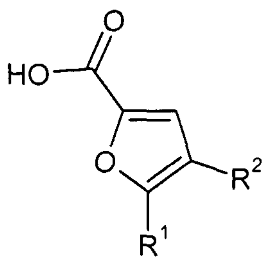

(II),

in welcher

$R^1$ und $R^2$ die oben angegebene Bedeutung aufweisen, mit Verbindungen der Formel $R^3$, mit der oben angegebenen Bedeutung, oder einem Salz der Verbindungen der Formel $R^3$ umgesetzt werden.

Die Umsetzung erfolgt im Allgemeinen in inerten Lösungsmitteln, in Gegenwart eines Dehydratisierungsreagenzes, gegebenenfalls in Gegenwart einer Base, bevorzugt in einem Temperaturbereich von -30°C bis 50°C bei Normaldruck.

Inerte Lösungsmittel sind beispielsweise Halogenkohlenwasserstoffe wie Dichlormethan oder Trichlormethan, Kohlenwasserstoffe wie Benzol oder Toluol, Nitromethan, Tetrahydrofuran, 1,4-Dioxan, Dimethylformamid oder Acetonitril. Ebenso ist es möglich, Gemische der Lösemittel einzusetzen. Besonders bevorzugt sind Dichlormethan, Dimethylformamid, Tetrahydrofuran oder Toluol.

Basen sind beispielsweise Alkalicarbonate, wie z.B. Natrium- oder Kaliumcarbonat, oder -hydrogencarbonat, oder organische Basen wie Trialkylamine z.B. Triethylamin, *N*-Methylmorpholin, *N*-Methylpiperidin, 4-Dimethylaminopyridin oder Diisopropylethylamin.

Als Dehydratisierungsreagenzien eignen sich hierbei beispielsweise Carbodiimide wie z.B. *N,N'*-Diethyl-, *N,N'*-Dipropyl-, *N,N'*-Diisopropyl-, *N,N'*-Dicyclohexylcarbodiimid, *N*-(3-Dimethylaminoisopropyl)-*N'*-ethylcarbodiimid-hydrochlorid (EDC), *N*-Cyclohexylcarbodiimid-*N'*-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-*tert*-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)phosphorylchlorid, oder *O*-(Benzotriazol-1-yl)-*N,N,N',N'*-tetramethyluroniumhexafluorophosphat (HBTU), 2-(2-Oxo-1-(2H)-pyridyl)-1,1,3,3-tetramethyluroniumtetrafluoroborat (TPTU) oder *O*-(7-Azabenzotriazol-1-yl)-*N,N,N',N'*-tetramethyluroniumhexafluorophosphat (HATU), oder 1-Hydroxybenztriazol (HOBt), oder Benzotriazol-1-yloxytris(dimethylamino)phosphoniumhexafluorophosphat (BOP), oder Benzotriazol-1-yloxytris(pyrrolidino)phosphoniumhexafluorophosphat (PyBOP), oder *N*-Hydroxysuccinimid, oder Mischungen aus diesen, mit Basen.

Vorzugsweise wird die Kondensation mit PyBOP, TBTU oder mit EDC in Gegenwart von HOBt durchgeführt.

In einem alternativen Verfahren können die Verbindungen der Formel (II) zunächst mit Thionylchlorid und in der zweiten Stufe mit Verbindungen der Formel $R^3$ oder einem Salz von Verbindungen der Formel $R^3$ in Gegenwart einer Base, wie z.B. Triethylamin, umgesetzt werden.

Die nach den oben angegebenen Verfahren hergestellten Verbindungen der Formeln (I) und (Ia) tragen gegebenenfalls Schutzgruppen, die nach dem Fachmann bekannten Bedingungen abgespalten werden können, um weitere Verbindungen der Formeln (I) und (Ia) zu erhalten.

Die Verbindungen der Formel $R^3$ oder Salze der Verbindungen der Formel $R^3$ sind bekannt oder lassen sich nach bekannten Verfahren aus den entsprechenden Edukten synthetisieren.

Die Verbindungen der Formel (II) sind bekannt oder können hergestellt werden, indem in Verbindungen der Formel

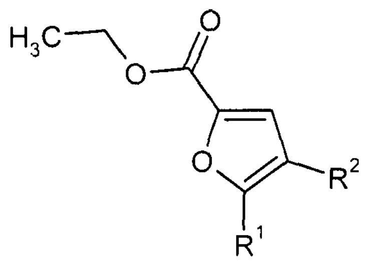

(III),

in welcher

$R^1$ und $R^2$ die oben angegebene Bedeutung aufweisen,

der Ester mit einer Base verseift wird.

Die Verseifung des Esters mit einer Base erfolgt im Allgemeinen in inerten Lösungsmitteln, bevorzugt in einem Temperaturbereich von Raumtemperatur bis zum Rückfluss des Lösungsmittels bei Normaldruck.

Basen sind beispielsweise Alkalihydroxide wie Natrium-, Lithium- oder Kaliumhydroxid, oder Alkalicarbonate wie Cäsiumcarbonat, Natrium- oder Kaliumcarbonat, bevorzugt sind Lithium-, Kalium- oder Natriumhydroxid.

Inerte Lösungsmittel sind beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-*tert*-butylether, 1,2-Dimethoxyethan, 1,4-Dioxan, Tetrahydrofuran, Glykoldimethylether oder Diethylenglykoldimethylether, Alkohole wie Methanol, Ethanol, n-Propanol, iso-Propanol, n-Butanol oder *tert*-Butanol, Kohlenwasserstoffe wie Benzol, Xylol, Toluol, Hexan, Cyclohexan oder Erdölfraktionen, oder andere Lösungsmittel wie Dimethylformamid, Dimethylacetamid, Dimethylsulfoxid, Acetonitril oder Pyridin, oder Wasser, oder Gemische von Lösungsmitteln. Als Lösungsmittel sind bevorzugt 1,4-Dioxan, Tetrahydrofuran und/oder Methanol. Bevorzugt ist Lithiumhydroxid in Tetrahydrofuran- oder 1,4-Dioxan-Wasser-Gemischen oder Kaliumhydroxid in Methanol.

Die Verbindungen der Formel (III) sind bekannt oder können hergestellt werden, indem Verbindungen der Formel

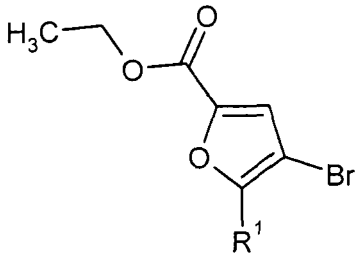

(IV),

in welcher
$R^1$ die oben angegebene Bedeutung aufweist,
unter Suzuki-Kupplungsbedingungen mit Verbindungen der Formel

$R^2$-Q (V),

in welcher

$R^2$ die oben angegebene Bedeutung aufweist und
Q für $-B(OH)_2$, einen Boronsäure-Ester, bevorzugt Boronsäurepinakolester, oder $-BF_3^-K^+$ steht,

umgesetzt werden.

Die Suzuki-Kupplungen erfolgen im Allgemeinen in inerten Lösungsmitteln, in Gegenwart eines Katalysators, gegebenenfalls in Gegenwart eines Zusatzreagenzes, bevorzugt in einem Temperaturbereich von Raumtemperatur bis 130°C bei Normaldruck.

Katalysatoren sind beispielsweise für Suzuki-Reaktionsbedingungen übliche Palladium-Katalysatoren, bevorzugt sind Katalysatoren wie z.B. Dichlorbis(triphenylphosphin)palladium, Tetrakistriphenylphosphinpalladium(0), Palladium(II)acetat, Palladium(II)acetat/Triscyclohexylphosphin oder Bis-(diphenylphosphanferrocenyl)-palladium-(II)-chlorid oder Palladium(II)acetat mit einem Liganden wie Dicyclohexyl[2',4',6'-tri(propan-2-yl)biphenyl-2-yl]phosphan.

Zusatzreagenzien sind beispielsweise Kaliumacetat, Cäsium-, Kalium- oder Natriumcarbonat, Kalium-*tert*.-butylat, Cäsiumfluorid oder Kaliumphosphat, bevorzugt sind Zusatzreagenzien wie z.B. Kaliumacetat und/oder eine wässrige Natriumcarbonatlösung.

Inerte Lösungsmittel sind beispielsweise Ether wie Dioxan, Tetrahydrofuran oder 1,2-Dimethoxyethan, Kohlenwasserstoffe wie Benzol, Xylol oder Toluol, oder Carbonsäureamide wie Dimethylformamid oder Dimethylacetamid, Alkylsulfoxide wie Dimethylsulfoxid, oder N-Methylpyrrolidon, oder Gemische der Lösungsmittel mit Alkoholen wie Methanol oder Ethanol und/oder Wasser, bevorzugt ist 1,2-Dimethoxyethan.

Die Verbindungen der Formel (V) sind bekannt oder lassen sich nach bekannten Verfahren aus den entsprechenden Edukten synthetisieren.

Die Verbindungen der Formel (IV) sind bekannt oder können hergestellt werden, indem die Verbindung der Formel

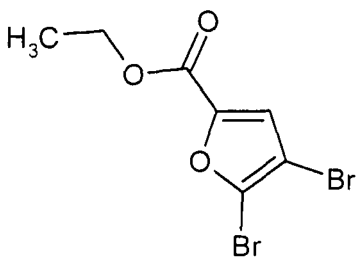

(VI),

unter den oben angegebenen Suzuki-Kupplungsbedingungen mit Verbindungen der Formel

$R^1$-Q (VII),

in welcher

$R^1$ die oben angegebene Bedeutung aufweist und

Q für $-B(OH)_2$, einen Boronsäure-Ester, bevorzugt Boronsäurepinakolester, oder $-BF_3^-K^+$ steht,

umgesetzt wird.

Die Verbindungen der Formel (VII) sind bekannt oder lassen sich nach bekannten Verfahren aus den entsprechenden Edukten synthetisieren.

Gegenstand der Erfindung ist weiterhin ein Verfahren zur Herstellung der Verbindungen der Formel (I) und (Ia), wobei Verbindungen der Formel

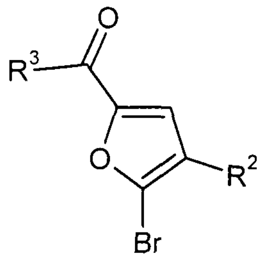

(VIII),

in welcher
$R^2$ und $R^3$ die oben angegebene Bedeutung aufweisen,
unter den oben angegebenen Suzuki-Kupplungsbedingungen mit Verbindungen der Formel

$R^1$-Q (VII),

in welcher

$R^1$ die oben angegebene Bedeutung aufweist und
Q für $-B(OH)_2$, einen Boronsäure-Ester, bevorzugt Boronsäurepinakolester, oder $-BF_3^-K^+$ steht,

umgesetzt werden.

Die Verbindungen der Formel (VIII) sind bekannt oder können hergestellt werden, indem Verbindungen der Formel

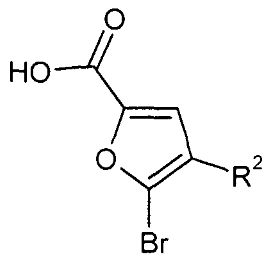

(IX),

in welcher
$R^2$ die oben angegebene Bedeutung aufweist,
mit Verbindungen der Formel $R^3$, mit der oben angegebenen Bedeutung, oder einem Salz der Verbindungen der Formel $R^3$, entsprechend der oben angegebenen Reaktion von (II) zu (I) bzw. (Ia), umgesetzt werden.

Die Verbindungen der Formel (IX) sind bekannt oder können hergestellt werden, indem in Verbindungen der Formel

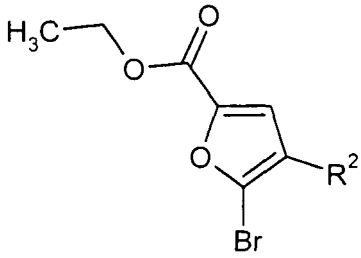

(X),

in welcher
$R^2$ die oben angegebene Bedeutung aufweist,
der Ester mit einer Base, wie oben bei der Reaktion von (III) zu (II) beschrieben, verseift wird.

Die Verbindungen der Formel (X) sind bekannt oder können hergestellt werden, indem Verbindungen der Formel

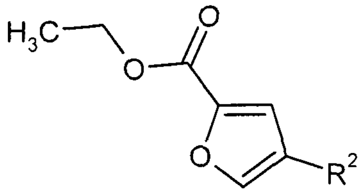

(XI),

in welcher
$R^2$ die oben angegebene Bedeutung aufweist, bromiert werden.

Inerte Lösungsmittel für die Bromierung sind beispielsweise Halogenkohlenwasserstoffe wie Dichlormethan, Trichlormethan, Tetrachlormethan, Trichlorethan, Tetrachlorethan, 1,2-Dichlorethan oder Trichlorethylen, Ether wie Diethylether, Dioxan, Tetrahydrofuran, Glykoldimethylether oder Diethylenglykoldimethylether, Kohlenwasserstoffe wie Hexan oder Cyclohexan, organische Carbonsäuren wie Essigsäure oder andere Lösungsmittel wie Ethylacetat, Dimethylformamid oder Dimethylsulfoxid. Ebenso ist es möglich, Gemische der genannten Lösungsmittel einzusetzen. Bevorzugt sind Essigsäure, Diethylether, Dioxan, Tetrahydrofuran, Ethylacetat, Trichlormethan und/oder Tetrachlormethan.

Als Bromierungsmittel eignen sich die üblichen anorganischen oder organischen Reagenzien. Hierzu gehören bevorzugt Brom, N-Bromsuccinimid, Kupferdibromid, Pyridinhydrotribromid, Dimethylbenzylammoniumtribromid oder Phenyltrimethylammoniumtribromid. Besonders bevorzugt sind Brom und Kupferdibromid.

Die Bromierung wird im Allgemeinen in einem Temperaturbereich von 20°C bis 150°C, bevorzugt von 0°C bis 80°C durchgeführt. Die Umsetzung kann bei normalem, erhöhtem oder erniedrigtem Druck erfolgen (z.B. von 0.5 bis 5 bar). Im Allgemeinen erfolgt die Bromierung bei Normaldruck.

Die Verbindungen der Formel (XI) sind bekannt oder können hergestellt werden, indem die Verbindung der Formel

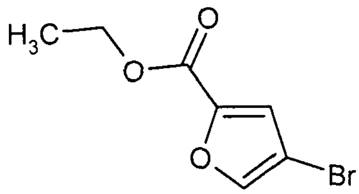

(XII),

unter den oben angegebenen Suzuki-Kupplungsbedingungen mit Verbindungen der Formel

$R^2$-Q (V),

in welcher

$R^2$ die oben angegebene Bedeutung aufweist und
Q für $-B(OH)_2$, einen Boronsäure-Ester, bevorzugt Boronsäurepinakolester, oder $-BF_3^-K^+$ steht,

umgesetzt wird.

Die Verbindung der Formel (XII) ist bekannt oder lässt sich nach bekannten Verfahren aus der Verbindung der Formel (VI) synthetisieren.

Die Herstellung der erfindungsgemäßen Verbindungen kann durch das folgende Syntheseschema verdeutlicht werden.

Syntheseschema:

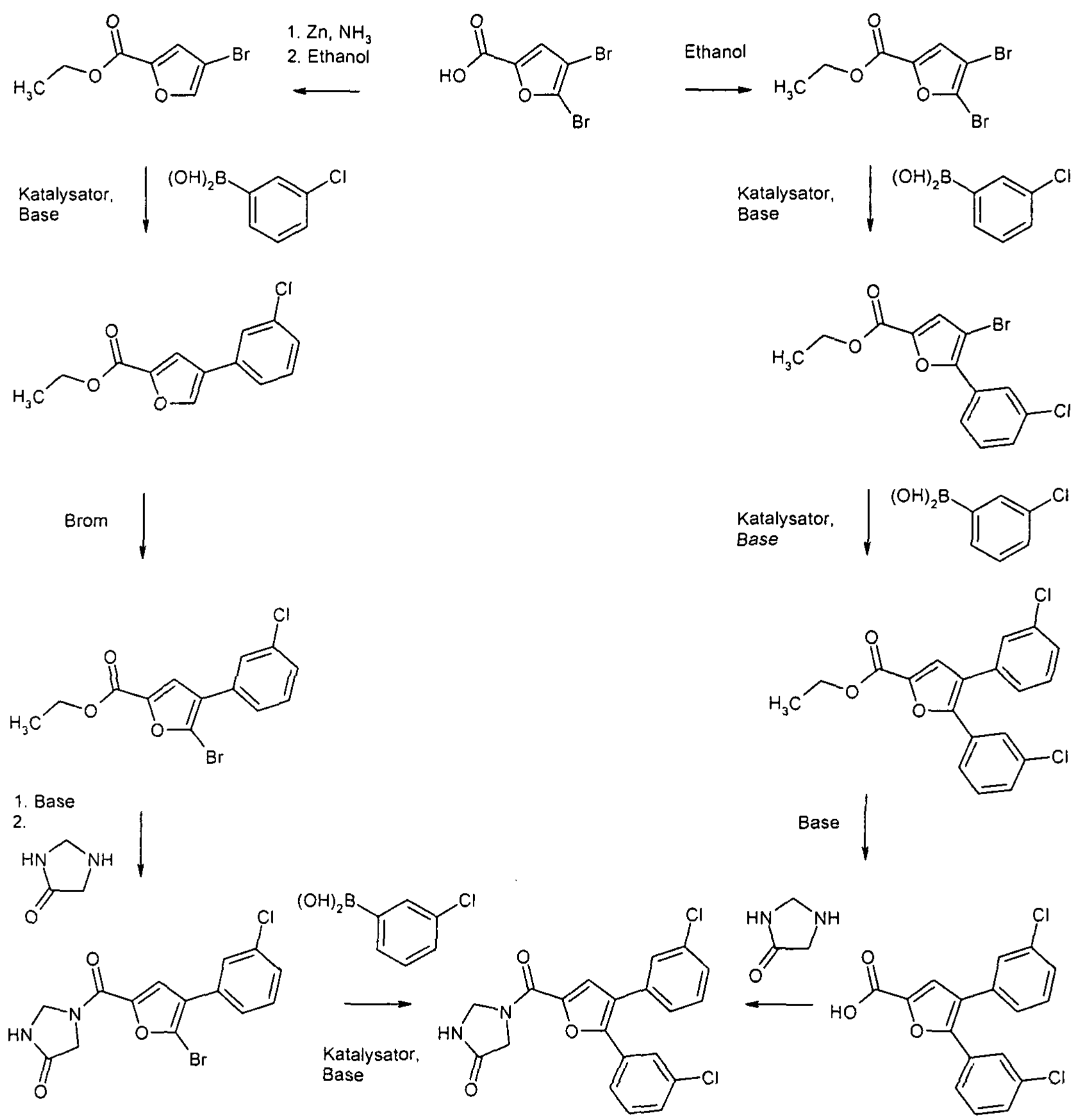


Die erfindungsgemäßen Verbindungen zeigen ein nicht vorhersehbares, wertvolles pharmakologisches Wirkspektrum.

Sie eignen sich daher zur Verwendung als Arzneimittel zur Behandlung und/oder Prophylaxe von Krankheiten bei Menschen und Tieren.

Die Verbindungen der vorliegenden Erfindung zeichnen sich insbesondere durch ein vorteilhaftes antiretrovirales Wirkspektrum aus.

Weiterer Gegenstand der vorliegenden Erfindung sind die erfindungsgemäßen Verbindungen zur Verwendung in der Behandlung und/oder Prophylaxe von Erkrankungen, die durch Retroviren hervorgerufen werden, insbesondere von HI-Viren.

Weiterer Gegenstand der vorliegenden Erfindung sind die erfindungsgemäßen Verbindungen zur Verwendung in der Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor

genannten Erkrankungen.

Weiterer Gegenstand der vorliegenden Erfindung sind die erfindungsgemäßen Verbindungen zur Verwendung in der Behandlung und/oder Prophylaxe von Erkrankungen, insbesondere der zuvor genannten Erkrankungen, unter Verwendung einer therapeutisch wirksamen Menge der erfindungsgemäßen Verbindungen.

Als Indikationsgebiete in der Humanmedizin können beispielsweise genannt werden:

1.) Die Behandlung und Prophylaxe von menschlichen Retrovirusinfektionen
2.) Die Behandlung und Prophylaxe von durch HIV-1 (Virus der humanen Immundefizienz; früher HTLV JII/LAV genannt) und HIV-2 verursachten Infektionen und Erkrankungen (AIDS) und den damit assoziierten Stadien wie ARC (AIDS related complex) und LAS (Lymphadenophathie-Syndrom) sowie der durch dieses Virus verursachten Immunschwäche und Enzephalopathie.
3.) Die Behandlung von HIV-Infektionen hervorgerufen durch einfach-, mehrfach- oder multi-resistente HI-Viren.

Resistente HI-Viren bedeutet z.B. Viren mit Resistenzen gegen nukleosidische RT-Inhibitoren (NRTI), nicht-nukleosidische RT-Inhibitoren (NNRTI) oder Proteaseinhibitoren (PI) oder Viren mit Resistenzen gegen andere Wirkprinzipien, z.B. T20 (Fusionsinhibitoren).

4.) Für die Behandlung oder die Prophylaxe des AIDS-carrier Zustandes (AIDS-Überträger-Zustand).

5.) Für die Behandlung oder die Prophylaxe einer HTLV-I oder HTLV-II Infektion

Als Indikationen in der Tiermedizin können beispielsweise angeführt werden:

Infektionen mit

a) Maedivisna (bei Schafen und Ziegen)

b) progressivem Pneumonievirus (PPV) (bei Schafen und Ziegen)

c) caprine arthritis encephalitis Virus (bei Schafen und Ziegen)

d) Zwoegerziekte Virus (bei Schafen)

e) infektiösem Virus der Anämie (des Pferdes)

f) Infektionen verursacht durch das Katzenleukämievirus

g) Infektionen verursacht durch das Virus der Katzen-Immundefizienz (FIV)

h) Infektionen verursacht durch das Virus der Affen-Immundefizienz (SIV)

Bevorzugt werden aus dem Indikationsgebiet in der Humanmedizin die oben aufgeführten Punkte 2, 3 und 4.

Insbesondere geeignet sind die Substanzen zur Bekämpfung von HI-Viren, die Resistenzen gegen bekannte nicht-nukleosidische Inhibitoren der Reversen Transkriptase, wie z.B. Efavirenz oder Nevirapin, zeigen.

Weiterer Gegenstand der vorliegenden Erfindung sind Arzneimittel, enthaltend mindestens eine erfindungsgemäße Verbindung und mindestens einen oder mehrere weitere Wirkstoffe, insbesondere zur Behandlung und/oder Prophylaxe der zuvor genannten Erkrankungen.

Die erfindungsgemäßen Verbindungen können auch vorteilhaft, insbesondere in den oben aufgeführten Punkten 2, 3 und 4, als Bestandteile einer Kombinationstherapie mit einer oder mehreren anderen, in diesen Anwendungsbereichen aktiven Verbindungen eingesetzt werden. Beispielhaft können diese Verbindungen in Kombination mit wirksamen Dosen von antiviral wirksamen Substanzen, die auf den unten aufgeführten Wirkprinzipien beruhen, eingesetzt werden:

Inhibitoren der HIV Protease; beispielhaft seien genannt: Saquinavir, Indinavir, Ritonavir, Nelfinavir, Amprenavir, Lopinavir, Atazanavir, Fosamprenavir, Tipranavir, Darunavir;

Nukleosidische, nukleotidische und nicht-nukleosidische Inhibitoren der HIV Reversen Transkriptase; beispielhaft seien genannt: Zidovudin, Lamivudin, Didanosin, Zalcitabin, Stavudin, Lamivudin, Abacavir, Tenofovir, Adefovir,

Emtricitabin, Amdoxovir, Apricitabin, Racivir, Nevirapin, Delavirdin, Efavirenz, Etravirin, Rilpivirin, UK-453,061;

Inhibitoren der HIV Integrase, beispielhaft seien genannt: Raltegravir, Elvitegravir;

Inhibitoren der HIV Fusion; beispielhaft sei genannt: Enfuvirtid;

Inhibition der CXCR4/CCR5/gp120 Wechselwirkung; beispielhaft seien genannt: Maraviroc, Vicriviroc, INCB009471, AMD-070;

Inhibition der Polyproteinreifung; beispielhaft sei genannt: Bevirimat.

Diese Auswahl soll zur Verdeutlichung der Kombinationsmöglichkeiten, nicht jedoch zur Einschränkung auf die hier aufgeführten Beispiele dienen; prinzipiell ist jede Kombination der erfindungsgemäßen Verbindungen mit antiviral wirksamen Substanzen als im Rahmen der Erfindung zu betrachten.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rektal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophilisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (wie beispielsweise Pflaster), Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Im Allgemeinen hat es sich sowohl in der Human- als auch in der Veterinärmedizin als vorteilhaft erwiesen, den oder die erfindungsgemäßen Wirkstoffe in Gesamtmengen von 0,1 bis 200 mg/kg, vorzugsweise 1 bis 100 mg/kg Körpergewicht je 24 Stunden, gegebenenfalls in Form mehrerer Einzelgaben, zur Erzielung des gewünschten Ergebnisses zu verabreichen. Eine Einzelgabe enthält den oder die Wirkstoffe vorzugsweise in Mengen von 1 bis 80 mg/kg, insbesondere 1 bis 30 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen. Die Angabe "w/v" bedeutet "weight/volume" (Gewicht/Volumen). So bedeutet beispielsweise "10% w/v": 100 ml Lösung oder Suspension enthalten 10 g Substanz.

**A) Beispiele**

**Abkürzungen:**

| | |
|---|---|
| aq. | wässrig, wässrige Lösung |
| DCI | direkte chemische Ionisation (bei MS) |
| DMA | *N,N*-Dimethylacetamid |
| DMF | *N,N*-Dimethylformamid |
| DMSO | Dimethylsulfoxid |
| d. Th. | der Theorie (bei Ausbeute) |
| EDC | *N'*-(3-Dimethylaminopropyl)-*N*-ethylcarbodiimid x HCl |
| eq. | Äquivalent(e) |
| ESI | Elektrospray-Ionisation (bei MS) |
| h | Stunde(n) |
| HATU | *O*-(7-Azabenzotriazol-1-yl)-*N,N,N',N'*-tetramethyluronium-hexafluorophosphat |
| HPLC | Hochdruck-, Hochleistungsflüssigchromatographie |
| konz. | konzentriert |
| LC-MS | Flüssigchromatographie-gekoppelte Massenspektroskopie |
| min | Minute(n) |
| MS | Massenspektroskopie |
| NMR | Kernresonanzspektroskopie |
| PyBOP | Benzotriazol-1-yloxy-tris(pyrrolidino)phosphonium-hexafluorophosphat |
| $R_t$ | Retentionszeit (bei HPLC) |
| RT | Raumtemperatur |
| TBTU | *O*-(Benzotriazol-1-yl)-*N,N,N',N'*-tetramethyluronium-tetrafluoroborat |
| TFA | Trifluoressigsäure |
| THF | Tetrahydrofuran |
| TMOF | Trimethylorthoformiat |

**LC-MS/GC-MS-Methoden:**

Methode 1:

Gerätetyp MS: Micromass ZQ; Gerätetyp HPLC: HP 1100 Series; UV DAD; Säule: Phenomenex Gemini 3μ 30 mm x 3.00 mm; Eluent A: 1 l Wasser + 0.5 ml 50%ige Ameisensäure, Eluent B: 1 l Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 210 nm.

Methode 2:

Instrument: Micromass Quattro LCZ mit HPLC Agilent Serie 1100; Säule: Phenomenex Synergi 2μ Hydro-RP Mercury 20 mm x 4 mm; Eluent A: 1 l Wasser + 0.5 ml 50%ige Ameisensäure, Eluent B: 1 l Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min → 2.5 min/3.0 min/4.5 min 2 ml/min; Ofen: 50°C; UV-Detektion: 208-400 nm.

Methode 3:

Gerätetyp MS: Micromass ZQ; Gerätetyp HPLC: Waters Alliance 2795; Säule: Phenomenex Synergi 2μ Hydro-RP Mercury 20 mm x 4 mm; Eluent A: 1 l Wasser + 0.5 ml 50%ige Ameisensäure, Eluent B: 1 l Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min → 2.5 min/3.0 min/4.5 min 2 ml/min; Ofen: 50°C; UV-Detektion: 210 nm.

Methode 4:

Instrument: Micromass Quattro LCZ mit HPLC Agilent Serie 1100; Säule: Phenomenex Onyx Monolithic C18, 100 mm x 3 mm. Eluent A: 1 l Wasser + 0.5 ml 50%ige Ameisensäure, Eluent B: 1 l Acetonitril + 0.5 ml 50%ige Ameisensäure; Gradient: 0.0 min 90%A → 2 min 65%A → 4.5 min 5%A → 6 min 5%A; Fluss: 2 ml/min; Ofen: 40°C; UV-Detektion: 208-400 nm.

Methode 5:

Instrument: Micromass QuattroPremier mit Waters UPLC Acquity; Säule: Thermo Hypersil GOLD 1.9μ 50 x 1mm; Eluent A: 1 l Wasser + 0.5ml 50%ige Ameisensäure , Eluent B: 1 l Acetonitril + 0,5 ml 50%ige Ameisensäure; Gradient: 0.0 min 90%A → 0.1 min 90%A → 1.5 min 10%A → 2.2 min 10%A; Ofen: 50°C; Fluss: 0.33 ml/min; UV-Detektion: 210 nm.

Methode 6:

Gerätetyp MS: Waters ZQ; Gerätetyp HPLC: Waters Alliance 2795; Säule: Phenomenex Onyx Monolithic C18, 100 mm x 3 mm; Eluent A: 1 l Wasser + 0.5 ml 50%ige Ameisensäure, Eluent B: 1 l Acetonitril + 0.5 ml 50%ige Ameisensäure; Gradient: 0.0 min 90%A → 2 min 65%A → 4.5 min 5%A → 6 min 5%A; Fluss: 2 ml/min; Ofen: 40°C; UV-Detektion: 210 nm.

Methode 7:

Gerätetyp MS: Micromass ZQ; Gerätetyp HPLC: Waters Alliance 2795; Säule: Phenomenex Synergi 2.5 μ MAX-RP 100A Mercury 20 mm x 4 mm; Eluent A: 1 1 Wasser + 0.5 ml 50%ige Ameisensäure, Eluent B: 1 l Acetonitril + 0.5 ml 50%ige Ameisensäure; Gradient: 0.0 min 90%A → 0.1 min 900/0A → 3.0 min 5%A → 4.0 min 5%A → 4.01 min 90%A; Fluss: 2 ml/min; Ofen: 50°C; UV-Detektion: 210 nm.

Methode 8:

Instrument: Micromass Quattro LCZ mit HPLC Agilent Serie 1100; Säule: Phenomenex Gemini 3μ 30 mm x 3.00 mm; Eluent A: 1 l Wasser + 0.5 ml 50%ige Ameisensäure, Eluent B: 1 l Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 208-400 nm.

Methode 9:

Instrument: Micromass Quattro LCZ mit HPLC Agilent Serie 1100; Säule: Phenomenex Synergi 2.5 μ MAX-RP 100A Mercury 20 mm x 4 mm; Eluent A: 1 l Wasser + 0.5 ml 50%ige Ameisensäure, Eluent B: 1 l Acetonitril + 0.5 ml 50%ige Ameisensäure; Gradient: 0.0 min 90%A → 0.1 min 90%A → 3.0 min 5%A → 4.0 min 5%A → 4.1 min 90%A; Fluss: 2 ml/min; Ofen: 50°C; UV-Detektion: 208- 400 nm.

Methode 10:

Gerätetyp MS: Waters (Micromass) Quattro Micro; Gerätetyp HPLC: Agilent 1100 Serie; Säule: Thermo Hypersil GOLD 3μ 20 mm x 4 mm; Eluent A: 1 l Wasser + 0.5 ml 50%ige Ameisensäure, Eluent B: 1 l Acetonitril + 0.5 ml 50%ige Ameisensäure; Gradient: 0.0 min 100%A → 3.0 min 10%A → 4.0 min 10%A → 4.01 min 100%A (Fluss 2.5 ml) → 5.00 min 100%A; Ofen: 50°C; Fluss: 2 ml/min; UV-Detektion: 210 nm.

Methode 11:

Instrument: Micromass GCT, GC6890; Säule: Restek RTX-35, 15 m x 200 μm x 0.33 μm; konstanter Fluss mit Helium: 0.88 ml/min; Ofen: 70°C; Inlet: 250°C; Gradient: 70°C, 30°C/min → 310°C (3 min halten).

Methode 12:

Instrument: Micromass GCT, GC6890; Säule: Restek RTX-35, 15 m x 200 μm x 0.33 μm; konstanter Fluss mit Helium: 0.88 ml/min; Ofen: 70°C; Inlet: 250°C; Gradient: 70°C, 30°C/min → 310°C (12 min halten).

**Ausgangsverbindungen und Intermediate:**

**Beispiel 1A**

4-Bromfuran-2-carbonsäure

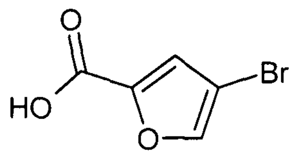

10.0 g (37.1 mmol) 4,5-Dibromfuran-2-carbonsäure werden in wässriger AmmoniakLösung (7.3%ig) vorgelegt und auf 0°C abgekühlt. Unter starkem Rühren werden portionsweise 2.54 g (38.9 mmol) Zinkpulver so zugegeben, dass die Temperatur 7°C nicht übersteigt. Es wird 10 Minuten bei 0°C gerührt und anschließend durch Zugabe von wässriger HCl-Lösung ein saurer pH-Wert eingestellt. Man extrahiert die Suspension mit Essigsäureethylester, trocknet die organische Phase über Natriumsulfat, filtriert und engt ein. Man erhält 7.27 g (99% d. Th.) der Titelverbindung.
$^1$H-NMR (400 MHz, DMSO-$d_6$): $\delta$ = 13.5 (s, 1H), 8.17 (s, 1H), 7.40 (s, 1H).
LC-MS (Methode 1): $R_t$ = 1.52 min; MS (ESIpos): m/z = 191 [M+H]$^+$.

**Beispiel 2A**

4,5-Dibromfuran-2-carbonsäureethylester

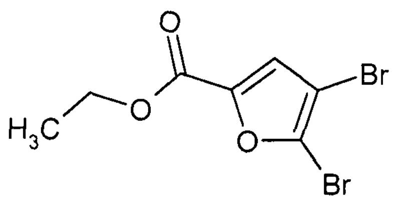

25.0 g (92.6 mmol) 4,5-Dibromfuran-2-carbonsäure werden in 250 ml Ethanol vorgelegt, mit 4.9 ml (92.6 mmol) Schwefelsäure (98%ig) versetzt und über Nacht unter Rückfluss erhitzt. Das Reaktionsgemisch wird eingeengt und zu dem Rückstand gesättigte wässrige Natriumhydrogencarbonat-Lösung bis zu einem basischen pH-Wert gegeben. Der Niederschlag wird abgesaugt, mit Wasser gewaschen und im Hochvakuum getrocknet. Man erhält 26.9 g (97% d. Th.) der Titelverbindung.
$^1$H-NMR (400 MHz, DMSO-$d_6$): $\delta$ = 7.62 (s, 1H), 4.30 (q, 2H), 1.29 (t, 3H).
LC-MS (Methode 1): $R_t$ = 2.43 min; MS (ESIpos): m/z = 297 [M+H]$^+$.

**Beispiel 3A**

4-Bromfuran-2-carbonsäureethylester

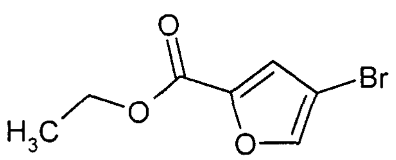

Die Herstellung der Titelverbindung erfolgt ausgehend von der Verbindung aus Beispiel 1A analog zur Synthese der Verbindung aus Beispiel 2A. Man erhält 19.1 g (89% d. Th.) der Titelverbindung.

$^1$H-NMR (400 MHz, DMSO-$d_6$): δ = 8.22 (s, 1H), 7.50 (s, 1H), 4.29 (q, 2H), 1.29 (t, 3H).
GC-MS (Methode 11): $R_t$ = 3.89 min; MS (EIpos): m/z = 218 $[M]^+$.

**Beispiel 4A**

3-Fluor-5-(4,4,5,5-tetramethyl-1,3,2-dioxaborolan-2-yl)benzolcarbonitril

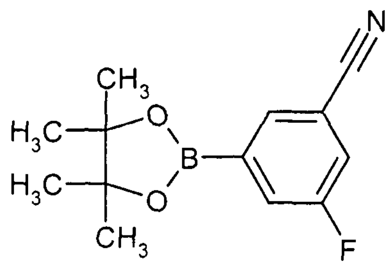

3.60 g (18.0 mmol) 3-Brom-5-fluorbenzolcarbonitril, 5.03 g (19.8 mmol) 4,4,4',4',5,5,5',5'-Octamethyl-2,2'-bi-1,3,2-dioxaborolan und 5.30 g (54.0 mmol) Kaliumacetat werden unter Argon in 72 ml entgastem 1,4-Dioxan/DMSO (10/1) vorgelegt und mit 441 mg (0.54 mmol) 1,1'-Bis(diphenylphosphin)ferrocendichlorpalladium(II)-Dichlormethan-Komplex versetzt. Es wird über Nacht bei 90°C gerührt. Anschließend gibt man Wasser hinzu, trennt die Phasen, extrahiert die wässrige Phase mit Essigsäureethylester und engt die vereinigten organischen Phasen ein. Das Rohprodukt wird mittels Flash-Chromatographie (Laufmittel: Cyclohexan/Essigsäureethylester 10:1) gereinigt. Man erhält 4.48 g (92% d. Th.) der Titelverbindung.
$^1$H-NMR (400 MHz, DMSO-$d_6$): δ = 8.01 (ddd, 1H), 7.82 (s, 1H), 7.70 (ddd, 1H), 1.32 (s, 12H).
GC-MS (Methode 11): $R_t$ = 4.94 min; MS (EIpos): m/z = 247 $[M]^+$.

**Beispiel 5A**

4-Brom-5-(3-chlorphenyl)furan-2-carbonsäureethylester

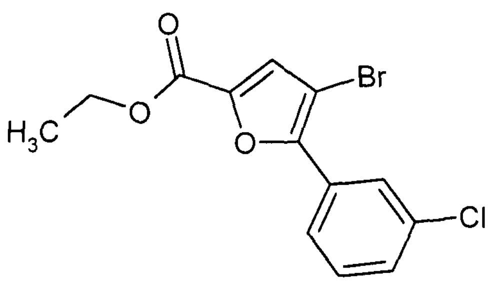

500 mg (1.68 mmol) der Verbindung aus Beispiel 2A und 276 mg (1.76 mmol) 3-Chlorphenylboronsäure werden unter Argon in 17 ml entgastem Toluol/Wasser (12:5) vorgelegt und mit 960 mg (9.06 mmol) Natriumcarbonat und 58.0 mg (0.05 mmol) Tetrakis(triphenylphosphin)palladium(0) versetzt. Es wird über Nacht bei 100°C gerührt. Die Phasen werden getrennt und die wässrige Phase einmal mit Essigsäureethylester extrahiert. Die organischen Phasen werden vereinigt, über Natriumsulfat getrocknet, filtriert und eingeengt. Das Rohprodukt wird über präparative HPLC (RP18-Säule; Eluent: Acetonitril/Wasser-Gradient) gereinigt. Man erhält 190 mg (33% d. Th.) der Titelverbindung.
$^1$H-NMR (400 MHz, DMSO-$d_6$): δ = *7.96-7.90* (m, 2H), *7.66* (s, 1H), *7.62-7.59* (m, 2H), 4.34 (q, 2H), 1.32 (t, 3H).
LC-MS (Methode 10): $R_t$ = 2.84 min; MS (ESIpos): m/z = 331 $[M+H]^+$.

**Beispiel 6A**

4-Brom-5-(3-chlor-4-fluorphenyl)furan-2-carbonsäureethylester

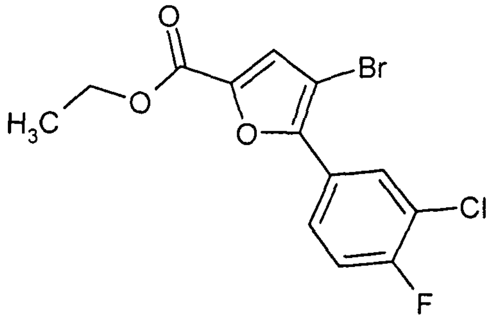

Die Herstellung der Titelverbindung erfolgt ausgehend von der Verbindung aus Beispiel 2A analog zur Synthese der Verbindung aus Beispiel 5A. Man erhält 0.84 g (72% d. Th.) der Titelverbindung.
$^1$H-NMR (400 MHz, DMSO-$d_6$): $\delta$ = *8.08* (dd, 1H), *7.95* (ddd, 1H), *7.66* (s, 1H), *7.64* (t, 1H), 4.34 (q, 2H), 1.31 (t, 3H).
LC-MS (Methode 1): $R_t$ = 3.08 min; MS (ESIpos): m/z = 349 $[M+H]^+$.

**Beispiel 7A**

4-Brom-5-(4-fluorphenyl)furan-2-carbonsäureethylester

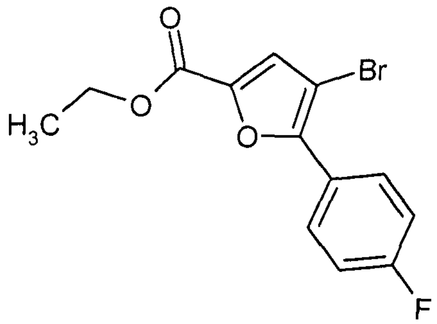

Die Herstellung der Titelverbindung erfolgt ausgehend von der Verbindung aus Beispiel 2A analog zur Synthese der Verbindung aus Beispiel 5A. Die Extraktion erfolgt mit Dichlormethan. Das Rohprodukt wird mittels Flash-Chromatographie (Laufmittel: Cyclohexan/Essigsäureethylester 20:1) gereinigt. Man erhält 13.8 g mit 79%iger Reinheit (76% d. Th.) der Titelverbindung.
$^1$H-NMR (400 MHz, DMSO-$d_6$): $\delta$ = 8.03-7.96 (m, 2H), 7.62 (s, 1H), 7.46-7.38 (m, 2H), 4.33 (q, 2H), 1.31 (t, 3H).
LC-MS (Methode 1): $R_t$ = 2.99 min; MS (ESIpos): m/z = 313 $[M+H]^+$.

**Beispiel 8A**

4-Brom-5-(3-cyanphenyl)furan-2-carbonsäureethylester

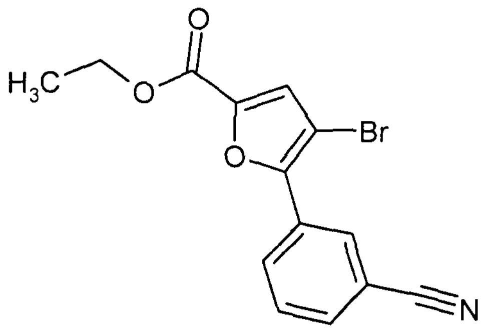

3.00 g (10.1 mmol) der Verbindung aus Beispiel 2A werden unter Argon in 96 ml 1,2-Dimethoxyethan vorgelegt und mit 1.63 g (11.1 mmol) 3-Cyanophenylboronsäure, 9.84 g (30.2 mmol) Cäsiumcarbonat, 0.14 g (0.30 mmol) Dicyclohexyl[2',4',6'-tri(propan-2-yl)biphenyl-2-yl]phosphan und 0.16 g (0.71 mmol) Palladium(II)acetat versetzt. Es wird über Nacht bei RT gerührt. Anschließend gibt man 0.44 g (3.02 mmol) 3-Cyanophenylboronsäure hinzu und rührt weitere 24 h bei RT. Man engt ein und reinigt das Rohprodukt mittels Flash-Chromatographie (Laufmittel: Cyclohexan/Essigsäureethylester 20:1). Man erhält 1.15 g (36% d. Th.) der Titelverbindung.
$^1$H-NMR (400 MHz, DMSO-$d_6$): $\delta$ = 8.31 (s, 1H), 8.25 (d, 1H), 7.99 (d, 1H), 7.79 (t, 1H), 7.69 (s, 1H), 4.35 (q, 2H), 1.31 (t, 3H).
LC-MS (Methode 5): $R_t$ = 1.34 min; MS (ESIpos): m/z = 320 $[M+H]^+$.

**Beispiel 9A**

4-(3-Chlorphenyl)furan-2-carbonsäureethylester

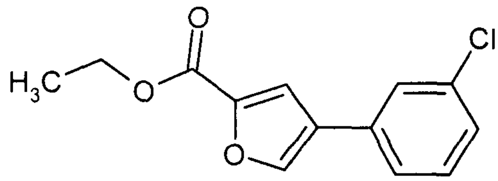

100 mg (0.46 mmol) der Verbindung aus Beispiel 3A werden unter Argon in 3 ml 1,2-Dimethoxyethan vorgelegt und mit 107 mg (0.69 mmol) 3-Chlorphenylboronsäure, 2.4 ml (2.28 mmol) wässriger Natriumcarbonat-Lösung (10%ig) und 32 mg (0.03 mmol) Tetrakis(triphenylphosphin)palladium(0) versetzt. Es wird 15 min bei 80°C unter Mikrowellenbestrahlung in einem geschlossenen Glasgefäß erhitzt. Das Rohprodukt wird über präparative HPLC (RP18-Säule; Eluent: Acetonitril/Wasser-Gradient) gereinigt. Man erhält 81.0 mg (71% d. Th.) der Titelverbindung.
$^1$H-NMR (400 MHz, DMSO-$d_6$): $\delta$ = 8.58 (s, 1H), 7.91 (s, 1H), 7.86 (t, 1H), 7.70 (d, 1H), 7.44 (t, 1H), 7.37 (d, 1H), 4.31 (q, 2H), 1.31 (t, 3H).
LC-MS (Methode 1): $R_t$ = 2.85 min; MS (ESIpos): m/z = 251 $[M+H]^+$.

**Beispiel 10A**

4-(3-Chlor-5-fluorphenyl)furan-2-carbonsäureethylester

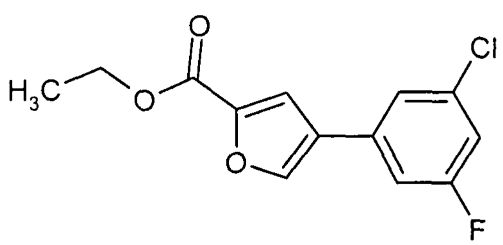

700 mg (3.20 mmol) der Verbindung aus Beispiel 3A und 613 mg (3.52 mmol) 5-Fluor-3-chlorphenylboronsäure werden unter Argon in 20 ml 1,2-Dimethoxyethan vorgelegt und mit 16.9 ml (15.98 mmol) wässriger Natriumcarbonat-Lösung (10%ig) und 37 mg (0.03 mmol) Tetrakis(triphenylphosphin)palladium(0) versetzt. Es wird 2 Stunden bei 80°C gerührt. Die Reaktionsmischung wird mit Wasser verdünnt und mit Essigsäureethylester extrahiert. Die organische Phase wird über Natriumsulfat getrocknet, filtriert, eingeengt und im Hochvakuum getrocknet. Man erhält 604 mg mit 75%iger Reinheit (53% d. Th.) der Titelverbindung.
$^1$H-NMR (400 MHz, DMSO-$d_6$): $\delta$ = 8.64 (s, 1H), 7.97 (s, 1H), 7.76 (s, 1H), 7.67 (d, 1H), 7.36 (dt, 1H), 4.32 (q, 2H), 1.31 (t, 3H).
LC-MS (Methode 1): $R_t$ = 2.75 min; MS (ESIpos): m/z = 269 $[M+H]^+$.

**Beispiel 11A**

4-(3-Chlor-5-fluorphenyl)-5-(3-cyanphenyl)furan-2-carbonsäureethylester

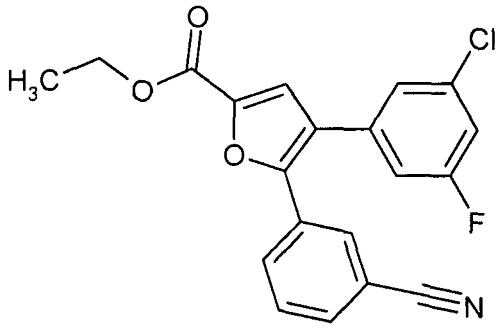

1.45 g (4.53 mmol) der Verbindung aus Beispiel 8A werden unter Argon in 50 ml 1,2-Dimethoxyethan vorgelegt und mit 0.79 g (4.53 mmol) 3-Chlor-5-fluorphenylboronsäure, 4.43 g (13.6 mmol) Cäsiumcarbonat, 0.15 g (0.32 mmol) Dicyclohexyl[2',4',6'-tri(propan-2-yl)biphenyl-2-yl]phosphan und 0.03 g (0.14 mmol) Palladium(II)acetat versetzt. Es wird über Nacht bei 50°C gerührt. Man engt ein und reinigt das Rohprodukt mittels Flash-Chromatographie (Laufmittel: Cyclohexan/Essigsäureethylester 20:1). Man erhält 1.05 g mit 68%iger Reinheit (43% d. Th.) der Titelverbindung.
LC-MS (Methode 7): $R_t$ = 2.57 min; MS (ESIpos): m/z = 370 $[M+H]^+$.

**Beispiel 12A**

5-(3-Chlor-4-fluorphenyl)-4-(3-chlor-5-fluorphenyl)furan-2-carbonsäureethylester

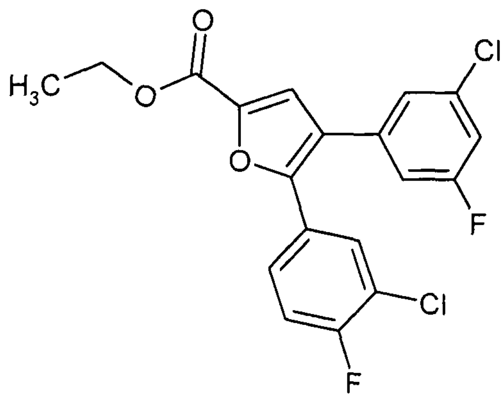

Die Herstellung der Titelverbindung erfolgt ausgehend von der Verbindung aus Beispiel 6A analog zur Synthese der Verbindung aus Beispiel 11A. Die Aufreinigung erfolgt mit dem Laufmittel: Cyclohexan/Essigsäureethylester (50:1). Man erhält 1.07 g mit 81%iger Reinheit (63% d. Th.) der Titelverbindung.

$^{1}$H-NMR (400 MHz, DMSO-$d_6$): $\delta$ = 7.74-7.68 (m, 2H), 7.57-7.45 (m, 3H), 7.38 (s, 1H), 7.32 (dt, 1H), 4.35 (q, 2H), 1.32 (t, 3H).
LC-MS (Methode 1): $R_t$ = 3.33 min; MS (ESIpos): m/z = 397 $[M+H]^+$.

**Beispiel 13A**

4-(3-Chlorphenyl)-5-(4-fluorphenyl)furan-2-carbonsäureethylester

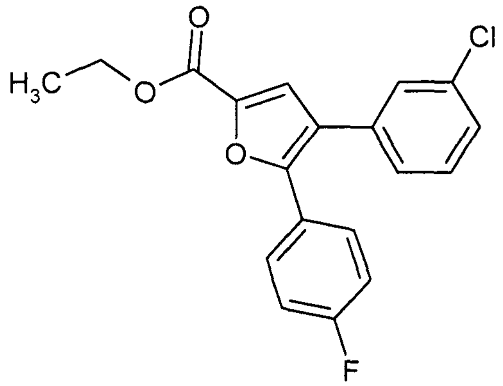

Die Herstellung der Titelverbindung erfolgt ausgehend von der Verbindung aus Beispiel 7A analog zur Synthese der Verbindung aus Beispiel 11A. Die Extraktion erfolgt mit Dichlormethan und die Aufreinigung mit dem Laufmittel: Cyclohexan/ Essigsäureethylester (30:1). Man erhält 4.12 g mit 50%iger Reinheit (41% d. Th.) der Titelverbindung.
LC-MS (Methode 1): $R_t$ = 3.26 min; MS (ESIpos): m/z = 345 $[M+H]^+$.

**Beispiel 14A**

4,5-Bis(3-chlorphenyl)furan-2-caröonsäureethylester

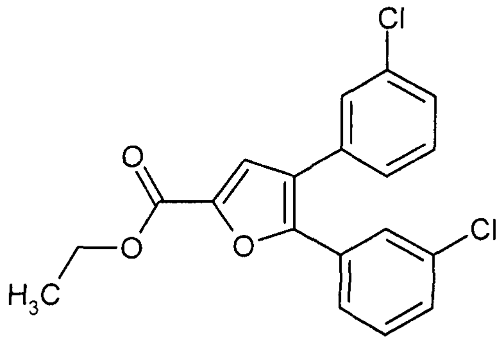

Die Herstellung der Titelverbindung erfolgt ausgehend von der Verbindung aus Beispiel 5A analog zur Synthese der Verbindung aus Beispiel 11A. Die Extraktion erfolgt mit Dichlormethan und die Aufreinigung über präparative HPLC (RP18-Säule; Eluent: Acetonitril/Wasser-Gradient). Man erhält 452 mg (34% d. Th.) der Titelverbindung.
$^{1}$H-NMR (400 MHz, DMSO-$d_6$): $\delta$ = 7.65 (s, 1H), 7.55-7.41 (m, 7H), 7.40-7.36 (m, 1H), 4.35 (q, 2H), 1.32 (t, 3H).
GC-MS (Methode 12): $R_t$ = 9.33 min; MS (EIpos): m/z = 360 $[M]^+$.

**Beispiel 15A**

5-(3-Chlor-4-fluorphenyl)-4-(3-cyan-5-fluorphenyl)furan-2-carbonsäureethylester

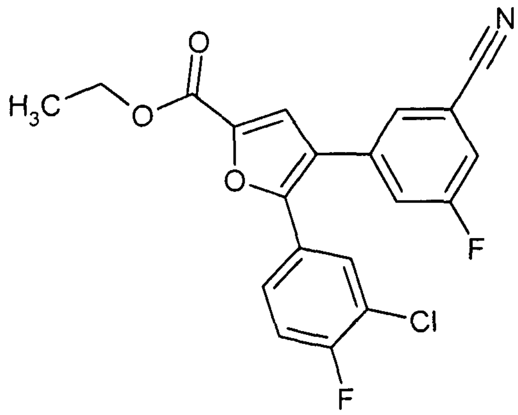

400 mg (1.15 mmol) der Verbindung aus Beispiel 6A und 313 mg (1.27 mmol) der Verbindung aus Beispiel 4A werden unter Argon in 11 ml 1,2-Dimethoxyethan vorgelegt und mit 1.12 g (3.45 mmol) Cäsiumcarbonat, 38.4 mg (0.08 mmol) Dicyclohexyl[2',4',6'-tri(propan-2-yl)biphenyl-2-yl]phosphan und 7.8 mg (0.04 mmol) Palladium(II)acetat versetzt. Es wird zwei Tage bei 50°C gerührt. Man engt ein und reinigt den Rückstand über präparative HPLC (RP18-Säule; Eluent: Acetonitril/Wasser-Gradient). Man erhält 34.0 mg (8% d. Th.) der Titelverbindung.
$^1$H-NMR (400 MHz, DMSO-$d_6$): $\delta$ = 7.90 (ddd, 1H), 7.83 (t, 1H), 7.76 (s, 1H), 7.74-7.68 (m, 2H), 7.53 (t, 1H), 7.47 (ddd, 1H), 4.35 (q, 2H), 1.32 (t, 3H).
LC-MS (Methode 1): $R_t$ = 3.04 min; MS (ESIpos): m/z = 388 $[M+H]^+$.

**Beispiel 16A**

5-(3-Chlorphenyl)-4-(3-cyan-5-fluorphenyl)furan-2-carbonsäureethylester

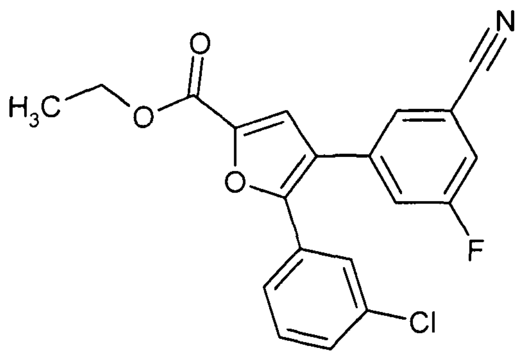

Die Herstellung der Titelverbindung erfolgt ausgehend von der Verbindung aus Beispiel 5A analog zur Synthese der Verbindung aus Beispiel 15A. Man erhält 111 mg (52% d. Th.) der Titelverbindung.
$^1$H-NMR (400 MHz, DMSO-$d_6$): $\delta$ = 7.90 (ddd, 1H), 7.83 (t, 1H), 7.75 (s, 1H), 7.70 (ddd, 1H), 7.57-7.53 (m, 2H), 7.49 (t, 1H), 7.42 (dt, 1H), 4.36 (q, 2H), 1.33 (t, 3H).
LC-MS (Methode 7): $R_t$ = 2.57 min; MS (ESIpos): m/z = 370 $[M+H]^+$.

**Beispiel 17A**

4-(3-Cyan-5-fluorphenyl)-5-(3-cyanphenyl)furan-2-carbonsäureethylester

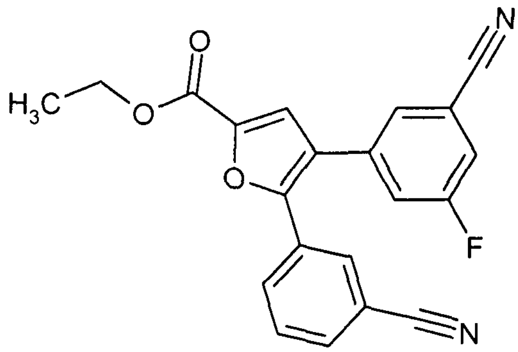

Die Herstellung der Titelverbindung erfolgt ausgehend von der Verbindung aus Beispiel 8A analog zur Synthese der Verbindung aus Beispiel 15A. Man erhält 190 mg (56% d. Th.) der Titelverbindung.
$^1$H-NMR (400 MHz, DMSO-$d_6$): δ = 7.98-7.93 (m, 2H), 7.91 (ddd, 1H), 7.82 (t, 1H), 7.81-7.76 (m, 2H), 7.72-7.64 (m, 2H), 4.36 (q, 2H), 1.33 (t, 3H).
LC-MS (Methode 1): $R_t$ = 2.71 min; MS (ESIpos): m/z = 361 $[M+H]^+$.

## Beispiel 18A

5-Brom-4-(3-chlorphenyl)furan-2-carbonsäureethylester

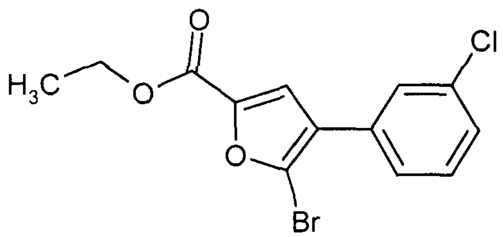

0.82 g (3.27 mmol) der Verbindung aus Beispiel 9A werden in 21 ml Essigsäure vorgelegt, mit 0.17 ml (3.27 mmol) Brom versetzt und über Nacht bei 60°C gerührt. Es wird vorsichtig gesättigte wässrige Natriumcarbonat-Lösung zugegeben und mit Essigsäureethylester extrahiert. Man wäscht die organische Phase zweimal mit Wasser, trocknet über Natriumsulfat, filtriert und engt ein. Das Rohprodukt wird mittels Flash-Chromatographie (Laufmittel: Cyclohexan/Essigsäureethylester 40:1) gereinigt. Man erhält 1.05 g (91% d. Th.) der Titelverbindung.
$^1$H-NMR (400 MHz, DMSO-$d_6$): δ = 7.85 (s, 1H), 7.80 (t, 1H), 7.71 (dt, 1H), 7.55-7.46 (m, 2H), 4.33 (q, 2H), 1.31 (t, 3H).
LC-MS (Methode 1): $R_t$ = 2.87 min; MS (ESIpos): m/z = 329 $[M+H]^+$.

## Beispiel 19A

5-Brom-4-(3-chlor-5-fluorphenyl)furan-2-carbonsäureethylester

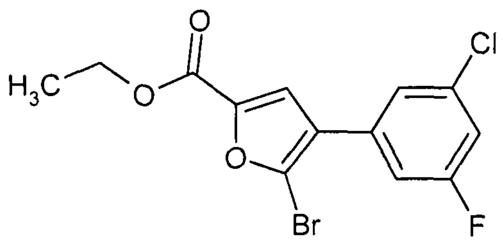

Die Herstellung der Titelverbindung erfolgt ausgehend von der Verbindung aus Beispiel 10A analog zur Synthese der Verbindung aus Beispiel 18A. Nach der Neutralisation mit gesättigter wässriger Natriumcarbonat-Lösung wird der entstehende Niederschlag abgesaugt, mit Wasser gewaschen und im Hochvakuum getrocknet. Man erhält 0.66 g mit 78%iger Reinheit (65% d. Th.) der Titelverbindung.
$^1$H-NMR (400 MHz, DMSO-$d_6$): $\delta$ = 7.92 (s, 1H), 7.73-7.68 (m, 1H), 7.62 (dt, 1H), 7.51 (dt, 1H), 4.33 (q, 2H), 1.31 (t, 3H).
LC-MS (Methode 1): $R_t$ = 2.99 min; MS (ESIpos): m/z = 347 $[M+H]^+$.

**Beispiel 20A**

4-(3-Chlor-5-fluorphenyl)-5-(3-cyanphenyl)furan-2-carbonsäure

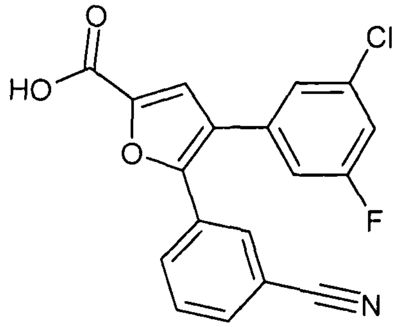

1.05 g (2.84 mmol) der Verbindung aus Beispiel 11A werden in 30 ml Tetrahydrofuran vorgelegt und bei Raumtemperatur mit 0.68 g (28.4 mmol) Lithiumhydroxid und 10 ml Wasser versetzt. Man rührt bei Raumtemperatur über Nacht, gibt anschließend 1N wässrige HCl-Lösung bis zu einem sauren pH-Wert hinzu, extrahiert mit Essigsäureethylester, wäscht die organische Phase mit Wasser, trocknet über Natriumsulfat, filtriert und engt ein. Das Rohprodukt wird über präparative HPLC (RP18-Säule; Eluent: Acetonitril/Wasser-Gradient) gereinigt. Man erhält 0.60 g (62% d. Th.) der Titelverbindung.
$^1$H-NMR (400 MHz, DMSO-$d_6$): $\delta$ = 13.5 (s, 1H), 7.97-7.89 (m, 2H), 7.81-7.76 (m, 1H), 7.67 (t, 1H), 7.62 (s, 1H), 7.49 (dt, 1H), 7.37 (s, 1H), 7.32 (dt, 1H).
LC-MS (Methode 1): $R_t$ = 2.58 min; MS (ESIpos): m/z = 342 $[M+H]^+$.

**Beispiel 21A**

5-(3-Chlor-4-fluorphenyl)-4-(3-chlor-5-fluorphenyl)furan-2-carbonsäure

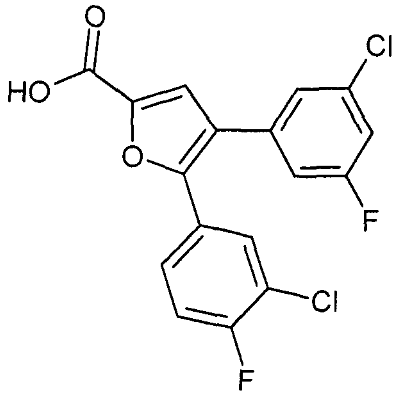

Die Herstellung der Titelverbindung erfolgt ausgehend von der Verbindung aus Beispiel 12A analog zur Synthese der Verbindung aus Beispiel 20A. Das Rohprodukt wird nach der Extraktion im Hochvakuum getrocknet und ohne chromatographische Aufreinigung weiter umgesetzt. Man erhält 0.65 g mit 75%iger Reinheit (80% d. Th.) der Titelverbindung.

$^1$H-NMR (400 MHz, DMSO-$d_6$): δ = 13.4 (s, 1H), 7.71 (dd, 1H), 7.60 (s, 1H), 7.56-7.44 (m, 3H), 7.37 (s, 1H), 7.32 (dt, 1H). LC-MS (Methode 5): $R_t$ = 1.43 min; MS (ESIneg): m/z = 367 $[M-H]^-$.

**Beispiel 22A**

5-Brom-4-(3-chlorphenyl)furan-2-carbonsäure

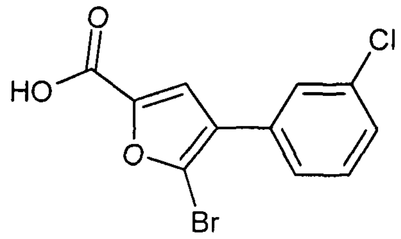

Die Herstellung der Titelverbindung erfolgt ausgehend von der Verbindung aus Beispiel 18A analog zur Synthese der Verbindung aus Beispiel 20A. Das Rohprodukt wird nach der Extraktion im Hochvakuum getrocknet und ohne chromatographische Aufreinigung weiter umgesetzt. Man erhält 47.0 mg (86% d. Th.) der Titelverbindung.
$^1$H-NMR (400 MHz, DMSO-$d_6$): δ = 13.6 (s, 1H), 7.78 (t, 1H), 7.74 (s, 1H), 7.69 (d, 1H), 7.52 (t, 1H), 7.47 (dt, 1H). LC-MS (Methode 1): $R_t$ = 2.70 min; MS (ESIpos): m/z = 301 $[M+H]^+$.

**Beispiel 23A**

5-Brom-4-(3-chlor-5-fluorphenyl)furan-2-carbonsäure

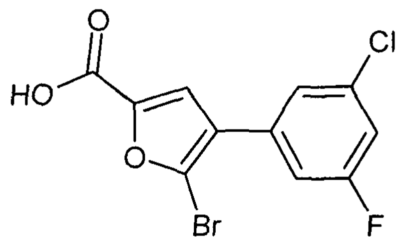

Die Herstellung der Titelverbindung erfolgt ausgehend von der Verbindung aus Beispiel 19A analog zur Synthese der Verbindung aus Beispiel 20A. Das Rohprodukt wird nach der Extraktion im Hochvakuum getrocknet und ohne chromatographische Aufreinigung weiter umgesetzt. Man erhält 590 mg mit 80%iger Reinheit der Titelverbindung.
$^1$H-NMR (400 MHz, DMSO-$d_6$): δ = 13.6 (s, 1H), 7.80 (s, 1H), 7.68 (s, 1H), 7.60 (dt, 1 H), 7.51 (dt, 1H). LC-MS (Methode 10): $R_t$ = 2.28 min; MS (ESIpos): m/z = 321 $[M+H]^+$.

**Beispiel 24A**

4-Brom-5-(3-chlorphenyl)furan-2-carbonsäure

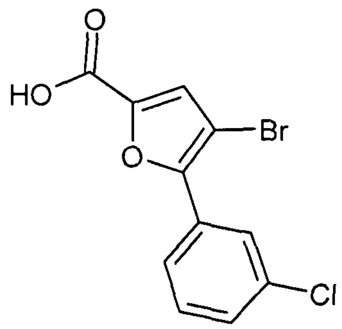

Die Herstellung der Titelverbindung erfolgt ausgehend von der Verbindung aus Beispiel 5A analog zur Synthese der Verbindung aus Beispiel 20A. Das Rohprodukt wird anstelle von Essigsäureethylester mit Dichlormethan extrahiert und ohne chromatographische Aufreinigung weiter umgesetzt. Man erhält 447 mg (98% d. Th.) der Titelverbindung.
$^1$H-NMR (400 MHz, DMSO-$d_6$): $\delta$ = 13.6 (s, 1H), 7.96-7.90 (m, 2H), 7.63-7.55 (m, 3H).
LC-MS (Methode 7): $R_t$ = 1.95 min; MS (ESIpos): m/z = 301 $[M+H]^+$.

**Beispiel 25A**

4,5-Bis(3-chlorphenyl)furan-2-carbonsäure

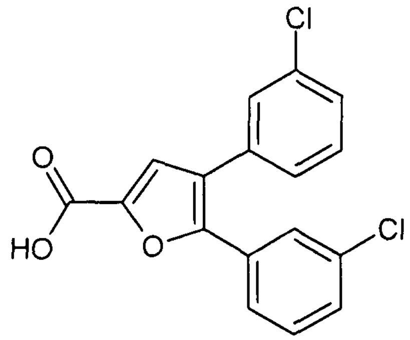

760 mg (2.10 mmol) der Verbindung aus Beispiel 14A werden in 20 ml Dioxan vorgelegt und bei Raumtemperatur mit 20 ml 2N wässriger Lithiumhydroxid-Lösung versetzt. Man rührt zwei Stunden bei 50°C und engt anschließend ein. Danach gibt man 1N wässrige HCl-Lösung bis zu einem sauren pH-Wert hinzu, extrahiert mit Dichlormethan, trocknet die organische Phase über Magnesiumsulfat, filtriert und engt ein. Man erhält 401 mg (57% d. Th.) der Titelverbindung.
$^1$H-NMR (400 MHz, DMSO-$d_6$): $\delta$ = 7.50 (s, 2H), 7.47-7.43 (m, 4H), 7.42-7.35 (m, 2H), 7.32 (s, 1H).
LC-MS (Methode 9): $R_t$ = 2.50 min; MS (ESIneg): m/z = 331 $[M-H]^-$.

**Beispiel 26A**

4-(3-Chlorphenyl)-5-(4-fluorphenyl)furan-2-carbonsäure

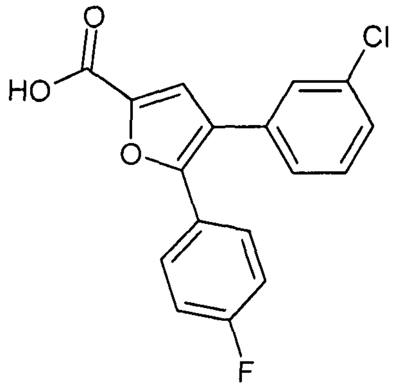

Die Herstellung der Titelverbindung erfolgt ausgehend von der Verbindung aus Beispiel 13A analog zur Synthese der Verbindung aus Beispiel 25A. Man erhält 4.03 g mit 52%iger Reinheit der Titelverbindung.
LC-MS (Methode 1): $R_t$ = 2.87 min; MS (ESIneg): m/z = 317 $[M-H]^-$.

**Beispiel 27A**

4-{[4-Brom-5-(3-chlorphenyl)furan-2-yl]carbonyl}piperazin-2-on

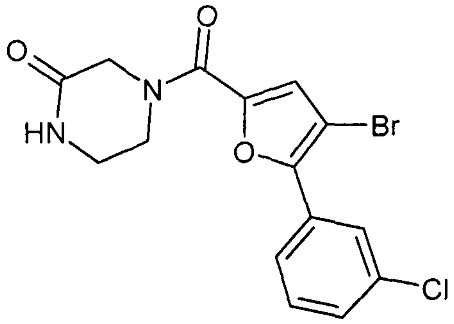

440 mg (1.46 mmol) der Verbindung aus Beispiel 24A, 161 mg (1.61 mmol) Piperazin-2-on und 1.14 g (2.19 mmol) PyBOP werden in 10 ml Tetrahydrofuran vorgelegt und bei Raumtemperatur mit 0.53 ml (3.06 mmol) N,N-Diisopropylethylamin versetzt. Man rührt bei Raumtemperatur über Nacht und reinigt anschließend die Reaktionsmischung über präparative HPLC (RP18-Säule; Eluent: Acetonitril/Wasser-Gradient). Man erhält 435 mg (78% d. Th.) der Titelverbindung.
$^1$H-NMR (400 MHz, DMSO-$d_6$): $\delta$ = 8.19 (s, 1H), 7.96-7.88 (m, 2H), 7.63-7.53 (m, 2H), 7.43 (s, 1H), 4.59-3.67 (m, 4H), 3.36-3.26 (m, 2H).
LC-MS (Methode 7): $R_t$ = 1.66 min; MS (ESIpos): m/z = 383 $[M+H]^+$.

**Beispiel 28A**

4-{[5-Brom-4-(3-chlorphenyl)furan-2-yl]carbonyl}piperazin-2-on

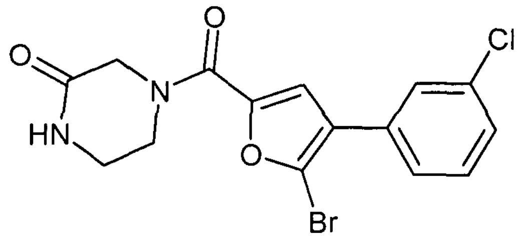

Die Herstellung der Titelverbindung erfolgt ausgehend von der Verbindung aus Beispiel 22A analog zur Synthese der Verbindung aus Beispiel 27A. Man erhält 48.0 mg (79% d. Th.) der Titelverbindung.
$^1$H-NMR (400 MHz, DMSO-$d_6$): $\delta$ = 8.18 (s, 1H), 7.80 (t, 1H), 7.71 (d, 1H), 7.60 (s, 1H), 7.53 (t, 1H), 7.49-7.45 (m, 1H), 4.35-4.00 (m, 2H), 3.93-3.77 (m, 2H), 3.33-3.27 (m, 2H).
LC-MS (Methode 7): $R_t$ = 1.64 min; MS (ESIpos): m/z = 383 $[M+H]^+$.

**Beispiel 29A**

1-{[4-Brom-5-(3-chlorphenyl)furan-2-yl]carbonyl}imidazolidin-4-on

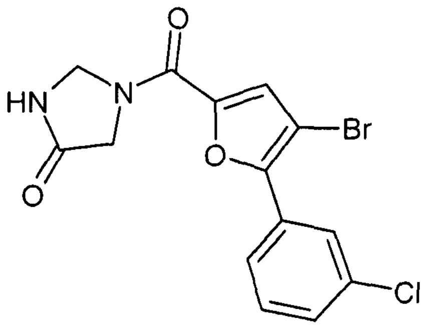

Die Herstellung der Titelverbindung erfolgt ausgehend von der Verbindung aus Beispiel 24A und der Verbindung aus Beispiel 35A analog zur Synthese der Verbindung aus Beispiel 27A. Es werden 1.1 Äquivalente PyBOP und 2.5 Äquivalente N,N-Diisopropylethylamin verwendet. Bei der Reaktion fällt das Produkt aus. Der Niederschlag wird abgesaugt, mit Tetrahydrofuran gewaschen und im Hochvakuum getrocknet. Man erhält 870 mg (71% d. Th.) der Titelverbindung.
$^1$H-NMR (400 MHz, DMSO-$d_6$): $\delta$ = 8.80-8.71 (m, 1H), 8.00-7.90 (m, 2H), 7.65-7.51 (m, 3H), 5.32 (s, 0.5H), 4.87 (s, 1.5H), 4.43 (s, 1.5H), 3.95 (s, 0.5H).
LC-MS (Methode 7): $R_t$ = 1.68 min; MS (ESIpos): m/z = 369 $[M+H]^+$.

**Beispiel 30A**

1-{[5-Brom-4-(3-chlorphenyl)furan-2-yl]carbonyl}imidazolidin-4-on

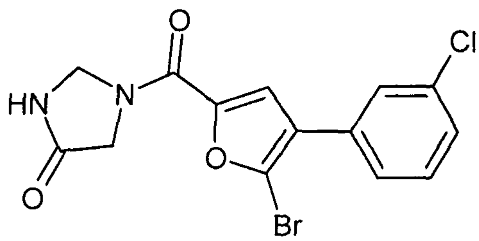

Die Herstellung der Titelverbindung erfolgt ausgehend von der Verbindung aus Beispiel 22A und der Verbindung

aus Beispiel 35A analog zur Synthese der Verbindung aus Beispiel 27A. Es werden 3.5 Äquivalente N,N-Diisopropylethylamin verwendet. Bei der Reaktion fällt das Produkt aus. Der Niederschlag wird abgesaugt, mit Tetrahydrofuran gewaschen und im Hochvakuum getrocknet. Man erhält 1.17 g (100% d. Th.) der Titelverbindung.
$^1$H-NMR (400 MHz, DMSO-$d_6$): $\delta$ = 8.77 (s, 1H), 7.87-7.64 (m, 3H), 7.56-7.44 (m, 2H), 5.22 (s, 0.5H), 4.85 (s, 1.5H), 4.35 (s, 1.5H), 3.94 (s, 0.5H).
LC-MS (Methode 1): $R_t$ = 2.06 min; MS (ESIpos): m/z = 369 $[M+H]^+$.

**Beispiel 31A**

1-{[5-Brom-4-(3-chlor-5-fluorphenyl)furan-2-yl]carbonyl}imidazolidin-4-on

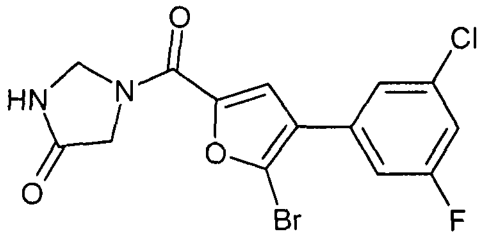

Die Herstellung der Titelverbindung erfolgt ausgehend von der Verbindung aus Beispiel 23A und der Verbindung aus Beispiel 35A analog zur Synthese der Verbindung aus Beispiel 27A. Es werden 5 Äquivalente N,N-Diisopropylethylamin verwendet. Durch Zugabe von Wasser in die Reaktionslösung wird das Produkt ausgefällt. Der Niederschlag wird abgesaugt, mit Tetrahydrofuran gewaschen und im Hochvakuum getrocknet. Man erhält 587 mg (97% d. Th.) der Titelverbindung.
LC-MS (Methode 1): $R_t$ = 2.15 min; MS (ESIpos): m/z = 389 $[M+H]^+$.

**Beispiel 32A**

$N^2$-Benzylglycinamid

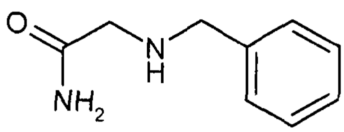

44.2 g (0.40 mol) Glycinamid-hydrochlorid werden in 2.2 1 Dichlormethan bei Raumtemperatur unter Argon vorgelegt, mit 112 ml (0.80 mol) Triethylamin versetzt und über Nacht bei Raumtemperatur gerührt. Dann werden 42.5 g (0.40 mol) Benzaldehyd zugegeben und die Reaktionsmischung wird über Nacht am Wasserabscheider unter Rückfluss erhitzt. Man engt ein, löst den Rückstand in 400 ml Tetrahydrofuran/Methanol (1:1), versetzt bei 0°C portionsweise mit 16.7 g (0.44 mol) Natriumborhydrid und rührt zwei Tage bei Raumtemperatur. Die Suspension wird abgesaugt, das Filtrat eingeengt und im Hochvakuum getrocknet. Der Rückstand wird in Essigsäureethylester gerührt, der Niederschlag abfiltriert, das Filtrat eingeengt und der Rückstand in Toluol über Nacht gerührt. Nach Filtration des Feststoffs und anschließendem Trocknen im Hochvakuum erhält man 56.5 g (84% d. Th.) der Titelverbindung.
$^1$H-NMR (400 MHz, DMSO-$d_6$): $\delta$ = 7.36-7.28 (m, 4H), 7.27-7.19 (m, 1H), 3.66 (d, 2H), 3.02 (d, 2H).
LC-MS (Methode 10): $R_t$ = 0.40 min; MS (ESIpos): m/z = 165 $[M+H]^+$.

**Beispiel 33A**

1-Benzyl-3-(hydroxymethyl)imidazolidin-4-on

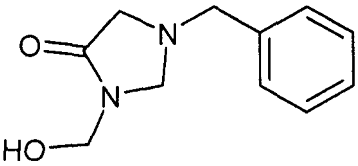

56.5 g (0.34 mol) der Verbindung aus Beispiel 32A werden mit 172 ml (6.20 mol) 37%iger Formaldehyd-Lösung versetzt und 30 Minuten unter Rückfluss erhitzt. Die Reaktionsmischung wird mit Dichlormethan extrahiert, und die vereinigten organischen Phasen werden über Natriumsulfat getrocknet, filtriert und eingeengt. Man erhält 74.5 g (100% d. Th.) der Titelverbindung.
LC-MS (Methode 5): $R_t$ = 0.51 min; MS (ESIpos): m/z = 207 $[M+H]^+$.

<u>**Beispiel 34A**</u>

1-Benzylimidazolidin-4-on-trifluoracetat

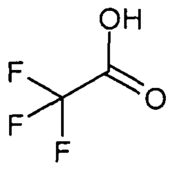

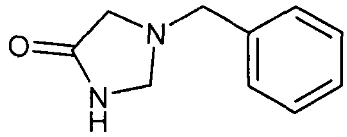

74.5 g (0.36 mol) der Verbindung aus Beispiel 33A werden für 6 h bei 150°C im Hochvakuum unter Abdestillieren flüchtiger Reaktionsprodukte erhitzt. Die Aufreinigung erfolgt durch HPLC (Säule: Sunfire C18 5$\mu$, 250 x 20 mm; Eluent: 0.2% Trifluoressigsäure/Wasser-Acetonitril-Gradient). Man erhält 28.4 g (27% d. Th.) der Titelverbindung.
$^1$H-NMR (400 MHz, DMSO-$d_6$): $\delta$ = 8.75 (s, 1H), 7.48-7.39 (m, 5H), 4.41 (s, 2H), 4.22 (s, 2H), 3.54 (s, 2H).
LC-MS (Methode 10): $R_t$ = 0.94 min; MS (ESIpos): m/z = 177 $[M-CF_3COOH+H]^+$.

<u>**Beispiel 35A**</u>

Imidazolidin-4-on-trifluoracetat

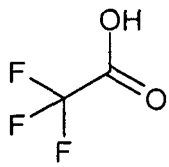

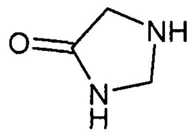

28.4 g (97.9 mmol) der Verbindung aus Beispiel 34A werden in 750 ml Ethanol gelöst und unter Argon mit 4.5 g (42.3 mmol) Palladium auf Aktivkohle (5%ig) versetzt. Es wird 24 h unter Wasserstoffatmosphäre hydriert. Die Suspension wird über Celite filtriert, eingeengt und am Hochvakuum getrocknet. Man erhält 19.2 g (98% d. Th.) der Titelverbindung.
$^1$H-NMR (400 MHz, DMSO-$d_6$): $\delta$ = 10.1 (s, 2H), 8.89 (s, 1H), 4.55 (s, 2H), 3.63 (s, 2H).
GC-MS (Methode 11): $R_t$ = 3.92 min; MS (EIpos): m/z = 86 $[M-CF_3COOH]^+$.

**Ausführungsbeispiele:**

**Beispiel 1**

3-[3-(3-Chlor-5-fluorphenyl)-5-(1,3-thiazolidin-3-ylcarbonyl)furan-2-yl]benzolcarbonitril

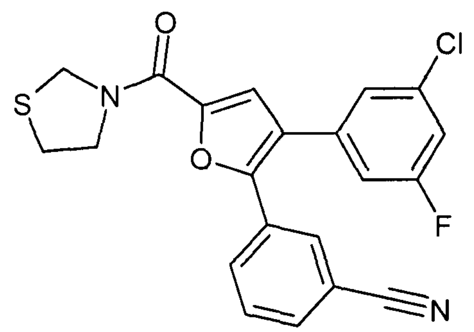

250 mg (0.73 mmol) der Verbindung aus Beispiel 20A, 71.8 mg (0.81 mmol) 1,3-Thiazolidin und 571 mg (1.10 mmol) PyBOP werden in 5 ml Tetrahydrofuran vorgelegt und bei Raumtemperatur mit 0.27 ml (1.54 mmol) N,N-Diisopropylethylamin versetzt. Man rührt bei Raumtemperatur über Nacht und reinigt anschließend die Reaktionsmischung über präparative HPLC (RP18-Säule; Eluent: Acetonitril/Wasser-Gradient). Man erhält 207 mg (65% d. Th.) der Titelverbindung.
$^1$H-NMR (400 MHz, DMSO-$d_6$): $\delta$ = 8.00 (s, 1H), 7.91 (dt, 1H), 7.80 (dt, 1H), 7.66 (t, 1H), 7.56 (s, 1H), 7.50 (dt, 1H), 7.39 (s, 1H), 7.32 (dt, 1H), 5.13-4.58 (m, 2H), 4.26-3.76 (m, 2H), 3.23-3.02 (m, 2H).
LC-MS (Methode 1): $R_t$ = 2.69 min; MS (ESIpos): m/z = 413 $[M+H]^+$.

**Beispiel 2**

[5-(3-Chlor-4-fluorphenyl)-4-(3-chlor-5-fluorphenyl)furan-2-yl](1,3-thiazolidin-3-yl)methanon

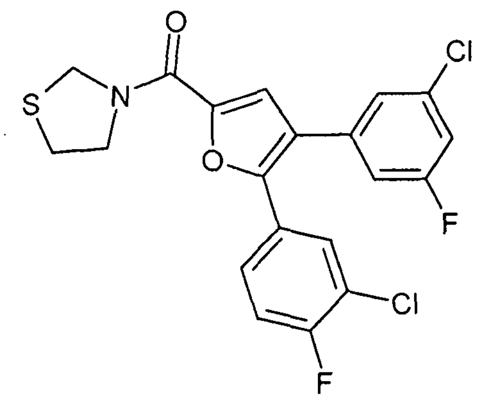

Die Herstellung der Titelverbindung erfolgt *ausgehend* von der Verbindung *aus* Beispiel 21A analog zur Synthese der Verbindung aus Beispiel 1. Man erhält 144 mg (48% d. Th.) der Titelverbindung.
$^1$H-NMR (400 MHz, DMSO-$d_6$): $\delta$ = 7.76-7.72 (m, 1H), 7.54 (s, 1H), 7.53-7.46 (m, 3H), 7.40 (s, 1H), 7.35-7.30 (m, 1H), 5.10-4.60 (m, 2H), 4.20-3.80 (m, 2H), 3.20-3.07 (m, 2H).
LC-MS (Methode 5): $R_t$ = 1.56 min; MS (ESIpos): m/z = 440 $[M+H]^+$.

**Beispiel 3**

3-{3-(3-Chlor-5-fluorphenyl)-5-[(1-oxido-1,3-thiazolidin-3-yl)carbonyl]furan-2-yl}benzolcarbonitril

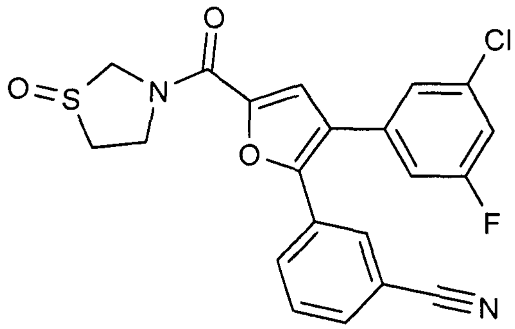

95.0 mg (0.23 mmol) der Verbindung aus Beispiel 1 werden in 3 ml Dichlormethan vorgelegt und mit 51.6 mg (0.23 mmol) meta-Chlorperbenzoesäure versetzt. Man rührt bei Raumtemperatur über Nacht, gibt anschließend gesättigte wässrige Natriumthiosulfat-Lösung hinzu und reinigt die Reaktionsmischung über präparative HPLC (RP18-Säule; Eluent: Acetonitril/Wasser-Gradient). Man erhält 63.0 mg (64% d. Th.) der Titelverbindung.
$^1$H-NMR (400 MHz, DMSO-$d_6$): $\delta$ = 8.04 (s, 1H), 7.92 (d, 1H), 7.81 (d, 1H), 7.66 (t, 1H), 7.68-7.56 (m, 1H), 7.51 (dt, 1H), 7.41 (s, 1H), 7.34 (d, 1H), 5.30-4.05 (m, 4H), 3.37-2.98 (m, 2H).
LC-MS (Methode 7): $R_t$ = 1.79 min; MS (ESIpos): m/z = 429 $[M+H]^+$.

**Beispiel 4**

[5-(3-Chlor-4-fluorphenyl)-4-(3-chlor-5-fluorphenyl)furan-2-yl](1-oxido-1,3-thiazolidin-3-yl)methanon

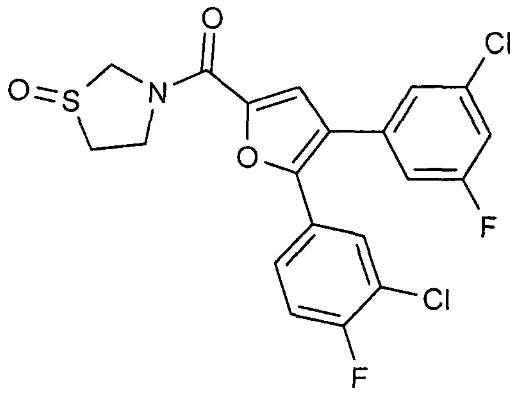

Die Herstellung der Titelverbindung erfolgt ausgehend von der Verbindung aus Beispiel 2 analog zur Synthese der Verbindung aus Beispiel 3. Man erhält 37.0 mg (89% d. Th.) der Titelverbindung.
$^1$H-NMR (400 MHz, DMSO-$d_6$): $\delta$ = 7.77 (d, 1H), 7.68-7.55 (m, 1H), 7.54-7.47 (m, 3H), 7.42 (s, 1H), 7.34 (dt, 1H), 5.25-4.90 (m, 1H), 4.87-4.70 (m, 0.5H), 4.60-4.00 (m, 2.5H), 3.40-2.95 (m, 2H).
LC-MS (Methode 1): $R_t$ = 2.56 min; MS (ESIpos): m/z = 456 $[M+H]^+$.

**Beispiel 5**

3-{3-(3-Chlor-5-fluorphenyl)-5-[(1,1-dioxido-1,3-thiazolidin-3-yl)carbonyl]furan-2-yl}benzolcarbonitril

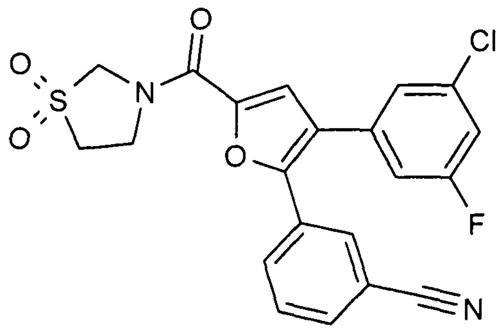

Die Herstellung der Titelverbindung erfolgt ausgehend von der Verbindung aus Beispiel 1 analog zur Synthese der Verbindung aus Beispiel 3. Anstelle von 1.0 Äquivalenten meta-Chlorperbenzoesäure werden 2.2 Äquivalente eingesetzt. Man erhält 80.0 mg (78% d. Th.) der Titelverbindung.
$^1$H-NMR (400 MHz, DMSO-$d_6$): $\delta$ = 8.06 (s, 1H), 7.92 (d, 1H), 7.81 (d, 1H), 7.69-7.61 (m, 2H), 7.51 (dt, 1H), 7.40 (s, 1H), 7.33 (dt, 1H), 5.40-4.00 (m, 4H), 3.65-3.50 (m, 2H).
LC-MS (Methode 7): $R_t$ = 2.08 min; MS (ESIpos): m/z = 445 [M+H]$^+$.

**Beispiel 6**

4-{[4-(3-Chlorphenyl)-5-(4-fluorphenyl)furan-2-yl]carbonyl}piperazin-2-on

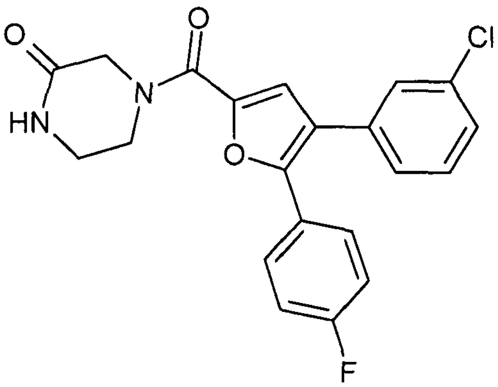

200 mg (0.63 mmol) der Verbindung aus Beispiel 26A, 94.8 mg (0.95 mmol) Piperazin-2-on und 493 mg (0.95 mmol) PyBOP werden in 11 ml Tetrahydrofuran vorgelegt und bei Raumtemperatur mit 0.23 ml (1.33 mmol) N,N-Diisopropylethylamin versetzt. Man rührt bei Raumtemperatur über Nacht und reinigt anschließend die Reaktionsmischung über präparative HPLC (RP18-Säule; Eluent: Acetonitril/Wasser-Gradient). Man erhält 52.0 mg (21% d. Th.) der Titelverbindung.
$^1$H-NMR (400 MHz, DMSO-$d_6$): $\delta$ = 8.19 (s, 1H), 7.58-7.50 (m, 3H), 7.46-7.43 (m, 2H), 7.41 (s, 1H), 7.39-7.35 (m, 1H), 7.33-7.27 (m, 2H), 4.63-4.08 (m, 2H), 4.04-3.75 (m, 2H), 3.36-3.30 (m, 2H).
LC-MS (Methode 1): $R_t$ = 2.43 min; MS (ESIpos): m/z = 399 [M+H]$^+$.

**Beispiel 7**

4-{[5-(3-Chlor-4-fluorphenyl)-4-(3-chlor-5-fluorphenyl)furan-2-yl]carbonyl}piperazin-2-on

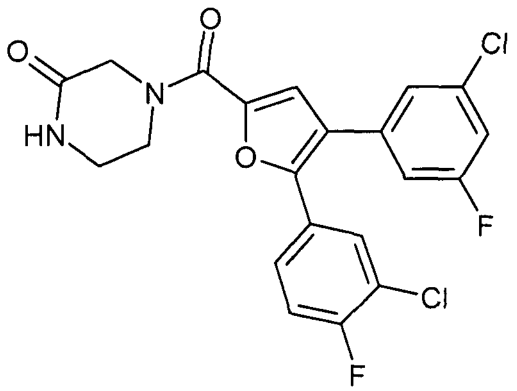

Die Herstellung der Titelverbindung erfolgt ausgehend von der Verbindung aus Beispiel 21A analog zur Synthese der Verbindung aus Beispiel 6. Anstelle von 1.5 Äquivalenten Piperazin-2-on werden 1.1 Äquivalente eingesetzt. Man erhält 33.0 mg (53% d. Th.) der Titelverbindung.
$^1$H-NMR (400 MHz, DMSO-$d_6$): δ = 8.19 (s, 1H), 7.71 (dd, 1H), 7.57-7.45 (m, 4H), 7.40 (s, 1H), 7.33 (dt, 1H), 4.52-4.05 (m, 2H), 4.02-3.76 (m, 2H), 3.38-3.26 (m, 2H).
LC-MS (Methode 5): $R_t$ = 1.31 min; MS (ESIpos): m/z = 451 $[M+H]^+$.

**Beispiel 8**

3-{3-(3-Chlor-5-fluorphenyl)-5-[(3-oxopiperazin-1-yl)carbonyl]furan-2-yl}benzolcarbonitril

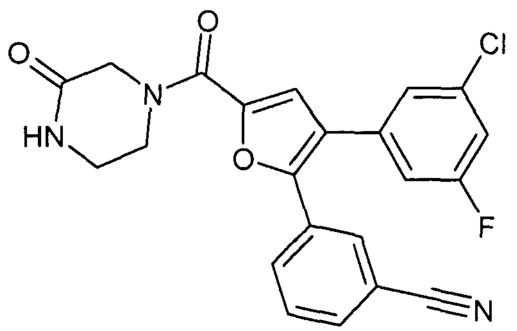

Die Herstellung der Titelverbindung erfolgt ausgehend von der Verbindung aus Beispiel 20A analog zur Synthese der Verbindung aus Beispiel 6. Anstelle von 1.5 Äquivalenten Piperazin-2-on werden 1.1 Äquivalente eingesetzt. Man erhält 55.0 mg (89% d. Th.) der Titelverbindung.
$^1$H-NMR (400 MHz, DMSO-$d_6$): δ = 8.20 (s, 1H), 7.96 (s, 1H), 7.90 (d, 1H), 7.78 (d, 1H), 7.66 (t, 1H), 7.53-7.44 (m, 2H), 7.40 (s, 1H), 7.33 (dt, 1H), 4.50-3.80 (m, 4H), 3.36-3.29 (m, 2H).
LC-MS (Methode 1): $R_t$ = 2.19 min; MS (ESIpos): m/z = 424 $[M+H]^+$.

**Beispiel 9**

[5-(3-Chlor-4-fluorphenyl)-4-(3-chlor-5-fluorphenyl)furan-2-yl](morpholin-4-yl)methanon

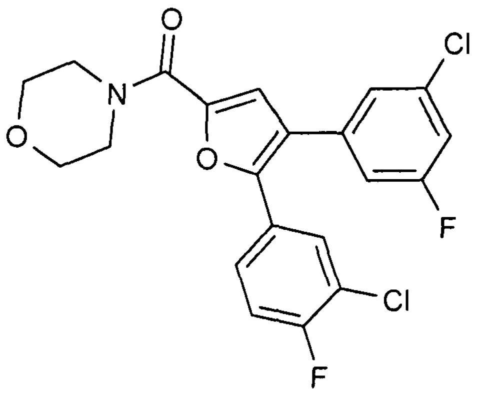

Die Herstellung der Titelverbindung erfolgt ausgehend von der Verbindung aus Beispiel 21A analog zur Synthese der Verbindung aus Beispiel 6. Es werden 1.5 Äquivalente Morpholin eingesetzt. Man erhält 205 mg (59% d. Th.) der Titelverbindung.
$^1$H-NMR (400 MHz, DMSO-$d_6$): δ = 7.69 (dd, 1H), 7.55-7.43 (m, 3H), 7.41-7.37 (dt, 2H), 7.32 (dt, 1H), 3.88-3.61 (m, 8H).
LC-MS (Methode 7): $R_t$ = 2.45 min; MS (ESIpos): m/z = 438 [M+H]$^+$.

**Beispiel 10**

3-[3-(3-Chlor-5-fluorphenyl)-5-([(3R)-3-hydroxypyrrolidin-1-yl]carbonyl}furan-2-yl]benzolcarbonitril

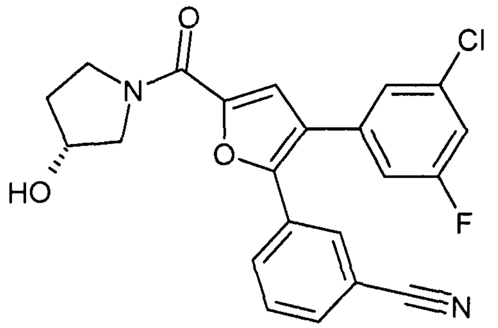

Die Herstellung der Titelverbindung erfolgt ausgehend von der Verbindung aus Beispiel 20A analog zur Synthese der Verbindung aus Beispiel 6. Es werden 1.1 Äquivalente (3R)-Pyrrolidin-3-ol eingesetzt. Man erhält 51.0 mg (85% d. Th.) der Titelverbindung.
$^1$H-NMR (400 MHz, DMSO-$d_6$): δ = 7.95 (s, 1H), 7.90 (d, 1H), 7.82-7.76 (m, 1H), 7.66 (t, 1H), 7.52-7.44 (m, 2H), 7.39 (s, 1H), 7.33 (dt, 1H), 5.04 (d, 1H), 4.41 (s, 0.5H), 4.32 (s, 0.5H), 4.02-3.90 (m, 1.5H), 3.75 (d, 0.5H), 3.65-3.44 (m, 2H), 2.06-1.78 (m, 2H).
LC-MS (Methode 1): $R_t$ = 2.26 min; MS (ESIpos): m/z = 411 [M+H]$^+$.

**Beispiel 11**

1-{[5-(3-Chlor-4-fluorphenyl)-4-(3-chlor-5-fluorphenyl)furan-2-yl]carbonyl}imidazolidin-4-on

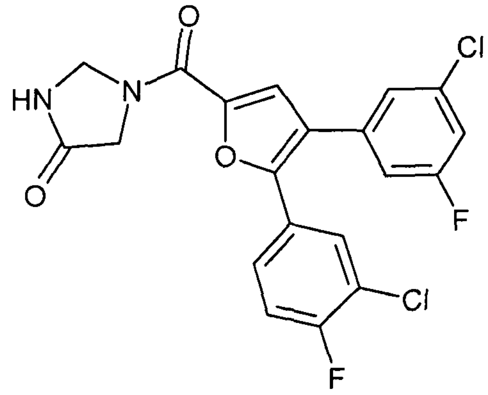

Die Herstellung der Titelverbindung erfolgt ausgehend von der Verbindung aus Beispiel 21A und der Verbindung aus Beispiel 35A analog zur Synthese der Verbindung aus Beispiel 6. Es werden 1.1 Äquivalente der Verbindung aus Beispiel 35A verwendet. Man erhält 203 mg (60% d. Th.) der Titelverbindung.
$^1$H-NMR (400 MHz, DMSO-$d_6$): $\delta$ = 8.77 (s, 1H), 7.76 (dd, 1H), 7.61 (s, 1H), 7.58-7.45 (m, 3H), 7.43-7.38 (m, 1H), 7.34 (d, 1H), 5.34 (s, 0.5H), 4.88 (s, 1.5H), 4.45 (s, 1.5H), 3.97 (s, 0,5H).
LC-MS (Methode 7): $R_t$ = 2.11 min; MS (ESIpos): m/z = 437 [M+H]$^+$.

**Beispiel 12**

3-{3-(3-Chlor-5-fluorphenyl)-5-[(4-oxoimidazolidin-1-yl)carbonyl]furan-2-yl}benzol-carbonitril

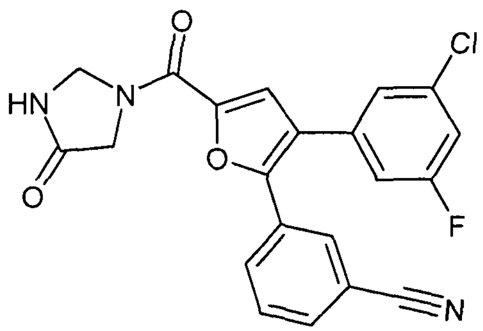

Die Herstellung der Titelverbindung erfolgt ausgehend von der Verbindung aus Beispiel 20A und der Verbindung aus Beispiel 35A analog zur Synthese der Verbindung aus Beispiel 6. Es werden 1.1 Äquivalente der Verbindung aus Beispiel 35A verwendet. Man erhält 42.0 mg (70% d. Th.) der Titelverbindung.
$^1$H-NMR (400 MHz, DMSO-$d_6$): $\delta$ = 8.78 (s, 1H), 8.03 (s, 1H), 7.92 (d, 1H), 7.80 (t, 1H), 7.71-7.57 (m, 2H), 7.50 (dt, 1H), 7.43-7.37 (m, 1H), 7.34 (d, 1H), 5.37 (s, 0.5H), 4.89 (s, 1.5H), 4.48 (s, 1.5H), 3.98 (s, 0.5H).
LC-MS (Methode 1): $R_t$ = 2.21 min; MS (ESIpos): m/z = 410 [M+H]$^+$.

**Beispiel 13**

4-{[4-(5-Chlor-2-fluorphenyl)-5-(3-chlorphenyl)furan-2-yl]carbonyl}piperazin-2-on

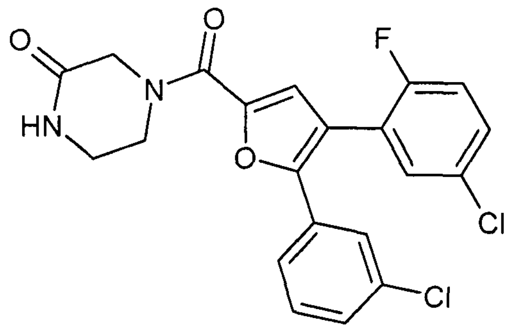

Die Herstellung der Titelverbindung erfolgt ausgehend von der Verbindung aus Beispiel 27A analog zur Synthese der Verbindung aus Beispiel 9A. Man erhält 2.4 mg (4% d. Th.) der Titelverbindung.
$^1$H-NMR (400 MHz, DMSO-$d_6$): $\delta$ = 8.19 (s, 1H), 7.67-6.63 (m, 1H), 7.60-7.55 (m, 1H), 7.50-7.45 (m, 3H), 7.44-7.35 (m, 3H), 4.58-3.73 (m, 4H), 3.38-3.29 (m, 2H).
LC-MS (Methode 10): $R_t$ = 2.30 min; MS (ESIpos): m/z = 433 $[M+H]^+$.

**Beispiel 14**

4-{[4-(3-Chlor-5-fluorphenyl)-5-(3-chlorphenyl)furan-2-yl]carbonyl}piperazin-2-on

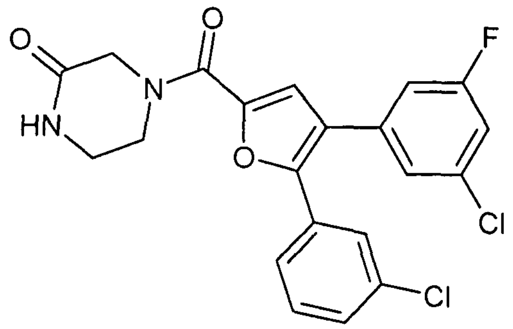

Die Herstellung der Titelverbindung erfolgt ausgehend von der Verbindung aus Beispiel 27A analog zur Synthese der Verbindung aus Beispiel 9A. Man erhält 36.3 mg (64% d. Th.) der Titelverbindung.
$^1$H-NMR (400 MHz, DMSO-$d_6$): $\delta$ = 8.22-8.17 (m, 1H), 7.55-7.53 (m, 1H), 7.53-7.41 (m, 5H), 7.41-7.38 (m, 1H), 7.33 (dt, 1H), 4.50-3.80 (m, 4H), 3.35-3.29 (m, 2H).
LC-MS (Methode 10): $R_t$ = 2.39 min; MS (ESIpos): m/z = 433 $[M+H]^+$.

**Beispiel 15**

1-{[5-(3-Chlorphenyl)-4-(3,5-difluorphenyl)furan-2-yl]carbonyl}imidazolidin-4-on

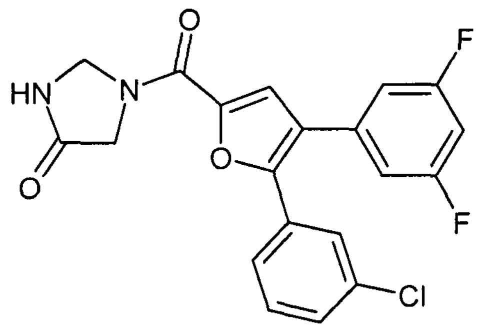

Die Herstellung der Titelverbindung erfolgt ausgehend von der Verbindung aus Beispiel 29A analog zur Synthese der Verbindung aus Beispiel 9A. Anstelle von 1.5 Äquivalenten Boronsäure werden 1.1 Äquivalente eingesetzt und die Mikrowellenreaktion wird 30 Minuten bei 120°C durchgeführt. Man erhält 51.0 mg (94% d. Th.) der Titelverbindung.
$^1$H-NMR (400 MHz, DMSO-$d_6$): $\delta$ = 8.80-8.73 (m, 1H), 7.62-7.44 (m, 5H), 7.30 (tt, 1H), 7.25-7.17 (m, 2H), 5.34 (s, 0.5H), 4.88 (s, 1.5H), 4.45 (s, 1.5H), 3.97 (s, 0.5H).
LC-MS (Methode 1): $R_t$ = 2.34 min; MS (ESIpos): m/z = 403 $[M+H]^+$.

## Beispiel 16

1-{[4-(3-Chlor-5-fluorphenyl)-5-(3-chlorphenyl)furan-2-yl]carbonyl}imidazolidin-4-on

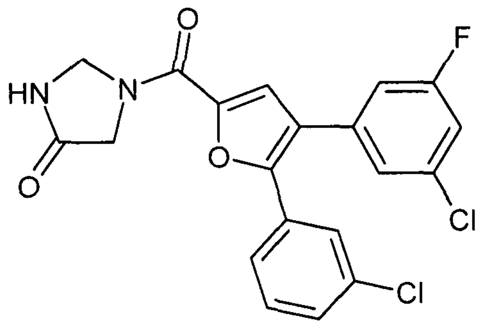

Die Herstellung der Titelverbindung erfolgt ausgehend von der Verbindung aus Beispiel 29A analog zur Synthese der Verbindung aus Beispiel 9A. Anstelle von 1.5 Äquivalenten Boronsäure werden 1.1 Äquivalente eingesetzt und die Mikrowellenreaktion wird 30 Minuten bei 120°C durchgeführt. Man erhält 50.0 mg (88% d. Th.) der Titelverbindung.
$^1$H-NMR (400 MHz, DMSO-$d_6$): $\delta$ = 8.80-8.74 (m, 1H), 7.64-7.55 (m, 2H), 7.54-7.44 (m, 4H), 7.43-7.38 (m, 1H), 7.34 (d, 1H), 5.34 (s, 0.5H), 4.88 (s, 1.5H), 4.45 (s, 1.5H), 3.97 (s, 0.5H).
LC-MS (Methode 1): $R_t$ = 2.46 min; MS (ESIpos): m/z = 419 $[M+H]^+$.

## Beispiel 17

4-{[5-(3-Chlorphenyl)-4-(3,5-difluorphenyl)furan-2-yl]carbonyl}piperazin-2-on

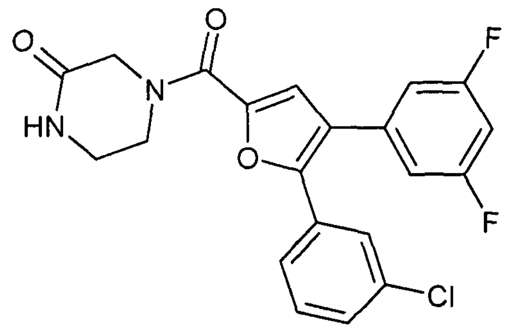

Die Herstellung der Titelverbindung erfolgt ausgehend von der Verbindung aus Beispiel 27A analog zur Synthese der Verbindung aus Beispiel 9A. Die Mikrowellenreaktion wird 5 min bei 80°C durchgeführt. Man erhält 42.9 mg (79% d. Th.) der Titelverbindung.
$^1$H-NMR (400 MHz, DMSO-$d_6$): $\delta$ = 8.19 (s, 1H), 7.56-7.41 (m, 5H), 7.30 (tt, 1H), 7.24-7.17 (m, 2H), 4.55-3.75 (m, 4H), 3.37-3.28 (m, 2H).
LC-MS (Methode 10): $R_t$ = 2.26 min; MS (ESIpos): m/z = 417 $[M+H]^+$.

**Beispiel 18**

4-{[4-(3-Chlorphenyl)-5-(3-methylphenyl)furan-2-yl]carbonyl}piperazin-2-on

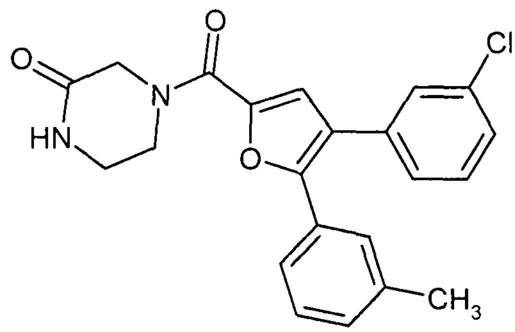

50.0 mg (0.13 mmol) der Verbindung aus Beispiel 28A und 19.5 mg (0.14 mmol) (3-Methylphenyl)boronsäure werden unter Argon in 1 ml 1,2-Dimethoxyethan vorgelegt und mit 0.69 ml (0.65 mmol) 10%iger wässriger Natriumcarbonat-Lösung und 4.5 mg (0.004 mmol) Tetrakis(triphenylphosphin)palladium(0) versetzt. Man rührt 2 Stunden bei 80°C und reinigt anschließend die Reaktionsmischung über präparative HPLC (RP18-Säule; Eluent: Acetonitril/Wasser-Gradient). Man erhält 28.0 mg (54% d. Th.) der Titelverbindung.
$^1$H-NMR (400 MHz, DMSO-$d_6$): $\delta$ = 8.19 (s, 1H), 7.52 (d, 1H), 7.45-7.35 (m, 5H), 7.34-7.17 (m, 3H), 4.49-4.06 (m, 2H), 4.03-3.70 (m, 2H), 3.36-3.28 (m, 2H), 2.29 (s, 3H).
LC-MS (Methode 5): $R_t$ = 1.25 min; MS (ESIpos): m/z = 395 $[M+H]^+$.

**Beispiel 19**

4-{[4-(3-Chlorphenyl)-5-(3,4-difluorphenyl)furan-2-yl]carbonyl}piperazin-2-on

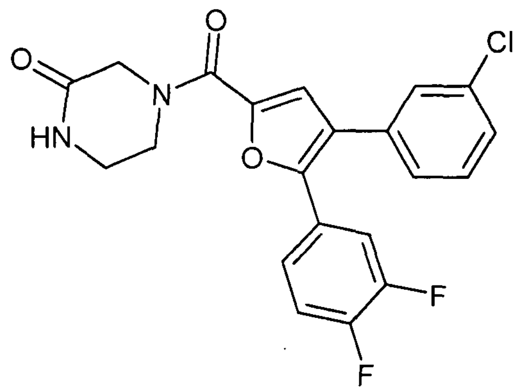

Die Herstellung der Titelverbindung erfolgt ausgehend von der Verbindung aus Beispiel 28A analog zur Synthese der Verbindung aus Beispiel 17. Anstelle von 1.1 Äquivalenten Boronsäure werden 1.5 Äquivalente eingesetzt. Man erhält 14.0 mg (12% d. Th.) der Titelverbindung.
$^1$H-NMR (400 MHz, DMSO-$d_6$): $\delta$ = 8.19 (s, 1H), 7.58-7.49 (m, 3H), 7.48-7.43 (m, 2H), 7.42 (s, 1H), 7.40-7.36 (m, 1H), 7.33-7.28 (m, 1H), 4.60-4.06 (m, 2H), 4.04-3.71 (m, 2H), 3.37-3.27 (m, 2H).
LC-MS (Methode 1): $R_t$ = 2.35 min; MS (ESIpos): m/z = 417 $[M+H]^+$.

**Beispiel 20**

3-{3-(3-Chlorphenyl)-5-[(3-oxopiperazin-1-yl)carbonyl]furan-2-yl}benzolcarbonitril

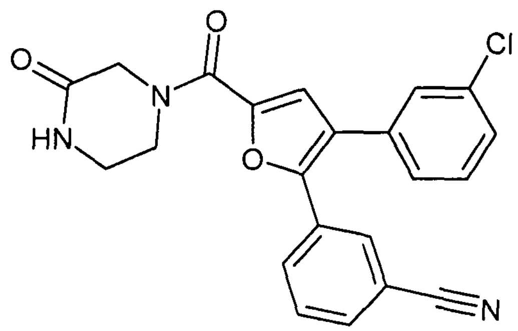

Die Herstellung der Titelverbindung erfolgt ausgehend von der Verbindung aus Beispiel 28A analog zur Synthese der Verbindung aus Beispiel 18. Man erhält 36.0 mg (66% d. Th.) der Titelverbindung.
$^1$H-NMR (400 MHz, DMSO-$d_6$): $\delta$ = 8.20 (s, 1H), 7.91 (s, 1H), 7.88 (d, 1H), 7.77 (d, 1H), 7.64 (t, 1H), 7.56 (s, 1H), 7.49-7.42 (m, 3H), 7.40-7.36 (m, 1H), 4.55-3.80 (m, 4H), 3.35-3.27 (m, 2H).
LC-MS (Methode 5): $R_t$ = 1.12 min; MS (ESIpos): m/z = 406 $[M+H]^+$.

**Beispiel 21**

4-{[4-(3-Chlorphenyl)-5-(4-fluor-3-methylphenyl)furan-2-yl]carbonyl}piperazin-2-on

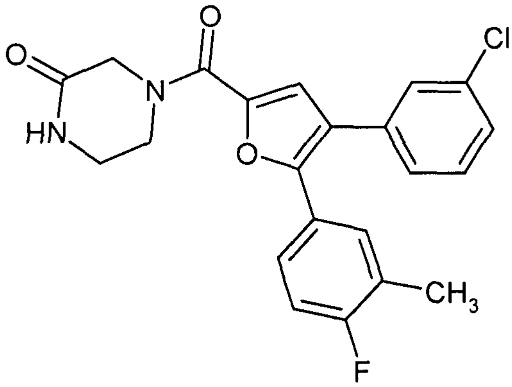

Die Herstellung der Titelverbindung erfolgt ausgehend von der Verbindung aus Beispiel 28A analog zur Synthese der Verbindung aus Beispiel 18. Man erhält 17.0 mg (32% d. Th.) der Titelverbindung.
$^1$H-NMR (400 MHz, DMSO-$d_6$): δ = 8.19 (s, 1H), 7.53 (d, 1H), 7.49-7.40 (m, 4H), 7.39-7.34 (m, 1H), 7.32-7.27 (m, 1H), 7.21 (t, 1H), 4.40-3.85 (m, 4H), 3.36-3.27 (m, 2H), 2.24-2.21 (m, 3H).
LC-MS (Methode 7): $R_t$ = 2.00 min; MS (ESIpos): m/z = 413 $[M+H]^+$.

**Beispiel 22**

4-{[5-(3-Chlor-4-fluorphenyl)-4-(3-chlorphenyl)furan-2-yl]carbonyl}piperazin-2-on

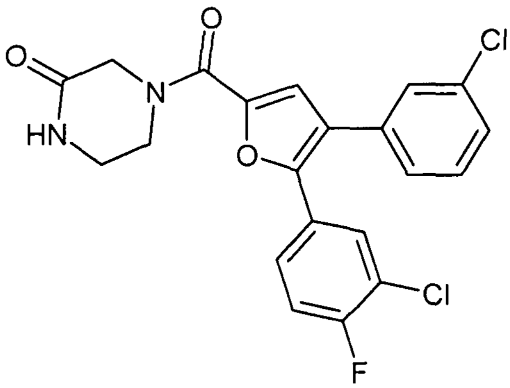

Die Herstellung der Titelverbindung erfolgt ausgehend von der Verbindung aus Beispiel 28A analog zur Synthese der Verbindung aus Beispiel 18. Anstelle von 1.1 Äquivalenten Boronsäure werden 1.5 Äquivalente eingesetzt. Man erhält 18.0 mg (33% d. Th.) der Titelverbindung.
$^1$H-NMR (400 MHz, DMSO-$d_6$): δ = 8.19 (s, 1H), 7.66 (dd, 1H), 7.56 (s, 1H), 7.54-7.44 (m, 4H), 7.43 (s, 1H), 7.40-7.35 (m, 1H), 4.55-3.75 (m, 4H), 3.36-3.28 (m, 2H).
LC-MS (Methode 1): $R_t$ = 2.63 min; MS (ESIpos): m/z = 433 $[M+H]^+$.

**Beispiel 23**

5-{3-(3-Chlor-5-fluorphenyl)-5-[(4-oxoimidazolidin-1-yl)carbonyl]furan-2-yl}-2-fluor-benzolcarbonitril

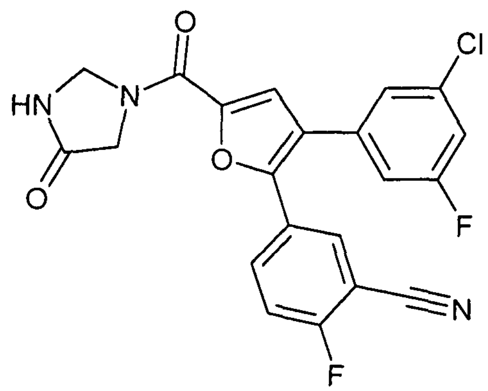

Die Herstellung der Titelverbindung erfolgt ausgehend von der Verbindung aus Beispiel 31A analog zur Synthese der Verbindung aus Beispiel 18. Anstelle von 0.03 Äquivalenten Katalysator werden 0.06 Äquivalente eingesetzt. Man erhält 2.0 mg (2% d. Th.) der Titelverbindung.
$^1$H-NMR (400 MHz, DMSO-$d_6$): $\delta$ = 8.77 (s, 1H), 8.17 (dd, 1H), 7.89-7.80 (m, 1H), 7.67-7.57 (m, 2H), 7.49 (dt, 1H), 7.42 (s, 1H), 7.33 (d, 1H), 5.36 (s, 0.5H), 4.89 (s, 1.5H), 4.47 (s, 1.5H), 3.97 (s, 0.5H).
LC-MS (Methode 1): $R_t$ = 2.30 min; MS (ESIpos): m/z = 428 $[M+H]^+$.

**Beispiel 24**

5-{3-(3-Chlorphenyl)-5-[(3-oxopiperazin-1-yl)carbonyl]furan-2-yl}-2-fluorbenzolcarbonitril

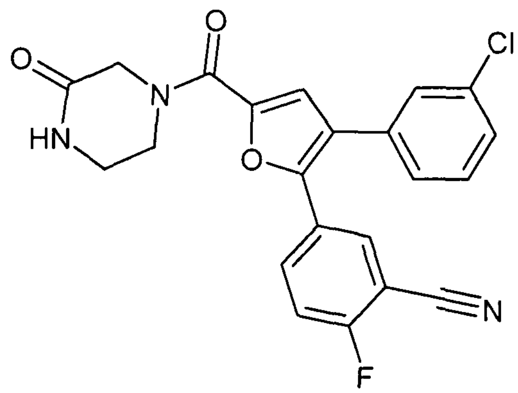

Die Herstellung der Titelverbindung erfolgt ausgehend von der Verbindung aus Beispiel 28A analog zur Synthese der Verbindung aus Beispiel 18. Man erhält 8.0 mg (14% d. Th.) der Titelverbindung.
$^1$H-NMR (400 MHz, DMSO-$d_6$): $\delta$ = 8.20 (s, 1H), 8.04 (dd, 1H), 7.84-7.77 (m, 1H), 7.61 (t, 1H), 7.58-7.54 (m, 1H), 7.50-7.42 (m, 3H), 7.36 (dt, 1H), 4.50-3.80 (m, 4H), 3.34-3.28 (m, 2H).
LC-MS (Methode 1): $R_t$ = 2.30 min; MS (ESIpos): m/z = 424 $[M+H]^+$.

**Beispiel 25**

1-{[5-(3-Chlor-4-fluorphenyl)-4-(3-chlorphenyl)furan-2-yl]carbonyl}imidazolidin-4-on

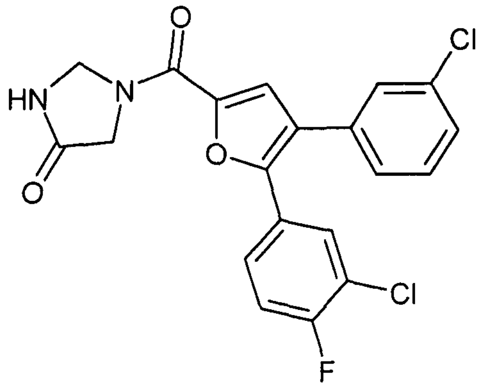

50.0 mg (0.14 mmol) der Verbindung aus Beispiel 30A und 26.0 mg (0.15 mmol) 3-Chlor-4-fluorphenylboronsäure werden unter Argon in 2 ml 1,2-Dimethoxyethan vorgelegt und mit 0.7 ml (0.68 mmol) 10%iger wässriger Natriumcarbonat-Lösung und 4.7 mg (0.004 mmol) Tetrakis(triphenylphosphin)palladium(0) versetzt. Man rührt über Nacht bei 80°C, überführt anschließend die Reaktionsmischung in ein Mikrowellengefäß, versetzt erneut mit der gleichen Menge Katalysator und Boronsäure und erhitzt 30 Minuten bei 120°C im geschlossenen Glasgefäß unter Mikrowellenbestrahlung. Anschließend reinigt man die Reaktionsmischung über präparative HPLC (RP18-Säule; Eluent: Acetonitril/Wasser-Gradient) und erhält 12.0 mg (21% d. Th.) der Titelverbindung.

$^1$H-NMR (400 MHz, DMSO-$d_6$): $\delta$ = 8.76 (s, 1H), 7.71 (dd, 1H), 7.59-7.54 (m, 2H), 7.53-7.49 (m, 2H), 7.48-7.45 (m, 2H), 7.42-7.36 (m, 1H), 5.35 (s, 0.5H), 4.88 (s, 1.5H), 4.46 (s, 1.5H), 3.97 (s, 0.5H).

LC-MS (Methode 7): $R_t$ = 2.04 min; MS (ESIpos): m/z = 419 $[M+H]^+$.

**Beispiel 26**

5-{3-(3-Chlorphenyl)-5-[(4-oxoimidazolidin-1-yl)carbonyl]furan-2-yl}-2-fluorbenzolcarbonitril

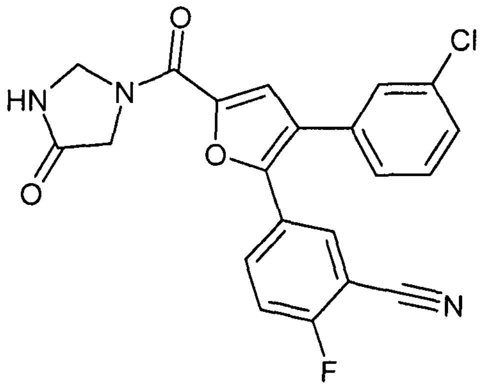

Die Herstellung der Titelverbindung erfolgt ausgehend von der Verbindung aus Beispiel 30A analog zur Synthese der Verbindung aus Beispiel 25. Man erhält 11.0 mg (20% d. Th.) der Titelverbindung.

$^1$H-NMR (400 MHz, DMSO-$d_6$): $\delta$ = 8.76 (s, 1H), 8.12 (dd, 1H), 7.87-7.78 (m, 1H), 7.66-7.53 (m, 3H), 7.50-7.42 (m, 2H), 7.39-7.35 (m, 1H), 5.37 (s, 0.5H), 4.89 (s, 1.5H), 4.48 (s, 1.5H), 3.97 (s, 0.5H).

LC-MS (Methode 7): $R_t$ = 1.82 min; MS (ESIpos): m/z = 410 $[M+H]^+$.

**Beispiel 27**

1-{[4-(3-Chlorphenyl)-5-(3-methoxyphenyl)furan-2-yl]carbonyl}imidazolidin-4-on

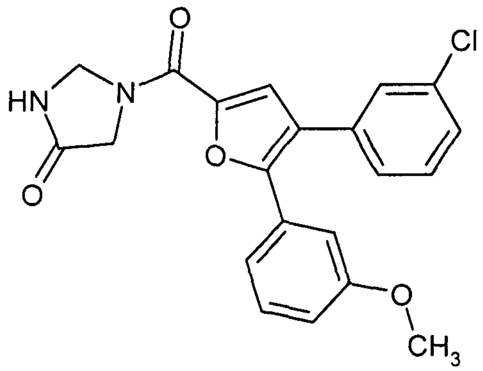

Die Herstellung der Titelverbindung erfolgt ausgehend von der Verbindung aus Beispiel 30A analog zur Synthese der Verbindung aus Beispiel 25. Man erhält 18.0 mg (34% d. Th.) der Titelverbindung.
$^1$H-NMR (400 MHz, DMSO-$d_6$): $\delta$ = 8.75 (s, 1H), 7.58-7.33 (m, 6H), 7.11 (d, 1H), 7.04 (s, 1H), 7.00 (dd, 1H), 5.36 (s, 0.5H), 4.88 (s, 1.5H), 4.45 (s, 1.5H), 3.97 (s, 0.5H), 3.70 (s, 3H).
LC-MS (Methode 7): $R_t$ = 1.87 min; MS (ESIpos): m/z = 397 $[M+H]^+$.

**Beispiel 28**

4-{[5-(3-Chlorphenyl)-4-(3,5-dimethylphenyl)furan-2-yl]carbonyl}piperazin-2-on

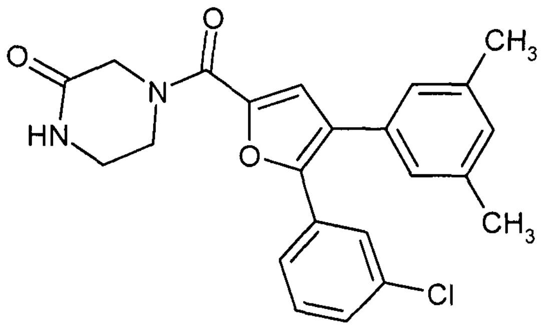

Die Herstellung der Titelverbindung erfolgt ausgehend von der Verbindung aus Beispiel 27A analog zur Synthese der Verbindung aus Beispiel 18. Man erhält 15.0 mg (28% d. Th.) der Titelverbindung.
$^1$H-NMR (400 MHz, DMSO-$d_6$): $\delta$ = 8.19 (s, 1H), 7.52 (s, 1H), 7.46-7.40 (m, 3H), 7.29 (s, 1H), 7.08-7.02 (m, 3H), 4.50-4.05 (m, 2H), 4.00-3.80 (m, 2H), 3.36-3.27 (m, 2H), 2.27 (s, 6H).
LC-MS (Methode 7): $R_t$ = 2.10 min; MS (ESIpos): m/z = 409 $[M+H]^+$.

**Beispiel 29**

4-{[5-(3-Chlorphenyl)-4-(3-methoxyphenyl)furan-2-yl]carbonyl}piperazin-2-on

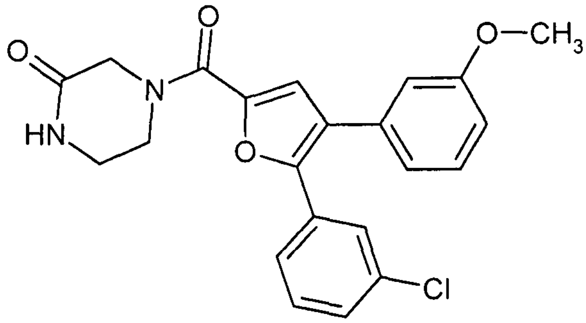

50.0 mg (0.13 mmol) der Verbindung aus Beispiel 27A werden in 5 ml 1,2-Dimethoxyethan/Wasser (4:1) vorgelegt, mit 19.8 mg (0.13 mmol) 3-Methoxyphenylboronsäure, 4.4 mg (0.009 mmol) Dicyclohexyl[2',4',6'-tri(propan-2-yl)biphenyl-2-yl]phosphan, 0.9 mg (0.004 mmol) Palladium(II)acetat und 127 mg (0.39 mmol) Cäsiumcarbonat versetzt und eine Stunde bei 120°C unter Mikrowellenbestrahlung in einem geschlossenen Glasgefäß erhitzt. Anschließend wird das Reaktionsgemisch über präparative HPLC (RP18-Säule; Eluent: Acetonitril/Wasser-Gradient) gereinigt. Man erhält 30.0 mg (56% d. Th.) der Titelverbindung.
$^1$H-NMR (400 MHz, DMSO-$d_6$): $\delta$ = 8.18 (s, 1H), 7.52 (s, 1H), 7.48-7.42 (m, 3H), 7.38-7.33 (m, 2H), 7.03-6.94 (m, 3H), 4.55-3.80 (m, 4H), 3.75 (s, 3H), 3.37-3.27 (m, 2H).
LC-MS (Methode 7): $R_t$ = 1.84 min; MS (ESIpos): m/z = 411 $[M+H]^+$.

**Beispiel 30**

4-{[4,5-Bis(3-chlorphenyl)furan-2-yl]carbonyl}piperazin-2-on

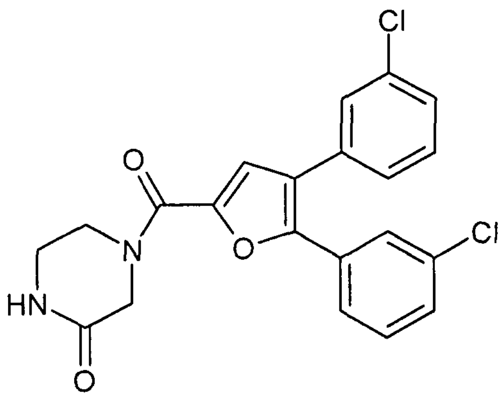

600 mg (1.07 mmol) der Verbindung aus Beispiel 25A werden in 14 ml trockenem Toluol vorgelegt, mit 0.24 ml (3.22 mmol) Thionylchlorid versetzt und 2.5 Stunden unter Rückfluss erhitzt. Das Reaktionsgemisch wird eingeengt, der Rückstand in 10 ml Dichlormethan aufgenommen und auf 0°C gekühlt. Dann werden 0.24 ml (1.72 mmol) Triethylamin und danach 172 mg (1.72 mmol) Piperazin-2-on zugegeben und die Reaktionsmischung wird über Nacht bei RT gerührt. Anschließend wird eingeengt und der Rückstand über präparative HPLC (RP18-Säule; Eluent: Acetonitril/Wasser-Gradient) gereinigt. Man erhält 302 mg (68% d. Th.) der Titelverbindung.
$^1$H-NMR (400 MHz, DMSO-$d_6$): $\delta$ = 8.19 (s, 1H), 7.57-7.54 (m, 1H), 7.52-7.49 (m, 1H), 7.49-7.37 (m, 7H), 4.52-3.75 (m, 4H), 3.37-3.27 (m, 2H).
LC-MS (Methode 7): $R_t$ = 1.99 min; MS (ESIpos): m/z = 415 $[M+H]^+$.

**Beispiel 31**

3-{2-(3-Chlor-4-fluorphenyl)-5-[(4-oxoimidazolidin-1-yl)carbonyl]furan-3-yl}-5-fluorbenzolcarbonitril

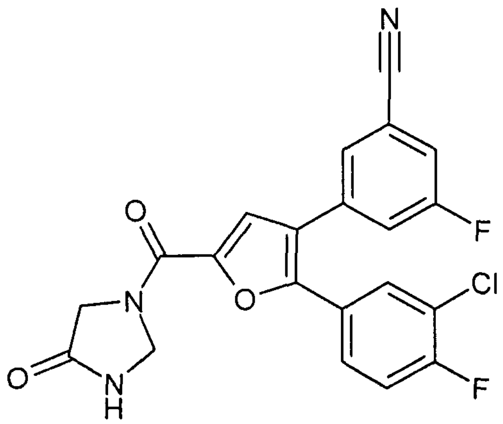

34.0 mg (0.09 mmol) der Verbindung aus Beispiel 15A werden in 1.5 ml Tetrahydrofuran vorgelegt und bei Raumtemperatur mit 21.0 mg (0.88 mmol) Lithiumhydroxid und 0.5 ml Wasser versetzt. Man rührt bei Raumtemperatur über Nacht, gibt anschließend 1N wässrige HCl-Lösung bis zu einem sauren pH-Wert hinzu, extrahiert mit Essigsäureethylester, wäscht die organische Phase mit Wasser, trocknet über Natriumsulfat, filtriert und engt ein. Das Rohprodukt wird zusammen mit 30.0 mg (0.15 mmol) der Verbindung aus Beispiel 35A und 106 mg (0.20 mmol) PyBOP in 2 ml Tetrahydrofuran vorgelegt und bei Raumtemperatur mit 0.08 ml (0.44 mmol) N,N-Diisopropylethylamin versetzt. Man rührt bei Raumtemperatur über Nacht und reinigt anschließend die Reaktionsmischung über präparative HPLC (RP18-Säule; Eluent: Acetonitril/Wasser-Gradient). Man erhält 28.0 mg (74% d. Th.) der Titelverbindung.
$^1$H-NMR (400 MHz, DMSO-$d_6$): $\delta$ = 8.79-8.75 (m, 1H), 7.92-7.83 (m, 2H), 7.78 (dd, 1H), 7.71 (d, 1H), 7.67-7.60 (m, 1H), 7.57-7.47 (m, 2H), 5.34 (s, 0.5H), 4.88 (s, 1.5H), 4.45 (s, 1.5H), 3.97 (s, 0.5H).
LC-MS (Methode 1): $R_t$ = 2.26 min; MS (ESIpos): m/z = 428 $[M+H]^+$.

**Beispiel 32**

3-{2-(3-Chlorphenyl)-5-[(4-oxoimidazolidin-1-yl)carbonyl]furan-3-yl}-5-fluorbenzolcarbonitril

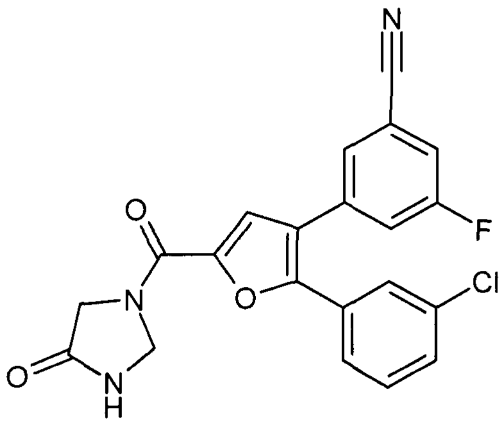

Die Herstellung der Titelverbindung erfolgt ausgehend von der Verbindung aus Beispiel 16A analog zur Synthese der Verbindung aus Beispiel 31. Man erhält 29.9 mg (49% d. Th.) der Titelverbindung.
$^1$H-NMR (400 MHz, DMSO-$d_6$): $\delta$ = 8.80-8.75 (m, 1H), 7.90 (ddd, 1H), 7.87-7.82 (m, 1H), 7.71 (d, 1H), 7.67-7.57 (m, 2H), 7.56-7.42 (m, 3H), 5.34 (s, 0.5H), 4.89 (s, 1.5H), 4.45 (s, 1.5H), 3.97 (s, 0.5H).
LC-MS (Methode 1): $R_t$ = 2.22 min; MS (ESIpos): m/z = 410 $[M+H]^+$.

**Beispiel 33**

3-{2-(3-Chlorphenyl)-5-[(3-oxopiperazin-1-yl)carbonyl]furan-3-yl}-5-fluorbenzolcarbonitril

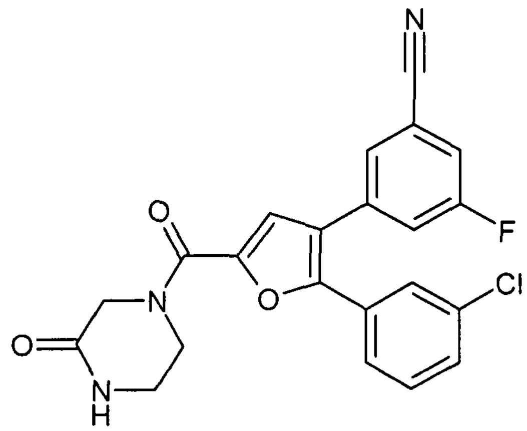

Die Herstellung der Titelverbindung erfolgt ausgehend von der Verbindung aus Beispiel 16A analog zur Synthese der Verbindung aus Beispiel 32. Man erhält 39.5 mg (62% d. Th.) der Titelverbindung.
$^1$H-NMR (400 MHz, DMSO-$d_6$): $\delta$ = 8.20 (s, 1H), 7.90 (ddd, 1H), 7.84 (t, 1H), 7.71 (ddd, 1H), 7.56-7.45 (m, 4H), 7.42 (dt, 1H), 4.55-3.80 (m, 4H), 3.37-3.27 (m, 2H).
LC-MS (Methode 1): $R_t$ = 2.22 min; MS (ESIpos): m/z = 424 $[M+H]^+$.

**Beispiel 34**

3-[2-(3-Cyanphenyl)-5-(morpholin-4-ylcarbonyl)furan-3-yl]-5-fluorbenzolcarbonitril

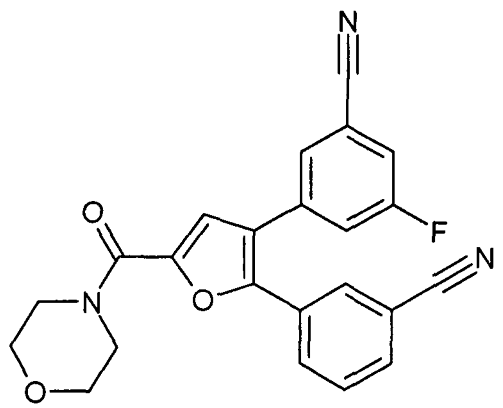

Die Herstellung der Titelverbindung erfolgt ausgehend von der Verbindung aus Beispiel 17A analog zur Synthese der Verbindung aus Beispiel 31. Man erhält 32.1 mg (58% d. Th.) der Titelverbindung.
$^1$H-NMR (400 MHz, DMSO-$d_6$): $\delta$ = 7.96 (t, 1H), 7.93-7.89 (m, 2H), 7.83 (t, 1H), 7.77 (ddd, 1H), 7.69 (ddd, 1H), 7.65 (t, 1H), 7.45 (s, 1H), 3.88-3.62 (m, 8H).
LC-MS (Methode 1): $R_t$ = 2.28 min; MS (ESIpos): m/z = 402 $[M+H]^+$.

B) **Bewertung der physiologischen Wirksamkeit**

**Abkürzungen:**

| | |
|---|---|
| DMSO | Dimethylsulfoxid |
| FKS | Fötales Kälber Serum (Biochrom AG, Berlin, Deutschland) |
| PBS | Phosphate buffered saline |
| MTP | Mikrotiterplatte |
| ELISA | Enzyme-linked immunosorbent assay |

Die Eignung der erfindungsgemäßen Verbindungen zur Behandlung von durch Retroviren hervorgerufenen Erkrankungen kann in folgenden Assay-Systemen gezeigt werden:

**In vitro Assays**

**Biochemischer Reverse Transkriptase Assay**

Es wird der "Reverse Transcriptase Assay, colorimetric" (Roche Diagnostics GmbH, Mannheim, Deutschland) entsprechend den Herstellerangaben verwendet. Die Prüfsubstanzen werden in DMSO gelöst und in 5er Schritten verdünnt in dem Test eingesetzt (DMSO Endkonzentration 1%). Die resultierenden Werte der photometrischen Auswertung (405/492 nm) sind bei der Negativkontrolle (Ansatz ohne Reverse Transkriptase) kleiner 0,1 und liegen bei der Positivkontrolle (Ansatz ohne Prüfsubstanz) im Bereich von 1,5. Die $IC_{50}$-Werte der Prüfsubstanzen werden als die Konzentration der Prüfsubstanzverdünnung ermittelt, bei der die gemessene optische Dichte 50% der Positivkontrolle beträgt.

Es wird gefunden, dass die erfindungsgemäßen Verbindungen die Reverse Transkriptase Aktivität hemmen. Experimentelle Daten sind in Tabelle A zusammengefasst.

**Light Assay mit Wildtyp und Inhibitor-resistenten HI-Reporterviren**

Für diesen Assay werden HIV-$1_{NL4-3}$ Reporterviren verwendet, die anstelle des *nef* Gens das *lu164* Gen (Luziferase 164) tragen. Die Viren werden durch Transfektion von 293T-Zellen mit dem entsprechenden proviralen pNL4-3 Plasmid generiert (Lipofectamine Reagent, Invitrogen, Karlsruhe, Deutschland). Ausgehend von der proviralen Plasmid-DNA werden mit dem "QuikChange II XL Site-Directed Mutagenesis Kit" (Stratagene, Cedar Creek, Texas, USA) Viren mit definierten Resistenzmutationen im Reverse Transkriptase Gen hergestellt. Es werden u.a. folgende Mutationen generiert: A98G, A98G-K103N-V108I, A98S, F227C, F227L, G190A, G190S, K101E, K101Q-K103N, K103N, K103N-F227L, K103N-G190A, K103N-G190S, K103N-M230L, K103N-N348I, K103N-P225H, K103N-V108I, K103N-V108I-P225H, K103N-V179F-Y181C, K103N-Y181C, K103N-Y181C-G190A, L100I, L100I-K103N, L100I-K103N-V179I-Y181C, L100I-K103N-Y181C, L234I, N348I, P225H, P236L, V106A, V106A-E138K, V106A-F227C, V106A-F227L, V106I, V106I-Y188L, V106M, V108I, V179F-Y181C, V179I, V179I-Y181C, Y181C, Y181C-G190A, Y181C-M230L, Y181I, Y188L. Mit diesen Reporterviren infizierte MT4 7F2 Zellen sekretieren Luziferase ins Medium, was die luminometrische Quantifizierung der Virusreplikation ermöglicht.

Für den Ansatz einer 96-well MTP werden 3 Millionen MT4 7F2 Zellen pelletiert, in 1 ml RPMI 1640 Medium ohne Phenolrot (Invitrogen, Karlsruhe, Deutschland)/10% FKS/10% AIM-V (Invitrogen, Karlsruhe, Deutschland) suspendiert und zusammen mit einer geeigneten Menge des entsprechenden HIV-$1_{NL4-3}$ Reportervirus für 2 Stunden bei 37°C inkubiert (Pelletinfektion). Nicht adsorbierte Viren werden anschließend mit PBS ausgewaschen, die infizierten Zellen wieder pelletiert und in 8 ml RPMI 1640 Medium ohne Phenolrot/2% oder 10% FKS/10% AIM-V suspendiert. Davon werden 80 $\mu$l pro Well in eine weiße 96-well MTP zu 20 $\mu$l Prüfsubstanz in geeigneter Verdünnung pipettiert. Um Randeffekte zu vermeiden werden die Randwells der MTP nicht für Substanzverdünnungen verwendet. Die zweite vertikale Reihe der MTP enthält nur infizierte Zellen (Viruskontrolle) und die elfte vertikale Reihe nur nicht infizierte Zellen (Zellkontrolle) jeweils in RPMI 1640 Medium ohne Phenolrot/2% oder 10% FKS/10% AIM-V. Die übrigen Wells der MTP enthalten die erfindungsgemäßen Verbindungen in unterschiedlichen Konzentrationen ausgehend von der dritten vertikalen Reihe, von der aus die Prüfsubstanzen in 3er Schritten bis zur zehnten vertikalen Reihe $3^7$-fach verdünnt werden. Die Prüfsubstanzen sind in DMSO gelöst, wobei die DMSO Endkonzentration im Testansatz 1% beträgt. Die Testansätze werden 5 Tage bei 37°C/5% $CO_2$ inkubiert und nach Zugabe von 15 $\mu$l Lu164-Substrat (5 mg/ml Coelenterazin gelöst in 30 $\mu$M Glutathion/DMSO, 100mM NaCl, 1 M MES, 100 mM Glutathion) luminometrisch ausgewertet. Die resultierenden Werte liegen bei der Viruskontrolle im Bereich von 1.000.000 RLUs (relative Lichteinheiten) und bei der Zellkontrolle

bei 300 bis 400 RLUs. Die $EC_{50}$-Werte der Prüfsubstanzen werden als die Konzentration ermittelt, bei der die in RLUs gemessene Virusreplikation 50% der unbehandelten infizierten Zellen beträgt.

Es wird gefunden, dass die erfindungsgemäßen Verbindungen die HIV-Replikation hemmen. Experimentelle Daten sind in Tabelle A zusammengefasst.

## PBL- und H9-Assay mit Wildtyp HIV-1

Primäre menschliche Blutlymphozyten (PBLs) werden über Ficoll-Paque Leucosep Röhrchen (Greiner Bio-One, Frickenhausen, Deutschland) aus Blut isoliert und in RPMI 1640 Medium (Invitrogen, Karlsruhe, Deutschland)/10% FKS mit Phytohaemagglutinin (90 μg/ml) und Interleukin-2 (40 U/ml) 3 Tage stimuliert.

Für den Ansatz einer 96-well MTP werden 3 Millionen PBLs pelletiert, in 1 ml RPMI 1640 Medium/10% FKS suspendiert und zusammen mit einer geeigneten Menge HIV-$1_{LAI}$ (NIH AIDS Research & Reference Reagent Program, Germantown, USA) für 2 Stunden bei 37°C inkubiert (Pelletinfektion). Nicht adsorbierte Viren werden anschließend mit PBS ausgewaschen, die infizierten Zellen wieder pelletiert und in 18 ml RPMI 1640 Medium/10% FKS/Interleukin-2 (40 U/ml) suspendiert. Davon werden 180 μl pro well in eine weiße 96-well MTP zu 20 μl Prüfsubstanz in geeigneter Verdünnung pipettiert. Alternativ wird das HIV nach Zubereitung der Substanzverdünnungen in der MTP zusammen mit den Zellen zupipettiert und wird nicht mehr ausgewaschen (Überstandsinfektion). Um Randeffekte zu vermeiden werden die Randwells der MTP nicht für Substanzverdünnungen verwendet. Die zweite vertikale Reihe der MTP enthält nur infizierte Zellen (Viruskontrolle) und die elfte vertikale Reihe nur nicht infizierte Zellen (Zellkontrolle) jeweils in RPMI 1640 Medium/10% FKS/Interleukin-2 (40 U/ml). Die übrigen Wells der MTP enthalten die erfindungsgemäßen Verbindungen in unterschiedlichen Konzentrationen ausgehend von der dritten vertikalen Reihe, von der aus die Prüfsubstanzen in 3er Schritten bis zur zehnten vertikalen Reihe $3^7$-fach verdünnt werden. Die Prüfsubstanzen sind in DMSO gelöst, wobei die DMSO Endkonzentration im Testansatz 1% beträgt. Die Testansätze werden bei 37°C/5% $CO_2$ inkubiert. Nach 5 und 7 Tagen erfolgt die Abnahme von jeweils 50 μl zellfreiem Überstand aus jedem Well zur Bestimmung der enthaltenen p24 Menge mittels p24 ELISA (HIV-1 $p24^{CA}$ Antigen Capture Assay Kit, NCI-Frederick Cancer Research and Development Center, Frederick, USA). Aus den resultierenden Werten der photometrischen Auswertung (450/620 nm) werden die $EC_{50}$-Werte der Prüfsubstanzen als die Konzentration ermittelt, bei der die p24 Menge 50% der unbehandelten infizierten Zellen beträgt.

Alternativ werden H9-Zellen (ATCC, Wesel, Deutschland) anstelle von PBLs zum Test der Prüfsubstanzen eingesetzt. H9-Zellen werden nach oben beschriebenem Muster in RPMI 1640 Medium mit 2% oder 10% FKS als HIV-$1_{LAI}$ Überstandsinfektion 5 Tage bei 37°C/5% $CO_2$ inkubiert (20 μl Substanzverdünnung und 80 μl Zellen/Virus pro Well). Anschließend wird je Well 10μl AlamarBlue (Invitrogen, Karlsruhe, Deutschland) zugegeben und die MTPs für 3 Stunden bei 37°C inkubiert, bevor die fluorimetrische Auswertung erfolgt (544/590 nm). Die resultierenden Werte liegen bei den unbehandelten nicht infizierten Zellen bei etwa 40.000 und bei den unbehandelten infizierten Zellen bei etwa 7.000. Im niedrigen Konzentrationsbereich werden die $EC_{50}$-Werte der Prüfsubstanzen als die Konzentration ermittelt, bei der die Fluoreszenz 50% der unbehandelten nicht infizierten Zellen beträgt (jeweils abzüglich der Werte der unbehandelten infizierten Zellen). Außerdem werden im hohen Konzentrationsbereich die $CC_{50}$-Werte der Prüfsubstanzen als die Konzentration ermittelt, bei der die Fluoreszenz 50% der unbehandelten nicht infizierten Zellen beträgt (jeweils abzüglich der Werte der unbehandelten infizierten Zellen).

Es wird gefunden, dass die erfindungsgemäßen Verbindungen die HIV-Replikation hemmen. Experimentelle Daten sind in Tabelle A zusammengefasst.

## Assay zur Bestimmung der zytotoxischen Wirkung der Prüfsubstanzen

Zur Bestimmung der zytotoxischen Wirkung der Prüfsubstanzen in nicht infizierten Zellen werden die Substanzen in entsprechenden Konzentrationen auf durchsichtige 96-well MTPs pipettiert und mit nicht infizierten Zellen (z.B. H9, PBLs, THP-1, MT4 7F2, CEM, Jurkat) inkubiert (analog zu den oben beschriebenen Assays). Nach 5 Tagen wird zu den Testansätzen je Well 1/10 Volumen AlamarBlue zugegeben und die MTPs werden für 3 Stunden bei 37°C inkubiert. Anschließend erfolgt die fluorimetrische Auswertung (544/590 nm). Die resultierenden Werte liegen bei nicht behandelten Zellen je nach Zellart zwischen 20.000 und 40.000. Die $CC_{50}$-Werte der Prüfsubstanzen werden als die Konzentration ermittelt, bei der die Fluoreszenz 50% der unbehandelten Zellen beträgt. Prüfsubstanzen, die im Konzentrationsbereich der Wirkung zytotoxische Befunde zeigen, werden nicht in ihrer antiviralen Wirksamkeit bewertet.

**Tabelle A:**

| Beispiel-Nr. | $IC_{50}$ (nM) RT-Assay | $EC_{50}$ (nM) H9 Zellen HIV-$1_{LAI}$ 10% FKS | $EC_{50}$ (nM) MT4 7F2 Zellen HIV-$1_{NL4-3}$ wt 2% FKS | $EC_{50}$ (nM) MT4 7F2 Zellen HIV-$1_{NL4-3}$ K103N-Y181C 2% FKS |
|---|---|---|---|---|
| Beispiel 1 | | 65 | 4.5 | 550 |
| Beispiel 2 | | 135 | 40 | 1625 |
| Beispiel 3 | | 4.5 | <1.5 | 160 |
| Beispiel 4 | | 12 | <1.5 | 261 |
| Beispiel 5 | | 17.5 | <1.5 | 305 |
| Beispiel 6 | | 400 | 48 | 283 |
| Beispiel 7 | | 46 | 5 | 186 |
| Beispiel 8 | | 7 | 1 | 160 |
| Beispiel 9 | | 100 | 19 | 500 |
| Beispiel 10 | | 14 | 1 | 183 |
| Beispiel 11 | 370 | 77 | 20 | 255 |
| Beispiel 12 | | 30 | 2 | 163 |
| Beispiel 13 | | 160 | 15 | 325 |
| Beispiel 14 | | 52 | 1 | 173 |
| Beispiel 15 | | 110 | 9 | 350 |
| Beispiel 16 | | 75 | 2 | 200 |
| Beispiel 17 | | 135 | 6 | 350 |
| Beispiel 18 | | 200 | 45 | 300 |
| Beispiel 19 | | 250 | 43 | 450 |
| Beispiel 20 | | 50 | 2 | 137 |
| Beispiel 21 | | 200 | 15 | 120 |
| Beispiel 22 | | 113 | 7 | 185 |
| Beispiel 23 | | 18 | 2 | 100 |
| Beispiel 24 | | 47 | 4 | 320 |
| Beispiel 25 | | 225 | 18 | 250 |
| Beispiel 26 | | 150 | 20 | 250 |
| Beispiel 27 | | 200 | 14 | 700 |

| Beispiel-Nr. | $IC_{50}$ (nM) RT-Assay | $EC_{50}$ (nM) H9 Zellen HIV-$1_{LAI}$ 10% FKS | ECso (nM) MT4 7F2 Zellen HIV-$Z_{NL4-3}$ wt 2% FKS | $EC_{50}$ (nM) MT4 7F2 Zellen HIV-$1_{NL4-3}$ K103N-Y181C 2% FKS |
|---|---|---|---|---|
| Beispiel 28 | | 200 | 30 | 400 |
| Beispiel 29 | | 450 | 53 | 400 |
| Beispiel 30 | 750 | 200 | 25 | 650 |
| Beispiel 31 | | 80 | 8 | 80 |

(fortgesetzt)

| Beispiel-Nr. | $IC_{50}$ (nM) RT-Assay | $EC_{50}$ (nM) H9 Zellen HIV-$1_{LAI}$ 10% FKS | ECso (nM) MT4 7F2 Zellen HIV-$Z_{NL4-3}$ wt 2% FKS | $EC_{50}$ (nM) MT4 7F2 Zellen HIV-$1_{NL4-3}$ K103N-Y181C 2% FKS |
|---|---|---|---|---|
| Beispiel 32 | | 70 | 2 | 150 |
| Beispiel 33 | | 25 | <1.5 | 200 |
| Beispiel 34 | | 30 | <1.5 | 300 |

**In vivo Assay**

**Tiermodell:**

NOD Scid Mäuse, in der Regel 5 - 6 Wochen alt, werden von kommerziellen Züchtern (z. B. Taconic oder Jackson Laboratory) bezogen. Die Tiere werden unter sterilen Bedingungen (einschließlich Streu und Futter) in Isolatoren gehalten.

Eine definierte Anzahl von Zellen (z. B. 5 x $10^6$ T-Zellen (z. B. C8166)) wird mit einer geeigneten m.o.i. (z.B. 0.01 $TCID^{50}$) mit HIV infiziert. Die infizierten Zellen werden in Kollagenschwämme eingebracht. Die so vorbehandelten Schwämme werden den Mäusen unter die Rückenhaut implantiert. Die Mäuse werden einmal oder mehrfach täglich peroral, intraperitoneal, subcutan oder intravenös behandelt, wobei die erste Behandlung vor der Implantation liegen kann. Die Behandlungsgruppen umfassen in der Regel 10 Mäuse. Mindestens eine Gruppe wird mit Placebo behandelt, mindestens eine Gruppe mit einer bekanntermaßen wirksamen Substanz (= Positivkontrolle) und in der Regel mehrere Gruppen mit der erfindungsgemäßen Substanz. Die Tagesdosis der erfindungsgemäßen Substanz liegt zwischen 0.01 mg und 100 mg pro kg Körpergewicht. Die Formulierung der Substanzen erfolgt in 2% DMSO/0.5% Methylcellulose in PBS oder einer anderen geeigneten Mischung, die die Löslichkeit der Substanzen unterstützt. Die Behandlungsdauer beträgt in der Regel viereinhalb Tage. Nach der letzten Substanzapplikation werden die Tiere getötet und die Schwämme entnommen. Die virusinfizierten Zellen werden durch Kollagenaseverdau aus dem Schwamm gewonnen.

Aus den Zellen wird Gesamt-RNA gewonnen, die in der quantitativen PCR auf den Gehalt an Virus-RNA überprüft wird. Die Menge an Virus-RNA wird anhand der Menge eines house-keeping Gens (z. B. GAPDH) normalisiert. Ermittelt wird die Menge an HIV-RNA nach Substanzbehandlung im Vergleich zur placebobehandelten Kontrollgruppe. Wurde ein HIV verwendet, das eine Luziferase trägt, kann zusätzlich oder ersatzweise eine Luziferase-Messung durchgeführt werden. In diesem Fall wird die HIV-Menge über die Höhe des Luziferase-Signals bestimmt, da es in diesem Fall als Maß für die Virusreplikation dient. Die statistische Auswertung erfolgt mittels geeigneter Computerprogramme, z. B. Graph Pad Prism.

B) **Bewertung der pharmakokinetischen Eigenschaften**

***In vivo* Studien**

Zur Bestimmung der *in vivo* Pharmakokinetik werden die Testsubstanzen Mäusen, Ratten und Hunden intravenös und oral appliziert. Bei intravenösen Studien wird zur Bestimmung der pharmakokinetischen Eigenschaften der Testsubstanzen eine Dosis von 0.5 mg/kg in allen Spezies gewählt. Bei oraler Gabe wird den Nagern 3 mg/kg appliziert, Hunden 1 mg/kg. Die Testsubstanzen werden zur intravenösen Gabe für Nager in 99% Plasma, 1% DMSO formuliert, bei oraler Gabe in PEG 400, Ethanol und Wasser in variierenden Anteilen. Letzteres Vehikel wird bei Hunden für beide Applikationsrouten verwendet.

Männliche Wistar Ratten werden vor Applikation der Testsubstanzen katheterisiert, so dass die Blutproben mit Hilfe des gelegten Katheters oder durch Punktion der Vena cava zu verschiedenen Zeitenpunkten über ein Intervall von 2 min bis zu 26 h entnommen werden können.

Weiblichen BalbC Mäusen werden die Testsubstanzen als bolus Injektion intravenös appliziert, die Probengewinnung erfolgt in diesem Falle ausschließlich durch Punktion der Vena cava über ein Intervall von 2 min bis zu 26 h. Bei weiblichen Beagle Hunden erfolgt die Applikation ausschließlich durch eine 15 minütige intravenöse Infusion. Die Proben werden durch Punktion der Vena brachialis oder der Vena jugularis über ein Intervall von 10 min bis zu 26 h gewonnen.

Die quantitative Bestimmung der Substanzen erfolgt aus dem gewonnenen Tierplasma und Eichproben, die in Plasma eingestellt werden. Die Plasmaproteine werden durch Fällung mit Acetonitril (ACN) entfernt. Anschließend

werden die Proben mittels HPLC auf einer Agilent 1100 LC Anlage (Agilent, Santa Clara, Californien, USA) unter Verwendung unterschiedlicher Säulen, z.B. Luna C8, LichroCart Purospher Star RP18e aufgetrennt. Das HPLC System ist über ein Turbo Ion Spray Interface an ein Triple Quadropole Massenspektrometer API 3000 (Applied Biosystems, Darmstadt, Deutschland) gekoppelt. Die Auswertung des Plasmakonzentrations-Zeitverlaufs erfolgt unter Einsatz eines internen Standards und Verwendung eines validierten Kinetikauswerteprogramms.

Neben Studien zur Bestimmung der pharmakokinetischen Parameter der Testsubstanzen *in vivo* werden Bestimmungen der relativen Bioverfügbarkeit aus Suspension (Formulierung: Tylose Suspension) versus Lösung in der Ratte sowie Hochdosisstudien im Vorfeld von Wirkversuchen und toxikologischen Studien in Mäusen, Ratten und Hunden durchgeführt.

**Plasmastabilität**

Das verwendete Plasma der unterschiedlichen Spezies (BalbC Maus, Wistar Ratte, Beagle Hund und Mensch) wird durch Blutabnahme, in mit Li-Heparin beschichtete Monovetten und anschließender Zentrifugation, frisch gewonnen. Zur Bestimmung der Plasmastabilität der Testsubstanzen wird je 500 ng/ml bei 37°C, zu je 2 ml in Plasma inkubiert. Zu verschiedenen Zeitpunkten, über ein Intervall bis zu 3 h, werden Proben dem Inkubationsgefäß entnommen. Die gewonnenen Proben werden mit ACN gefällt, um die Umsetzung zu stoppen und die Plasmaproteine abzutrennen. Die Proben werden äquivalent zu den *in vivo* Studien analysiert.

**Mikrosomale und Hepatozyten Inkubationen**

Inkubationen mit Lebermikrosomen verschiedener Spezies (BalbC Maus, Wistar Ratte, Beagle Hund, Mensch) werden in einem Gesamtvolumen von 1.5 ml bei 37°C auf einem modifizierten Multiprobe II® Robotersytem (Canberra Packard) bzw. Janus® Robotersystem (Perkin Elmer) durchgeführt.

Die Inkubationsmischungen enthalten jeweils 0.5 μg/ml Testsubstanz sowie 0.2 - 0.5 mg/ml mikrosomales Protein. Zusätzlich wird 0.05 M Phosphatpuffer (pH = 7.4), 1 mM EDTA, 5 mM Glucose-6-phosphat und 1.5 U/ml Glucose-6-phosphat Dehydroxygenase aus *Leuconostoc Mesenteroides* zugesetzt. Die mikrosomale Inkubationen wird durch Zugabe von $NADP^+$ (Endkonzentration: 1 mM) gestartet.

Zur Bestimmung der metabolischen Stabilität der Testsubstanzen in frisch isolierten und kultivierten Ratten-Hunde-, und Humanhepatozyten werden jeweils 1 Millionen Zellen/ml verwendet. Äquivalent dem mikrosomalen Assay werden den Hepatozyten jeweils 0.5 μg/ml Testsubstanz zugesetzt.

Nach 2, 5, 10, 20, 30, 45 und 60 min, oder nach 2, 10, 20, 30, 50, 70 und 90 min bei stabileren Verbindungen, werden 125 μl aus dem jeweiligen Inkubationsansatz entnommen und mit ACN versetzt, um die enzymatischen Reaktionen zu stoppen. Nach der Zentrifugation werden die Proben mittels LC-MS/MS (API 2000 oder 3000, Applied Biosystems) analysiert. "$CL_{blood}$ well-stirred"- und "$F_{max}$ well-stirred"-Werte werden aus den jeweiligen Halbwertszeiten der Verbindungen in den mikrosomalen Inkubationen errechnet. Der Substratabbau kann mit folgenden Formeln beschrieben werden (Houston JB, Utility of in-vitro drug-metabolism data in predicting in-vivo metabolic-clearance, Bioch. Pharm. 47 (9) 1469- 1479 (1994); Obach RS; Baxter JG; Liston TE; Silber BM; Jones BC; MacIntyre F; Rance DJ; Wastall P, The prediction of human pharmacokinetic parameters from preclinical and in vitro metabolism data, J. Pharmacol. Exp. Ther. 283 (1) 46- 58 (1997)):

$$CL'_{intrinsic}\ [ml/(min \cdot kg)] = (0.693\ /\ in\ vitro\ t_{1/2}\ [min]) \cdot (Lebergewicht\ [g\ Leber\ /kg\ Körpergewicht]) \cdot (mikrosomales\ Protein\ [mg]\ /\ Lebergewicht\ [g])\ /\ (mikrosomales\ Protein\ [mg]\ /\ Inkubationsvolumen\ [ml])$$

Die Blut-Clearance "$CL_{blood}$" wird ohne die Berücksichtigung von Proteinbindungen durch das "well-stirred" Modell beschrieben (Pang KS; Rowland M, Hepatic clearance of drugs. I. Theoretical considerations of a "well-stirred" model and a "parallel tube" model. Influence of hepatic blood flow, plasma and blood cell binding, and the hepatocellular enzymatic activity on hepatic drug clearance, J Pharmacokinet Biopharm 5 (6): 625-53 (1977)):

$$CL_{blood}\ well\text{-}stirred\ [l/(h \cdot kg)] = (Q_H\ [l/(h \cdot kg)] \cdot CL'_{intrinsic}\ [l/(h \cdot kg)]\ )\ /\ (Q_H\ [l/(h \cdot kg)] + CL'_{intrinsic}\ [l/(h \cdot kg)])$$

Für die Ratten beträgt das spezifische Lebergewicht 32 g/kg Körpergewicht und der hepatische Blutfluss 4.2

1/(h·kg). Der spezifische mikrosmale Proteingehalt der Rattenleber wurde mit 40 mg/g Leber veranschlagt. Die spezifischen Hochrechnungsfaktoren weiterer Spezies sind in folgender Tabelle wiedergegeben und beruhen zum Teil auf Literaturdaten, zum Teil auf eigenen Bestimmungen. Für Hepatozyten wird eine Zellzahl von 110 Mio/g Leber bei allen Spezies zur Berechnung herangezogen.

| | Maus m | Maus w | Ratte m | Hund m/w | Mensch m/w |
|---|---|---|---|---|---|
| Mikrosomales Protein/g Leber [mg] | 40 | 40 | 40 | 40 | 40 |
| Leber [g]/kg Körpergewicht | 50 | 43 | 32 | 39 | 21 |
| Leberblutfluss [1/(h·kg)] | 5.4 | 5.4 | 4.2 | 2.1 | 1.32 |

**C) <u>Ausführungsbeispiele für pharmazeutische Zusammensetzungen</u>**

Die erfindungsgemäßen Verbindungen können folgendermaßen in pharmazeutische Zubereitungen überführt werden:

Tablette:

Zusammensetzung:

100 mg der Verbindung von Beispiel 1, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

Herstellung:

Die Mischung aus erfindungsgemäßer Verbindung, Lactose und Stärke wird mit einer 5%igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

**Oral applizierbare Lösung:**

Zusammensetzung:

500 mg der Verbindung von Beispiel 1, 2.5 g Polysorbat und 97 g Polyethylenglycol 400. Einer Einzeldosis von 100 mg der erfindungsgemäßen Verbindung entsprechen 20 g orale Lösung.

Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

i.v. Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5%, PEG 400 Lösung 30%) gelöst. Die Lösung wird steril filtriert und in sterile und pyrogenfreie Injektionsbehältnisse abgefüllt.

**Patentansprüche**

**1.** Verbindung der Formel

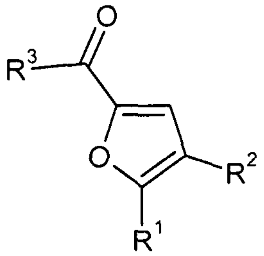

(I),

in welcher

$R^1$ für Phenyl steht,
wobei Phenyl substituiert ist mit 1 bis 3 Substituenten, wobei die Substituenten unabhängig voneinander ausgewählt werden aus der Gruppe bestehend aus Halogen, Hydroxy, Cyano, Nitro, Trifluormethyl, Trifluormethoxy, Trifluormethylthio, $(C_1-C_4)$-Alkyl und $(C_1-C_4)$-Alkoxy,
worin

$(C_1-C_4)$-Alkyl und $(C_1-C_4)$-Alkoxy ihrerseits ein- bis dreifach, gleich oder verschieden, mit Resten substituiert sein können, die ausgewählt werden aus der Reihe Halogen, Cyano, Hydroxy, $(C_1-C_4)$-Alkoxy, Amino, Mono- $(C_1-C_4)$-alkylamino, Di-$(C_1-C_4)$-alkyl-amino, $(C_3-C_7)$-Cycloalkyl und 4- bis 7-gliedriges Heterocyclyl, wobei die letztgenannten Cycloalkyl- und Heterocyclyl-Reste ihrerseits jeweils bis zu dreifach, gleich oder verschieden, mit Halogen, Cyano, $(C_1-C_4)$-Alkyl, Trifluormethyl, Hydroxy, $(C_1-C_4)$-Alkoxy, Trifluormethoxy, Oxo, Amino, Mono-$(C_1-C_4)$-alkyl-amino und Di-$(C_1-C_4)$-alkylamino substituiert sein können,

$R^2$ für Phenyl steht,
wobei Phenyl substituiert ist mit 1 bis 3 Substituenten, wobei die Substituenten unabhängig voneinander ausgewählt werden aus der Gruppe bestehend aus Halogen, Hydroxy, Cyano, Nitro, Trifluormethyl, Trifluormethoxy, Trifluormethylthio, $(C_1-C_4)$-Alkyl und $(C_1-C_4)$-Alkoxy,
worin

$(C_1-C_4)$-Alkyl und $(C_1-C_4)$-Alkoxy ihrerseits ein- bis dreifach, gleich oder verschieden, mit Resten substituiert sein können, die ausgewählt werden aus der Reihe Halogen, Cyano, Hydroxy, $(C_1-C_4)$-Alkoxy, Amino, Mono-$(C_1-C_4)$-alkylamino, Di-$(C_1-C_4)$-alkyl-amino, $(C_3-C_7)$-Cycloalkyl und 4- bis 7-gliedriges Heterocyclyl, wobei die letztgenannten Cycloalkyl- und Heterocyclyl-Reste ihrerseits jeweils bis zu dreifach, gleich oder verschieden, mit Halogen, Cyano, $(C_1-C_4)$-Alkyl, Trifluormethyl, Hydroxy, $(C_1-C_4)$-Alkoxy, Trifluormethoxy, Oxo, Amino, Mono-$(C_1-C_4)$-alkyl-amino und Di-$(C_1-C_4)$-alkylamino substituiert sein können, und

$R^3$ für einen über Stickstoff gebundenen Heterocyclyl-Rest steht,
wobei der Heterocyclyl-Rest substituiert sein kann mit 1 bis 3 Substituenten, wobei die Substituenten unabhängig voneinander ausgewählt werden aus der Gruppe bestehend aus Halogen, Hydroxy, Hydroxymethyl, Formyl, Amino, Oxo, Trifluormethyl, Trifluormethoxy, $(C_1-C_4)$-Alkyl, $(C_1-C_4)$-Alkoxy und $(C_1-C_4)$-Alkoxycarbonyl,

oder eines ihrer Salze, ihrer Solvate oder der Solvate ihrer Salze.

2. Verbindung nach Anspruch 1, **dadurch gekennzeichnet, dass**

$R^1$ für Phenyl steht,
wobei Phenyl substituiert ist mit 1 bis 2 Substituenten, wobei die Substituenten unabhängig voneinander ausgewählt werden aus der Gruppe bestehend aus Halogen, Hydroxy, Cyano, Nitro, Trifluormethyl, Trifluormethoxy, Trifluormethylthio, $(C_1-C_4)$-Alkyl und $(C_1-C_4)$-Alkoxy,
$R^2$ für Phenyl steht,
wobei Phenyl substituiert ist mit 1 bis 2 Substituenten, wobei die Substituenten unabhängig voneinander ausgewählt werden aus der Gruppe bestehend aus Halogen, Hydroxy, Cyano, Nitro, Trifluormethoxy, Trifluormethylthio, $(C_1-C_4)$-Alkyl und $(C_1-C_4)$-Alkoxy,
worin

$(C_1-C_4)$-Alkoxy seinerseits ein- bis dreifach, gleich oder verschieden, mit Resten substituiert sein kann, die ausgewählt werden aus der Reihe Halogen, Cyano, Hydroxy, $(C_1-C_4)$-Alkoxy, Amino, Mono-$(C_1-C_4)$-alkyl-amino, Di-$(C_1-C_4)$-alkylamino, $(C_3-C_7)$-Cycloalkyl und 4- bis 7-gliedriges Heterocyclyl,

wobei die letztgenannten Cycloalkyl- und Heterocyclyl-Reste ihrerseits jeweils bis zu dreifach, gleich oder verschieden, mit Halogen, Cyano, $(C_1-C_4)$-Alkyl, Trifluormethyl, Hydroxy, $(C_1-C_4)$-Alkoxy, Trifluormethoxy, Oxo, Amino, Mono-$(C_1-C_4)$-alkyl-amino und Di-$(C_1-C_4)$-alkylamino substituiert sein können, und

$R^3$ für einen über Stickstoff gebundenen Heterocyclyl-Rest steht,
wobei der Heterocyclyl-Rest substituiert sein kann mit 1 bis 3 Substituenten, wobei die Substituenten unabhängig voneinander ausgewählt werden aus der Gruppe bestehend aus Halogen, Hydroxy, Hydroxymethyl, Formyl, Amino, Oxo, Trifluormethyl, Trifluormethoxy, $(C_1-C_4)$-Alkyl, $(C_1-C_4)$-Alkoxy und $(C_1-C_4)$-Alkoxycarbonyl,

oder eines ihrer Salze, ihrer Solvate oder der Solvate ihrer Salze.

**3.** Verbindung nach Anspruch 1 oder 2, **dadurch gekennzeichnet, dass**

$R^1$ für Phenyl steht,
wobei Phenyl substituiert ist mit 1 bis 2 Substituenten, wobei die Substituenten unabhängig voneinander ausgewählt werden aus der Gruppe bestehend aus Halogen, Cyano, Trifluormethyl, Methyl und Methoxy,
$R^2$ für Phenyl steht,
wobei Phenyl substituiert ist mit 1 bis 2 Substituenten, wobei die Substituenten unabhängig voneinander ausgewählt werden aus der Gruppe bestehend aus Halogen, Cyano, Trifluormethoxy, Methyl und $(C_1-C_3)$-Alkoxy, und
$R^3$ für einen über Stickstoff gebundenen Heterocyclyl-Rest steht,
wobei der Heterocyclyl-Rest substituiert sein kann mit 1 bis 3 Substituenten, wobei die Substituenten unabhängig voneinander ausgewählt werden aus der Gruppe bestehend aus Halogen, Hydroxy, Amino, Oxo, Trifluormethyl, $(C_1-C_4)$-Alkyl und $(C_1-C_4)$-Alkoxy,

oder eines ihrer Salze, ihrer Solvate oder der Solvate ihrer Salze.

**4.** Verbindung nach einem der Ansprüche 1 bis 3, **dadurch gekennzeichnet, dass**

$R^1$ für Phenyl steht,
wobei Phenyl substituiert ist mit 1 bis 2 Substituenten, wobei die Substituenten unabhängig voneinander ausgewählt werden aus der Gruppe bestehend aus Halogen, Cyano und Methyl,
$R^2$ für Phenyl steht,
wobei Phenyl substituiert ist mit 1 bis 2 Substituenten, wobei die Substituenten unabhängig voneinander ausgewählt werden aus der Gruppe bestehend aus Halogen, Cyano, Methyl und Methoxy, und
$R^3$ für einen über Stickstoff gebundenen Heterocyclyl-Rest steht,
wobei der Heterocyclyl-Rest substituiert sein kann mit 1 bis 2 Substituenten aus der Gruppe Hydroxy und Oxo,

oder eines ihrer Salze, ihrer Solvate oder der Solvate ihrer Salze.

**5.** Verbindung nach einem der Ansprüche 1 bis 4, **dadurch gekennzeichnet, dass**

$R^1$ für Phenyl steht,
wobei Phenyl substituiert ist mit 1 bis 2 Substituenten, wobei die Substituenten unabhängig voneinander ausgewählt werden aus der Gruppe bestehend aus Halogen und Cyano,
$R^2$ für Phenyl steht,
wobei Phenyl substituiert ist mit 1 bis 2 Substituenten, wobei die Substituenten unabhängig voneinander ausgewählt werden aus der Gruppe bestehend aus Halogen und Cyano, und
$R^3$ für

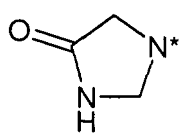, 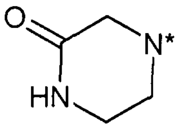, 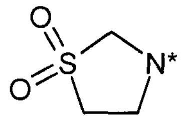,

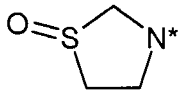, 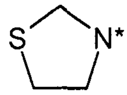 oder 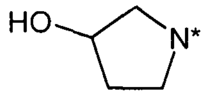

steht, wobei
* die Anknüpfungsstelle an die Carbonylgruppe bedeutet,

oder eines ihrer Salze, ihrer Solvate oder der Solvate ihrer Salze.

**6.** Verbindung nach einem der Ansprüche 1 bis 4, **dadurch gekennzeichnet, dass** sie der Formel

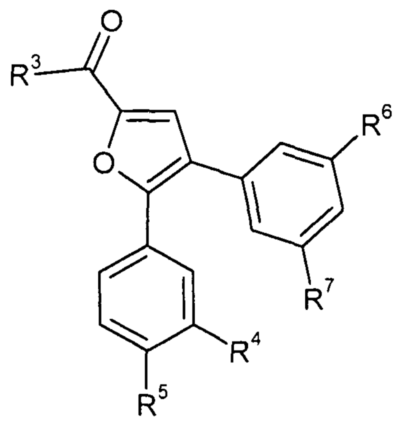

(Ia),

entspricht, in welcher

$R^3$ für einen über Stickstoff gebundenen Heterocyclyl-Rest steht,
wobei der Heterocyclyl-Rest substituiert sein kann mit 1 bis 2 Substituenten aus der Gruppe Hydroxy und Oxo,
$R^4$ für Halogen, Cyano oder Methyl steht,
$R^5$ für Wasserstoff oder Halogen steht,
$R^6$ für Halogen, Cyano, Methyl oder Methoxy steht, und
$R^7$ für Wasserstoff oder Halogen steht,

oder eines ihrer Salze, ihrer Solvate oder der Solvate ihrer Salze.

**7.** Verbindung nach Anspruch 6, **dadurch gekennzeichnet, dass**

$R^3$ für

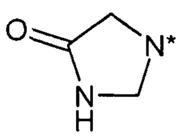, 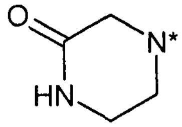, 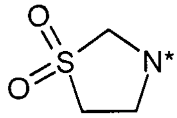,

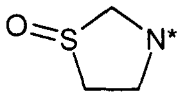, 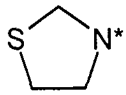 oder 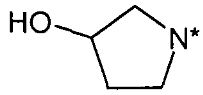

steht, wobei
* die Anknüpfungsstelle an die Carbonylgruppe bedeutet,
$R^4$ für Halogen, Cyano oder Methyl steht,
$R^5$ für Wasserstoff oder Halogen steht,
$R^6$ für Halogen, Cyano, Methyl oder Methoxy steht, und
$R^7$ für Wasserstoff oder Halogen steht,

oder eines ihrer Salze, ihrer Solvate oder der Solvate ihrer Salze.

**8.** Verbindung nach Anspruch 6 oder 7, **dadurch gekennzeichnet, dass**

$R^3$ für

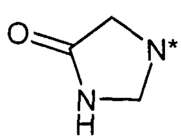, 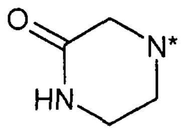, 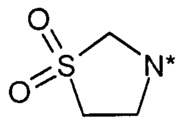,

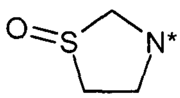, 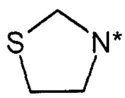 oder 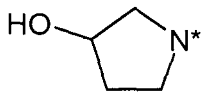

steht, wobei
* die Anknüpfungsstelle an die Carbonylgruppe bedeutet,
$R^4$ für Fluor, Chlor, Cyano oder Methyl steht,
$R^5$ für Wasserstoff oder Fluor steht,
$R^6$ für Fluor, Chlor, Cyano, Methyl oder Methoxy steht, und
$R^7$ für Wasserstoff oder Fluor steht,

oder eines ihrer Salze, ihrer Solvate oder der Solvate ihrer Salze.

**9.** Verbindung nach einem der Ansprüche 6 bis 8, **dadurch gekennzeichnet, dass**

$R^3$ für

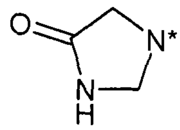

steht, wobei
* die Anknüpfungsstelle an die Carbonylgruppe bedeutet,
$R^4$ für Chlor oder Cyano steht,
$R^5$ für Wasserstoff oder Fluor steht,
$R^6$ für Chlor oder Cyano steht, und
$R^7$ für Wasserstoff oder Fluor steht,

oder eines ihrer Salze, ihrer Solvate oder der Solvate ihrer Salze.

**10.** Verfahren zur Herstellung einer Verbindung der Formel (I) nach Anspruch 1, **dadurch gekennzeichnet, dass** eine Verbindung

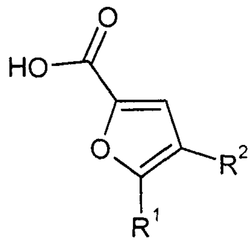

(II),

in welcher
$R^1$ und $R^2$ die oben angegebene Bedeutung aufweisen,
mit einer Verbindung der Formel $R^3$, mit der in Anspruch 1 angegebenen Bedeutung, oder einem Salz einer Verbindung der Formel $R^3$ umgesetzt wird.

**11.** Verfahren zur Herstellung einer Verbindung der Formel (I) nach Anspruch 1, **dadurch gekennzeichnet, dass** eine Verbindung der Formel

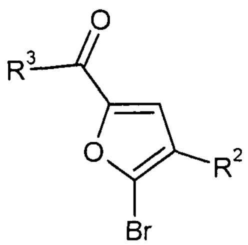

(VIII),

in welcher
$R^2$ und $R^3$ die in Anspruch 1 angegebene Bedeutung aufweisen,
unter Suzuki-Kupplungsbedingungen mit einer Verbindung der Formel

$R^1$-Q (VII),

in welcher

$R^1$ die in Anspruch 1 angegebene Bedeutung aufweist und
Q für $-B(OH)_2$, einen Boronsäure-Ester, bevorzugt Boronsäurepinakolester, oder $-BF_3^-K^+$ steht,

umgesetzt wird.

**12.** Verbindung nach einem der Ansprüche 1 bis 9 zur Behandlung und/oder Prophylaxe von Krankheiten.

**13.** Verwendung einer Verbindung nach einem der Ansprüche 1 bis 9 zur Herstellung eines Arzneimittels zur Behandlung und/oder Prophylaxe von Krankheiten.

**14.** Verwendung einer Verbindung nach einem der Ansprüche 1 bis 9 zur Herstellung eines Arzneimittels zur Behandlung und/oder Prophylaxe von retroviralen Erkrankungen.

**15.** Verwendung nach Anspruch 14, **dadurch gekennzeichnet, dass** die retrovirale Erkrankung eine Infektion mit dem HI-Virus ist.

**16.** Arzneimittel enthaltend mindestens eine Verbindung nach einem der Ansprüche 1 bis 9 in Kombination mit mindestens einem weiteren Wirkstoff.

**17.** Arzneimittel enthaltend mindestens eine Verbindung nach einem der Ansprüche 1 bis 9 in Kombination mit mindestens einem inerten, nicht-toxischen, pharmazeutisch geeigneten Hilfsstoff.

**18.** Arzneimittel nach Anspruch 16 oder 17 zur Verwendung in der Behandlung und/oder Prophylaxe von retroviralen Erkrankungen.

**19.** Arzneimittel zur Verwendung nach Anspruch 18, **dadurch gekennzeichnet, dass** die retrovirale Erkrankung eine Infektion mit dem HI-Virus ist.

**20.** Verbindung nach einem der Ansprüche 1-9 oder ein Arzneimittel nach einem der Ansprüche 16 bis 17 oder Arzneimittel zur Verwendung nach einem der Ansprüche 18 bis 19 zur Verwendung bei der Behandlung von viralen Erkrankungen in Menschen und Tieren durch Verabreichung einer antiviral wirksamen Menge der besagten Verbindung oder des besagten Arzneimittels.

## Claims

**1.** A compound of the formula

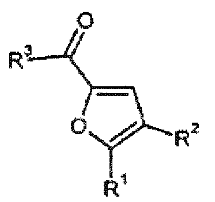

(I),

wherein

$R^1$ stands for phenyl,
where phenyl is substituted with 1 to 3 substituents, where the substituents are selected independently of one another from the group consisting of halogen, hydroxy, cyano, nitro, trifluoromethyl, trifluoromethoxy, trifluoromethylthio, $(C_1-C_4)$-alkyl and $(C_1-C_4)$-alkoxy,
wherein

$(C_1-C_4)$-alkyl and $(C_1-C_4)$-alkoxy in turn may be substituted one to three times, identically or differently, with residues selected from the group consisting of halogen, cyano, hydroxy, $(C_1-C_4)$-alkoxy, amino, mono-$(C_1-C_4)$-alkylamino, di-$(C_1-C_4)$-alkylamino, $(C_3-C_7)$-cycloalkyl and 4- to 7-membered heterocyclyl,

where the last-mentioned cycloalkyl and heterocyclyl residues in turn may each be substituted up to three times, identically or differently, with halogen, cyano, ($C_1$-$C_4$)-alkyl, trifluoromethyl, hydroxy, ($C_1$-$C_4$)-alkoxy, trifluoromethoxy, oxo, amino, mono-($C_1$-$C_4$)-alkylamino and di-($C_1$-$C_4$)-alkylamino,

$R^2$ stands for phenyl,
where phenyl is substituted with 1 to 3 substituents, where the substituents are selected independently of one another from the group consisting of halogen, hydroxy, cyano, nitro, trifluoromethyl, trifluoromethoxy, trifluoromethylthio, ($C_1$-$C_4$)-alkyl and ($C_1$-$C_4$)-alkoxy,
wherein

($C_1$-$C_4$)-alkyl and ($C_1$-$C_4$)-alkoxy in turn may be substituted one to three times, identically or differently, with residues selected from the group consisting of halogen, cyano, hydroxy, ($C_1$-$C_4$)-alkoxy, amino, mono-($C_1$-$C_4$)-alkylamino, di-($C_1$-$C_4$)-alkylamino, ($C_3$-$C_7$)-cycloalkyl and 4- to 7-membered heterocyclyl,
where the last-mentioned cycloalkyl and heterocyclyl residues in turn may each be substituted up to three times, identically or differently, with halogen, cyano, ($C_1$-$C_4$)-alkyl, trifluoromethyl, hydroxy, ($C_1$-$C_4$)-alkoxy, trifluoromethoxy, oxo, amino, mono-($C_1$-$C_4$)-alkylamino and di-($C_1$-$C_4$)-alkylamino, and

$R^3$ stands for a heterocyclyl residue bound with nitrogen,
where the heterocyclyl residue can be substituted with one to three substituents, where the substituents are selected independently of one another from the group consisting of halogen, hydroxy, hydroxymethyl, formyl, amino, oxo, trifluoromethyl, trifluoromethoxy, ($C_1$-$C_4$)-alkyl, ($C_1$-$C_4$)-alkoxy and ($C_1$-$C_4$)-alkoxycarbonyl,

or one of their salts, their solvates or the solvates of their salts.

2. The compound according to Claim 1, **characterized in that**

$R^1$ stands for phenyl,
where phenyl is substituted with 1 to 2 substituents, where the substituents are selected independently of one another from the group consisting of halogen, hydroxy, cyano, nitro, trifluoromethyl, trifluoromethoxy, trifluoromethylthio, ($C_1$-$C_4$)-alkyl and ($C_1$-$C_4$)-alkoxy,
$R^2$ stands for phenyl,
where phenyl is substituted with 1 to 2 substituents, where the substituents are selected independently of one another from the group consisting of halogen, hydroxy, cyano, nitro, trifluoromethoxy, trifluoromethylthio, ($C_1$-$C_4$)-alkyl and ($C_1$-$C_4$)-alkoxy,
wherein
($C_1$-$C_4$)-alkoxy in turn may be substituted one to three times, identically or differently, with residues selected from the group consisting of halogen, cyano, hydroxy, ($C_1$-$C_4$)-alkoxy, amino, mono-($C_1$-$C_4$)-alkylamino, di-($C_1$-$C_4$)-alkylamino, ($C_3$-$C_7$)-cycloalkyl and 4- to 7-membered heterocyclyl,

where the last-mentioned cycloalkyl and heterocyclyl residues in turn may each be substituted up to three times, identically or differently, with halogen, cyano, ($C_1$-$C_4$)-alkyl, trifluoromethyl, hydroxy, ($C_1$-$C_4$)-alkoxy, trifluoromethoxy, oxo, amino, mono-($C_1$-$C_4$)-alkylamino and di-($C_1$-$C_4$)-alkylamino, and

$R^3$ stands for a heterocyclyl residue bound with nitrogen,
where the heterocyclyl residue can be substituted with 1 to 3 substituents, where the substituents are selected independently of one another from the group consisting of halogen, hydroxy, hydroxymethyl, formyl, amino, oxo, trifluoromethyl., trifluoromethoxy, ($C_1$-$C_4$)-alkyl, ($C_1$-$C_4$)-alkoxy and ($C_1$-$C_4$)-alkoxycarbonyl,

or one of their salts, their solvates or the solvates of their salts.

3. The compound according to Claim 1 or 2, **characterized in that**

$R^1$ stands for phenyl,
where phenyl is substituted with 1 to 2 substituents, where the substituents are selected independently of one another from the group consisting of halogen, cyano, trifluoromethyl, methyl and methoxy,
$R^2$ stands for phenyl,
where phenyl is substituted with 1 to 2 substituents, where the substituents are selected independently of one another from the group consisting of halogen, cyano, trifluoromethoxy, methyl and ($C_1$-$C_3$)-alkoxy, and

$R^3$ stands for a heterocyclyl residue bound with nitrogen,
where the heterocyclyl residue can be substituted with 1 to 3 substituents, where the substituents are selected independently of one another from the group consisting of halogen, hydroxy, amino, oxo, trifluoromethyl, $(C_1\text{-}C_4)$-alkyl and $(C_1\text{-}C_4)$-alkoxy,

or one of their salts, their solvates or the solvates of their salts

**4.** The compound according to any of Claims 1 to 3, **characterized in that**

$R^1$ stands for phenyl,
where phenyl is substituted with 1 to 2 substituents, where the substituents are selected independently of one another from the group consisting of halogen, cyano and methyl,
$R^2$ stands for phenyl,
where phenyl is substituted with 1 to 2 substituents, where the substituents are selected independently of one another from the group consisting of halogen, cyano, methyl and methoxy, and
$R^3$ stands for a heterocyclyl residue bound with nitrogen,
where the heterocyclyl residue can be substituted with 1 to 2 substituents from the hydroxy and oxo group,

or one of their salts, their solvates or the solvates of their salts.

**5.** The compound according to any of Claims 1 to 4, **characterized in that**

$R^1$ stands for phenyl,
where phenyl is substituted with 1 to 2 substituents, where the substituents are selected independently of one another from the group consisting of halogen and cyano,
$R^2$ stands for phenyl,
where phenyl is substituted with 1 to 2 substituents, where the substituents are selected independently of one another from the group consisting of halogen and cyano, and
$R^3$ stands for

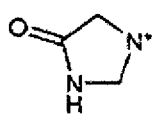 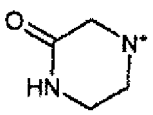 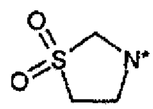

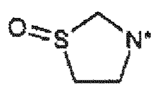 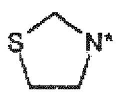 or 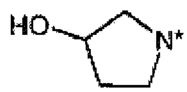

wherein
* indicates the binding site to the carbonyl group

or one of their salts, their solvates or the solvates of their salts.

**6.** The compound according to any of Claims 1 to 4, **characterized in that** it corresponds to the formula

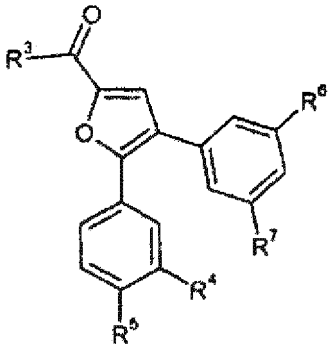

(Ia),

wherein

R3 stands for a heterocyclyl residue bound with nitrogen,
where the heterocyclyl residue can be substituted with 1 to 2 substituents from the hydroxy and oxo group,
$R^4$ stands for halogen, cyano or methyl,
$R^5$ stands for hydrogen or halogen,
$R^6$ stands for halogen, cyano, methyl or methoxy, and
$R^7$ stands for hydrogen or halogen,

or one of their salts, their solvates or the solvates of their salts.

**7.** The compound according to Claim 6, **characterized in that**

$R^3$ stands for

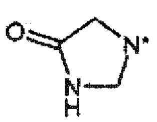 , 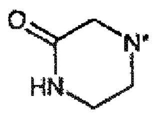 , 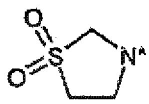

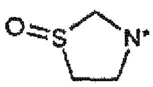 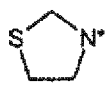 or 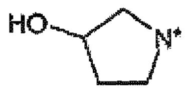

wherein
* indicates the binding site to the carbonyl group,
R4 stands for halogen, cyano or methyl,
R5 stands for hydrogen or halogen,
R6 stands for halogen, cyano, methyl or methoxy, and
R7 stands for hydrogen or halogen,

or one of their salts, their solvates or the solvates of their salts.

**8.** The compound according to Claim 6 or 7, **characterized in that**

$R^3$ stands for

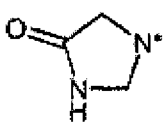 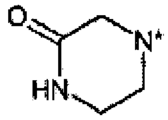 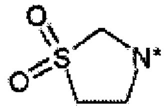

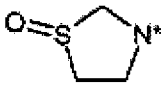 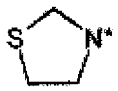 or 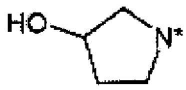

wherein

* indicates the binding site to the carbonyl group,
$R^4$ stands for fluorine, chlorine, cyano or methyl,
$R^5$ stands for hydrogen or fluorine,
$R^6$ stands for fluorine, chlorine, cyano, methyl or methoxy, and
$R^7$ stands for hydrogen or fluorine,

or one of their salts, their solvates or the solvates of their salts.

**9.** The compound according to any of Claims 6 to 8, **characterized in that**

$R^3$ stands for

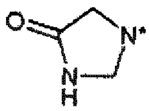

wherein
* indicates the binding site to the carbonyl group,
$R^4$ stands for chlorine or cyano,
$R^5$ stands for hydrogen or fluorine,
$R^6$ stands for chlorine or cyano, and
$R^7$ stands for hydrogen or fluorine,

or one of their salts, their solvates or the solvates of their salts.

**10.** A method for preparing a compound of Formula (I) according to Claim 1, **characterized in that** a compound of the formula

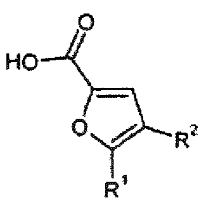 (II),

wherein
$R^1$ and $R^2$ have the meaning specified above,
is reacted with a compound of Formula $R^3$, with the meaning specified in Claim 1, or a salt of a compound of Formula

$R^3$.

**11.** A method for preparing a compound of Formula (I) according to Claim 1, **characterized in that** a compound of the formula

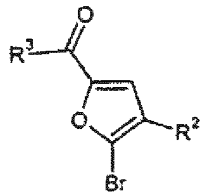

(VIII),

wherein
$R^2$ and $R^3$ have the meaning specified in Claim 1,
is reacted under Suzuki coupling conditions with a compound of the formula

$R^1$-Q (VII),

wherein

$R^1$ has the meaning given in Claim 1 and
Q stands for $-B(OH)_2$, a boronic acid ester, preferably a boronic acid pinacol ester, or $-BF_3^-K^+$.

**12.** The compound according to any of Claims 1 to 9 for the treatment and/or prevention of diseases.

**13.** Use of a compound according to any of Claims 1 to 9 to produce medication for the treatment and/or prevention of diseases.

**14.** Use of a compound according to any of Claims 1 to 9 to produce a medication for the treatment and/or prevention of retroviral diseases.

**15.** Use according to Claim 14, **characterized in that** the retroviral disease is an HIV infection.

**16.** A medication containing at least one compound according to any of Claims 1 to 9 in combination with at least one additional active ingredient.

**17.** A medication containing at least one compound according to any of Claims 1 to 9 in combination with at least one inert, non-toxic, pharmaceutically appropriate excipient.

**18.** The medication according to Claim 16 or 17 for the treatment and/or prevention of retroviral diseases.

**19.** The medication according to Claim 18, **characterized in that** the retroviral disease is an HIV infection.

**20.** The compound according to any of Claims 1-9 or a medication according to any of Claims 16 to 19 for use in the treatment of viral diseases in humans and animals by administering an antivirally effective quantity of said compound or said medication.

**Revendications**

**1.** Un composé de la formule

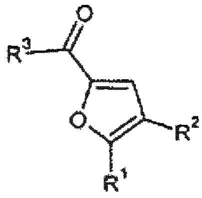

(I),

dans laquelle

$R^1$ représente phényle,
où phényle est substitué par 1 ou 3 substituants, les substituants étant sélectionnés indépendamment l'un de l'autre parmi le groupe constitué par halogène, hydroxy, cyano, nitro, trifluorométhyle, trifluorométhoxy, trifluorométhylthio, $(C_1-C_4)$- alkyl et $(C_1-C_4)$-alkoxy,
dans laquelle

$(C_1-C_4)$-alkyle et $(C_1-C_4)$-alkoxy peuvent à leur tour être substitués une à trois fois, de façon égale ou différente, par des résidus sélectionnés parmi le groupe constitué par halogène, cyano, hydroxy, $(C_1-C_4)$-alkoxy, amino, mono-$(C_1-C_4)$-alkylamino, di-$(C_1-C_4)$-alkylamino, $(C_3-C_7)$-cycloalkyle et hétérocycle ayant 4 à 7 membres,
dans laquelle lesdits résidus de cycloalkyle et d'hétérocycle peuvent à leur tour être substitués une à trois fois, de façon égale ou différente, par halogène, cyano, $(C_1-C_4)$-alkyle, trifluorométhyl, hydroxy, $(C_1-C_4)$-alkoxy, trifluorométhoxy, oxo, amino, mono-$(C_1-C_4)$-alkylamino et di-$(C_1-C_4)$-alkylamino,

$R^2$ représente phényle,
où phényle est substitué par 1 ou 3 substituants, les substituants étant sélectionnés indépendamment l'un de l'autre parmi le groupe constitué par halogène, hydroxy, cyano, nitro, trifluorométhyle, trifluorométhoxy, trifluorométhylthio, $(C_1-C_4)$- alkyl et $(C_1-C_4)$-alkoxy,
dans laquelle

$(C_1-C_4)$-alkyle et $(C_1-C_4)$-alkoxy peuvent à leur tour être substitués une à trois fois, de façon égale ou différente, par des résidus sélectionnés parmi le groupe constitué par halogène, cyano, hydroxy, $(C_1-C_4)$-alkoxy, amino, mono-$(C_1-C_4)$-alkylamino, di-$(C_1-C_4)$-alkylanlino, $(C_3-C_7)$-cycloalkyle et hétérocycle ayant 4 à 7 membres,
dans laquelle lesdits résidus de cycloalkyl et d'hétérocycle peuvent à leur tour être substitués une à trois fois, de façon égale ou différente, par halogène, cyano, $(C_1-C_4)$-alkyle, trifluoronnéthyl, hydroxy, $(C_1-C_4)$-alkoxy, trifluorométhoxy, oxo, amino, mono-$(C_1-C_4)$-alkylamino et di-$(C_1-C_4)$-alkylamino, et

$R^3$ représente un résidu d'hétérocyclyle lié par l'azote,
où le résidu d'hétérocyclyle peut être substitué par 1 à 3 substituants, les substituants étant sélectionnés indépendamment l'un de l'autre parmi le groupe constitué par halogène, hydroxy, hydroxyméthyl, formyl, amino, oxo, trifluorométhyl, trifluoromethoxy, $(C_1-C_4)$-alkyl, $(C_1-C_4)$-alkoxy et $(C_1-C_4)$-alkoxycarbonyl,

ou un de leurs sels, solvats ou les solvats de leurs sels.

2. Le composé selon la revendication 1, **caractérisé en ce que**

$R^1$ représente phényle,
où phényle est substitué par 1 ou 2 substituants, les substituants étant sélectionnés indépendamment l'un de l'autre parmi le groupe constitué par halogène, hydroxy, cyano, nitro, trifluorométhyle, trifluorométhoxy, trifluorométhylthio, $(C_1-C_4)$- alkyl et $(C_1-C_4)$-alkoxy,
$R^2$ représente phényle,
où phényle est substitué par 1 ou 2 substituants, les substituants étant sélectionnés indépendamment l'un de l'autre parmi le groupe constitué par halogène, hydroxy, cyano, nitro, trifluorométhoxy, trifluorométhylthio, $(C_1-C_4)$-alkyl et $(C_1-C_4)$-alkoxy,

dans laquelle

$(C_1-C_4)$-alkoxy peut à son tour être substitué une à trois fois, de façon égale ou différente, par des résidus sélectionnés parmi le groupe constitué par halogène, cyano, hydroxy, $(C_1-C_4)$-alkoxy, amino, mono-$(C_1-C_4)$-alkylamino, di-$(C_1-C_4)$-alkylamino, $(C_3-C_7)$-cycloalkyle et hétérocycle ayant 4 à 7 membres,

dans laquelle lesdits résidus de cycloalkyle et d'hétérocycle peuvent à leur tour être substitués une à trois fois, de façon égale ou différente, par halogène, cyano, $(C_1-C_4)$-alkyle, trifluorométhyl, hydroxy, $(C_1-C_4)$-alkoxy, trifluorométhoxy, oxo, amino, mono-$(C_1-C_4)$-alkylamino et di-$(C_1-C_4)$-alkylamino,

$R^3$ représente un résidu d'hétérocyclyle lié par l'azote,

où le résidu d'hétérocyclyle peut être substitué par 1 à 3 substituants, les substituants étant sélectionnés indépendamment l'un de l'autre parmi le groupe constitué par halogène, hydroxy, hydroxyméthyl, formyl, amino, oxo, trifluorométhyl, trifluoromethoxy, $(C_1-C_4)$-alkyl, $(C_1-C_4)$-alkoxy et $(C_1-C_4)$-alkoxycarbonyl,
ou un de leurs sels, solvats ou les solvats de leurs sels.

3. Le composé selon la revendication 1 ou 2, **caractérisé en ce que**

$R^1$ représente phényle,
où phényle est substitué par 1 ou 2 substituants, les substituants étant sélectionnés indépendamment l'un de l'autre parmi le groupe constitué par halogène, cyano, trifluorométhyle, methyle et méthoxy,
$R^2$ représente phényle,
où phényle est substitué par 1 ou 2 substituants, les substituants étant sélectionnés indépendamment l'un de l'autre parmi le groupe constitué par halogène, cyano, trifluorométhoxy, methyle et (C1-C3)-alkoxy, et
$R^3$ représente un résidu d'hétérocyclyle lié par l'azote,
où le résidu d'hétérocyclyle peut être substitué par 1 à 3 substituants, les substituants étant sélectionnés indépendamment l'un de l'autre parmi le groupe constitué par halogène, hydroxy, amino, oxo, trifluorométhyl, $(C_1-C_4)$-alkyl et $(C_1-C_4)$-alkoxy,

ou un de leurs sels, solvats ou les solvats de leurs sels.

4. Le composé selon l'une quelconque des revendications 1 à 3, **caractérisé en ce que**

$R^1$ représente phényle,
où phényle est substitué par 1 ou 2 substituants, les substituants étant sélectionnés indépendamment l'un de l'autre parmi le groupe constitué par halogène, cyano et methyle,
$R^2$ représente phényle,
où phényle est substitué par 1 ou 2 substituants, les substituants étant sélectionnés indépendamment l'un de l'autre parmi le groupe constitué par halogène, cyano, methyle et méthoxy, et
$R^3$ représente un résidu d'hétérocyclyle lié par l'azote, où le résidu d'hétérocyclyle peut être substitué par 1 à 2 substituants parmi le groupe constitué par hydroxy et oxo,

ou un de leurs sels, solvats ou les solvats de leurs sels.

5. Le composé selon l'une quelconque des revendications 1 à 4, **caractérisé en ce que**

$R^1$ représente phényle,
où phényle est substitué par 1 ou 2 substituants, les substituants étant sélectionnés indépendamment l'un de l'autre parmi le groupe constitué par halogène et cyano,
$R^2$ représente phényle,
où phényle est substitué par 1 ou 2 substituants, les substituants étant sélectionnés indépendamment l'un de l'autre parmi le groupe constitué par halogène et cyano, et
$R^3$ représente

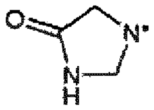, 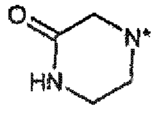, 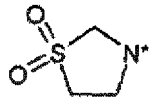

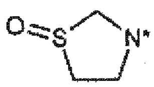 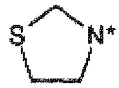 ou 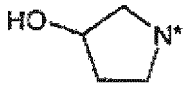

où
* indique le site de liaison au groupe carbonyl

ou un de leurs sels, solvats ou les solvats de leurs sels.

**6.** Le composé selon l'une quelconque des revendications 1 à 4, **caractérisé en ce qu'**il correspond à la formule

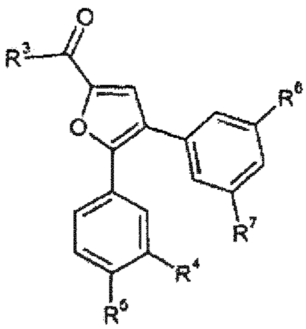

(Ia),

dans laquelle

$R^3$ représente un résidu d'hétérocyclyle lié par l'azote,
où le résidu d'hétérocyclyle peut être substitué par 1 à 2 substituants du groupe constitué par hydroxy et oxo,
$R^4$ représente halogène, cyano ou méthyl,
$R^5$ représente hydrogène ou halogène,
$R^6$ représente halogène, cyano, méthyl ou méthoxy, et
$R^7$ représente hydrogène ou halogène,

ou un de leurs sels, solvats ou les solvats de leurs sels.

**7.** Le composé selon la revendication 6, **caractérisé en ce que**

$R^3$ représente

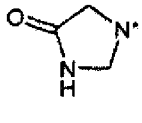, 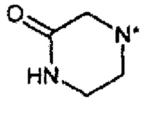, 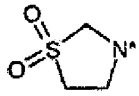

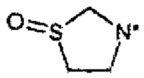 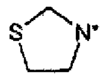 ou 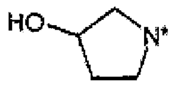

où
* indique le site de liaison au groupe carbonyl,

R$^4$ représente halogène, cyano ou méthyl,
R$^5$ représente hydrogène ou halogène,
R$^6$ représente halogène, cyano, méthyl ou méthoxy, et
R$^7$ représente hydrogène ou halogène,

ou un de leurs sels, solvats ou les solvats de leurs sels.

**8.** Le composé selon la revendication 6 ou 7, **caractérisé en ce que**

R$^3$ représente

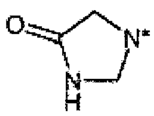, 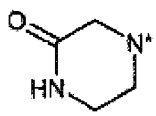, 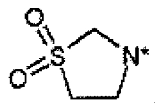,

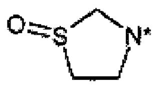, 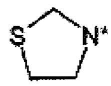 ou 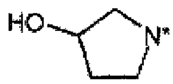

où
* indique le site de liaison au groupe carbonyl,
R$^4$ représente fluor, chlore, cyano ou méthyl,
R$^5$ représente hydrogène ou fluor,
R$^6$ représente fluor, chlore, cyano, méthyl ou méthoxy, et
R$^7$ représente hydrogène ou fluor,

ou un de leurs sels, solvats ou les solvats de leurs sels.

**9.** Le composé selon l'une quelconque des revendications 6 à 8, **caractérisé en ce que**

R$^3$ représente

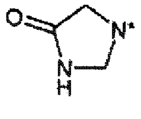

où
* indique le site de liaison au groupe carbonyl,

R$^4$ représente chlore ou cyano,
R$^5$ représente hydrogène ou fluor,

$R^6$ représente chlore ou cyano, et
$R^7$ représente hydrogène ou fluor,

ou un de leurs sels, solvats ou les solvats de leurs sels.

**10.** Un procédé de production d'un composé de la formule (I) selon la revendication 1, **caractérisé en ce qu'**un composé de la formule suivante

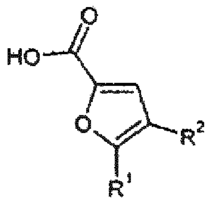
(II),

dans laquelle
$R^1$ et $R^2$ ont la signification spécifiée ci-dessus,
est amené à réagir avec un composé de la formule $R^3$, dont la signification est indiquée à la revendication 1, ou un sel d'un composé de la formule $R^3$.

**11.** Le procédé de production d'un composé de la formule (I) selon la revendication 1, **caractérisé en ce qu'**un composé de la formule suivante

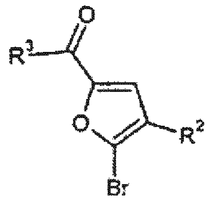
(VIII),

dans laquelle
$R^2$ et $R^3$ ont la signification spécifiée dans la revendication 1,
est réalisée dans des conditions de couplage Suzuki, avec composé de la formule

$R^1$-Q (VII),

dans laquelle
$R^1$ a la signification spécifiée dans la revendication 1 et
Q représente $-B(OH)_2$. un ester d'acide boronique, de préférence un ester pinacolique d'acide boronique ou $-BF_3^-K^+$.

**12.** Le composé selon l'une quelconque des revendications 1 à 9 destiné au traitement et/ou à la prévention de maladies.

**13.** Utilisation d'un composé selon l'une quelconque des revendications 1 à 9 pour produire un médicament destiné au traitement et/ou la prévention de maladies.

**14.** Utilisation d'un composé selon l'une quelconque des revendications 1 à 9 pour produire un médicament destiné au traitement et/ou à la prévention de maladies rétrovirales.

**15.** Utilisation selon la revendication 14, **caractérisée en ce que** la maladie rétrovirale est une infection au VIH.

**16.** Un médicament contenant au moins un composé selon l'une quelconque des revendications 1 à 9 en combinaison avec au moins une substance active supplémentaire.

**17.** Le médicament contenant au moins un composé selon l'une quelconque des revendications 1 à 9 en combinaison avec au moins un excipient inerte, non toxique, pharmaceutiquement acceptable.

**18.** Le médicament selon la revendication 16 ou 17 destiné au traitement et/ou à la prévention de maladies rétrovirales.

**19.** Le médicament selon la revendication 18, **caractérisé en ce que** la maladie rétrovirale est une infection au VIRIL.

**20.** Composé selon une quelconque des revendications 1 à 9 ou médicament selon une quelconque des revendications 16 à 19 destiné au traitement de maladies virales chez les humains et les animaux par administration d'une quantité antivirale efficace dudit composé ou médicament.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 5342835 A
- WO 2004076453 A1
- WO 2005007625 A2
- WO 2008043775 A1
- WO 2006062982 A2
- WO 2008080056 A
- WO 2008090382 A1
- GB 453061 A


### In der Beschreibung aufgeführte Nicht-Patentliteratur


- **PALELLA et al.** *N. Engl. J. Med.,* 1998, vol. 238, 853-860
- **FLEXNER.** *Nature Reviews Drug Discovery,* 2007, vol. 6, 959-966
- **CARPENTER et al.** *J. Am. Med. Assoc.,* 2000, vol. 283, 381-390
- **FINZI et al.** *Nature Med.,* 1999, vol. 5, 512-517
- **RAMRATNAM et al.** *Nature Med.,* 2000, vol. 6, 82-85
- **KAVLICK ; MITSUYA.** Antiretroviral Chemotherapy. ASM Press, 2001, 279-312
- **HOUSTON JB.** Utility of in-vitro drug-metabolism data in predicting in-vivo metabolic-clearance. *Bioch. Pharm.,* 1994, vol. 47 (9), 1469-1479
- **OBACH RS ; BAXTER JG ; LISTON TE ; SILBER BM ; JONES BC ; MACINTYRE F ; RANCE DJ ; WASTALL P.** The prediction of human pharmacokinetic parameters from preclinical and in vitro metabolism data. *J. Pharmacol. Exp. Ther.,* 1997, vol. 283 (1), 46-58
- *J Pharmacokinet Biopharm,* 1977, vol. 5 (6), 625-53